# EUROPEAN PATENT APPLICATION

(11) **EP 3 381 947 A1**
(43) Date of publication of application: **03.10.2018**
(21) Application number: 16868628.5
(22) Date of filing: 24.11.2016
(51) Int. Cl.: C07K 19/00, C07K 14/37, C07K 14/415, C07K 14/435, C12N 9/10, C12N 15/09

(54) **DNA POLYMERASE VARIANT**

(30) Priority: 27.11.2015 JP 2015231974
(71) Applicant: Kyushu University National University Corporation, Fukuoka 812-8581 (JP); Takara Bio Inc., Kusatsu-shi Shiga 525-0058 (JP)
(72) Inventor: ISHINO, Yoshizumi, Fukuoka-shi Fukuoka 812-8581 (JP); ISHINO, Sonoko, Fukuoka-shi Fukuoka 812-8581 (JP); YAMAGAMI, Takeshi, Fukuoka-shi Fukuoka 812-8581 (JP); UEMORI, Takashi, Kusatsu-shi Shiga 525-0058 (JP); TAKATSU, Nariaki, Kusatsu-shi Shiga 525-0058 (JP)
(74) Representative: Vossius & Partner Patentanwälte Rechtsanwälte mbB
(86) International application number: PCT/JP2016/084808
(87) International publication number: WO 2017/090685

(57) **Abstract**

The present invention relates to a fusion polypeptide containing, in a direction of from an N-terminal side to a C-terminal side, one or more peptides which bind to a PCNA, and a polypeptide having a DNA polymerase activity; a method for amplifying nucleic acids using the polypeptide; and a composition and a kit, containing the polypeptide. According to the present invention, it is made possible to amplify a long-strand DNA in a short time in amplifying nucleic acids in the presence of PCNA even with a Pol I-type DNA polymerase.

## Description

### TECHNICAL FIELD

The present invention relates to a DNA polymerase variant. The DNA polymerase variant of the present invention is particularly useful in amplifying nucleic acids in the presence of PCNA.

### BACKGROUND ART

DNA polymerases are enzymes capable of freshly synthesizing a DNA strand in line with a DNA strand serving as a template *in vitro,* and a DNA strand is freshly synthesized so long as there are, besides a template DNA, an oligonucleotide that serves as primers and four kinds of deoxynucleotides (dATP, dGTP, dCTP, and dTTP) in the reaction. The DNA polymerases have been utilized in numerous manipulations such as methods for amplifying nucleic acids including nucleotide sequencing and a polymerase chain reaction (PCR).

As the associated factors which improve DNA synthesis-related various properties (extensibility, speediness, accuracy, etc.) of DNA polymerases, various proteins have been found from thermophilic archaebacteria. As the associated factors, for example, plural proteins derived from *Pyrococcus furiosus* have been isolated (Patent Publication 1). In addition, as the associated factors, PCNA (proliferating cell nuclear antigen), RFC-S (replication factor C small subunit), or RFC-L (replication factor C large subunit) have been isolated from *Thermococcus kodakarensis* KOD1 strain (Patent Publication 2).

The PCNA, as a homopolymer, forms a cyclic structure called "sliding clamp," which accelerates a DNA synthesis reaction. The PCNA is highly conserved from yeasts to human, and in eukaryotic cells a PCNA plays an important role in cell divisions, DNA replications, repairs, cell cycle regulations, or post-replication modifications such as DNA methylation and chromatin remodeling.

The RFC (replication factor C) is a protein complex composed of five subunits, and is also called "clamp loader" from its function of loading PCNA to DNA. Also, the RFC is equivalent to a γ-complex of *Escherichia coli.* The functions of the RFC as a clamp loader will be explained as follows: (1) An RFC binds to a DNA strand; (2) using energy generated by hydrolysis of ATP, an RFC opens a cyclic PCNA; (3) PCNA clamps a DNA strand; and (4) the ATP is further hydrolyzed, whereby the RFC is dissociated from the DNA, and the PCNA binds to the DNA.

A PCNA forms a complex with various proteins other than DNA polymerases and an RFC, and is involved in repairs and replications of a DNA and other genetic controlling functions. It has been known that in human at least twelve proteins bind to a PCNA. Each of the proteins binds to a PCNA via a PIP box (PCNA interaction protein box), so that the protein would be detained on a DNA strand.

It has been elucidated that there are some amino acid sequences highly homologous in the PIP box, and that some proteins bind to a PCNA via a PIP box site (Non-Patent Publication 1).

As mentioned above, a PCNA and an RFC cooperatively functions in nature to carry out DNA replications. Utilizing a PCNA which particularly plays a central role among them, an attempt has been made to improve the efficiency of PCR. A family A (Pol I-type) DNA polymerase derived from thermophilic eubacteria *Thermus aquaticus* (which is also referred to as "Taq polymerase"), which is widely used in PCR, does not interact with a PCNA. However, it has been reported that a chimeric fusion protein (chimeric Taq) in which 50 amino acids including a PIP box derived from Pol B of *Archaeoglobus fulgidus* are fused to a Taq polymerase at a C-terminal, so as to have the same form as a family B (α-type) DNA polymerase, amplifies the DNA in the presence of a PCNA derived from *A. fulgidus.* However, when the size of an amplified DNA was 5 kb, bands ascribed to the synthesized products became thin, so that there were still some disadvantages in extensibility (Non-Patent Publication 2).

### PRIOR ART PUBLICATIONS

### PATENT PUBLICATIONS

Patent Publication 1: WO 1999/00506
Patent Publication 2: Japanese Patent Laid-Open No. 2002-360261

### NON-PATENT PUBLICATIONS

Non-Patent Publication 1: Genes to Cells, 7(9), 911-922 (2002)
Non-Patent Publication 2: J. Biol. Chem., 277(18), 16179-16188 (2002)

### SUMMARY OF THE INVENTION

### PROBLEMS TO BE SOLVED BY THE INVENTION

As mentioned above, in the prior art, it can hardly be said that a DNA polymerase is provided which satisfies all of various properties such as extensibility, speediness, and accuracy.

The present invention is aimed at solving the problem of the conventional family A (Pol I-type) DNA polymerases as described above, and an object thereof is to provide a DNA polymerase which is more convenient and easy to use, and has excellent extensibility and speediness, and a method for amplifying nucleic acids using the polymerase.

### MEANS TO SOLVE THE PROBLEMS

The present inventors have intensively studied for the purpose of providing a novel family A (Pol I-type) DNA polymerase which can be utilized for amplifying nucleic acids in the presence of PCNA, and as a result, found that a reaction for amplifying nucleic acids is accelerated in the presence of a PCNA by utilizing a fusion polypeptide containing, in a direction of from an N-terminal side to a C-terminal side, one or more peptides which bind to a PCNA, and a polypeptide having a DNA polymerase activity. Thus, the present invention was completed.

Summarizing the present invention, the present invention relates to:
[1] a fusion polypeptide containing, in a direction of from an N-terminal side to a C-terminal side,
   a) one or more peptides which bind to a PCNA, and
   b) a polypeptide having a DNA polymerase activity;
[2] the fusion polypeptide according to [1], characterized in that the peptide which bind to a PCNA is a peptide comprising a PIP box;
[3] the fusion polypeptide according to [2], characterized in that the PIP box is a peptide consisting of any one of amino acid sequences shown in SEQ ID NOs: 52 to 91 of the Sequence Listing;
[4] the fusion polypeptide according to [1] or [2], characterized in that the peptide which bind to a PCNA is a peptide comprising a PIP box derived from a DNA polymerase-associated factor;
[5] the fusion polypeptide according to any one of [1], [2] and [4], characterized in that the peptide which bind to a PCNA is a peptide comprising a PIP box derived from a replication factor C large subunit;
[6] the fusion polypeptide according to any one of [1], [2], [4], and [5], characterized in that the peptide which bind to a PCNA is the amino acid sequence shown in SEQ ID NO: 3 of the Sequence Listing, or a peptide containing the above amino acid sequence;
[7] the fusion polypeptide according to any one of [1] to [6], characterized in that the fusion polypeptide comprises a 5 to 50 amino acid linker peptide between the peptide which bind to a PCNA and the polypeptide having a DNA polymerase activity;
[8] the fusion polypeptide according to [7], characterized in that the linker peptide is an amino acid sequence composed of serine and glycine;
[9] the fusion polypeptide according to any one of [1] to [8], characterized in that the polypeptide having a DNA polymerase activity is a Pol I-type DNA polymerase or a fragment thereof;
[10] the fusion polypeptide according to any one of [1] to [9], characterized in that the polypeptide having a DNA polymerase activity is a Taq DNA polymerase or a fragment thereof;
[11] a nucleic acid encoding a fusion polypeptide as defined in any one of [1] to [10];
[12] a composition for amplifying nucleic acids containing a fusion polypeptide as defined in any one of [1] to [10];
[13] the composition for amplifying nucleic acids according to [12], further containing a PCNA;
[14] a kit containing a fusion polypeptide as defined in any one of [1] to [10];
[15] the kit according to [14], further containing a PCNA; and
[16] a method for producing a DNA complementary to a template DNA, characterized by the use of a composition containing a fusion polypeptide as defined in any one of [1] to [10] and a PCNA.

### EFFECTS OF THE INVENTION

According to the present invention, even a Pol I-type DNA polymerase, in the presence of PCNA, can amplify a long-strand DNA in a short time.

### BRIEF DESCRIPTION OF THE DRAWINGS

[FIG. 1] FIG. 1 is a photograph of SDS-PAGE gel relating to purification of a Taq DNA polymerase variant in Example 1. By analysis of SDS-PAGE, purities of Taq81 to Taq85 were confirmed.
[FIG. 2] FIG. 2 is a photograph of SDS-PAGE gel relating to purification of a Taq DNA polymerase variant in Example 1. By analysis of SDS-PAGE, purities of Taq92 to Taq94 were confirmed.
[FIG. 3] FIG. 3 is charts showing physical interaction analyses using a surface plasmon resonance (SPR) method in Example 2. By the SPR analyses, physical interactions between Taq DNA polymerase variants (Taq81 to Taq85 and Taq94) and PfuPCNA were measured.
[FIG. 4] FIG. 4 is a picture showing the results of amplifying a 1 kb DNA in Example 3(1). By PCR, DNA amplification abilities of Taq81 to Taq85 were confirmed.
[FIG. 5] FIG. 5 is a picture showing the results of amplifying an 8 kb DNA in Example 3(2). By PCR, DNA amplification abilities of Taq81 to Taq85 in the presence of PCNA were confirmed.
[FIG. 6] FIG. 6 is a picture showing the results of amplifying an 8 kb DNA in Example 3(2). By PCR, DNA amplification abilities of Taq92 to Taq94 in the presence of PCNA were confirmed.
[FIG. 7] FIG. 7 is a picture showing the results of amplifying a 12 kb DNA in Example 3(3). By PCR, DNA amplification abilities of Taq81 to Taq85 in the presence of PCNA were confirmed.
[FIG. 8] FIG. 8 is a picture showing the results of amplifying a 15 kb DNA in Example 3(3). By PCR, DNA amplification abilities of Taq81 to Taq85 in the presence of PCNA were confirmed.
[FIG. 9] FIG. 9 is a picture showing the results of amplifying a 12 kb DNA by PCR in Example 4. By PCR, DNA amplification abilities of Taq95 to Taq98 in the presence of PCNA were confirmed.
[FIG. 10] FIG. 10 is a graph showing relative values of amplifying a 12 kb DNA by PCR in Example 4.

### MODES FOR CARRYING OUT THE INVENTION

### Definitions, etc.

The term "peptide" as used herein refers to a compound in which two or more amino acid molecules are bonded by removing one water molecule from an amino group of one amino acid molecule and a carboxyl group of the other amino acid molecule. In general, those composed of about 10 or less amino acids are referred to as oligopeptides, and those composed of equal to or greater than the above number of amino acids are referred to as polypeptides, but there are no strict boundaries therebetween.

The term "fusion polypeptide" as used herein refers to a polypeptide comprising two or more polypeptides which are not fused in a natural state, and a polypeptide comprising a peptide and a polypeptide that is not fused in a natural state.

The term "PCNA" as used herein is an abbreviation for proliferating cell nuclear antigen, and is a constituent of a protein molecule called "sliding clamp" from its unique shape and functions. The PCNA is a replication cofactor that forms a ring-shaped structure as a homopolymer, clamps a DNA strand in its central hole, and binds to a DNA polymerase at its surface to detain the enzyme on the DNA, thereby accelerating a DNA strand synthesis reaction. The PCNA is highly conserved from yeasts to human, and in eukaryotic cells, PCNA plays important roles in cell divisions, DNA replications, repairs, cell cycle regulations, or post-replication modifications such as DNA methylation and chromatin remodeling. In addition, in the present invention, all the proteins having the above functions are embraced within the PCNAs even though the names differ.

The phrase "polypeptide having a DNA polymerase activity" as used herein refers to a polypeptide having an activity of synthesizing a DNA strand complementary to a template nucleic acid (DNA or RNA) using deoxyribonucleotide triphosphate as a substrate. In the present invention, a known DNA polymerase or a variant thereof can be used as the "polypeptide having a DNA polymerase activity."

As to the DNA polymerase in the present invention, when referred to "activity," unless specified otherwise, the activity includes a DNA synthesizing activity and a primer extension activity. The DNA synthesizing activity includes an activity of using a DNA as a template, and synthesizing a DNA complementary thereto; and an activity of using an RNA as a template, and synthesizing a DNA complementary thereto. The DNA synthesizing activity can be measured as an uptake activity of a substrate deoxyribonucleotide triphosphate (dNTP) as well known to the person skilled in the art. More concretely, in a complementary strand synthesis reaction using a template such as a calf thymus DNA or a salmon sperm DNA, which is partially digested with DNaseI, and dNTP labeled with a radioactive isotope, the DNA polymerase activity is measured as an amount of the uptake of radioactive isomer into the complementary strand. This method is called a nucleotide uptake assay, and is also a standard method for measuring a DNA polymerase activity. Alternatively, the activity of a DNA polymerase can be evaluated by measuring a chain length of a primer extension product synthesized by a DNA polymerase using a template DNA hybridized with a primer as a substrate.

The present invention will be explained in detail hereinbelow.

### (1) Fusion Polypeptide of the Present Invention

The fusion polypeptide of the present invention contains, in a direction of from an N-terminal side to a C-terminal side,
a) one or more peptides which bind to a PCNA, and
b) a polypeptide having a DNA polymerase activity.
Accordingly, the fusion polypeptide of the present invention can be said to be a DNA polymerase variant.

The phrase "peptides which bind to a PCNA" constituting the above fusion polypeptide is not particularly limited, so long as the peptides have the abilities of binding to a PCNA. Examples of the peptide include peptides containing a PIP box, which are the peptides existing in various PCNA-bindable proteins. The PIP box is an amino acid sequence existing in a protein interacting with a PCNA, and serves to detain the protein via the PCNA on the DNA strand. Here, in the present invention, all the peptides having the above functions would be embraced in the PIP boxes even though the names differ. For example, it has been known that thermophilic bacteria proteins involved in DNA replications or the like (for example, replication factor C large subunit etc.) have a PIP box. In the present invention, for example, examples of a preferred PIP box include, but not particularly limited to, an oligopeptide composed of at least eight amino acids, denoted by A1-A2-A3-A4-A5-A6-A7-A8, wherein A1 is glutamine residue, each of A2 and A3 is any amino acid residues, A4 is an amino acid residue selected from the group consisting of leucine residue, isoleucine residue, and methionine residue, each of A5 and A6 is any amino acid residues, A7 is phenylalanine residue or tryptophan residue, and A8 is an amino acid residue selected from the group consisting of phenylalanine residue, tryptophan residue, or leucine residue. Especially preferred includes one shown in SEQ ID NO: 3 of the Sequence Listing including eight amino acids QATLFDFL. Further, in the present invention, the peptide may be an oligopeptide containing 9 amino acids in which the above oligopeptide of eight amino acids further comprises lysine residue at an N-terminal side thereof. Examples of the amino acid sequences of the PIP box usable in the present invention are shown in Table 1 without intending to particularly limit the present invention thereto.

### [Table 1-1]

**Table 1**

| Name of Protein | Organism Species | Amino Acid Sequence | SEQ ID NO. in Sequence Listing |
|---|---|---|---|
| RFC-L | *P. furiosus* | QATLFDFL | 52 |
| | *M. jannaschii* | QLTLDAFF | 53 |
| PolBI | *P. furiosus* | QVGLTSWL | 54 |
| | *T. litoralis* | QTGLDAWL | 55 |
| | *A. fulgidus* | QMSLDSFF | 56 |
| PolBII | *P. occultum* | QRSLFDFF | 57 |
| DP2 | *M. jannaschii* | QVKLSDFF | 58 |
| | *M. thermoautotrophicum* | QSSLDVFL | 59 |
| Polδ p66 | *H. sapiens* | QVSITGFF | 60 |
| Pol32 | *S. cerevisiae* | QGTLESFF | 61 |
| Cdc27 | *S. pombe* | QKSIMSFF | 62 |
| Pol2 | *S. cerevisiae* | QTSLTKFF | 63 |
| Fen1(RAD2) | *P. furiosus* | QSTLESWF | 64 |
| | *M. jannaschii* | QKTLDAWF | 65 |
| | *A. fulgidus* | QATLERWF | 66 |
| | *H. sapiens* | QGRLDDFF | 67 |
| | *M. musculus* | | |
| | *X. laevis* | | |
| | *D. melanogaster* | QVRLDSFF | 68 |
| | *S. cerevisiae* | QGRLDGFF | 69 |
| | *S. pombe* | QGRLDSFF | 70 |

### [Table 1-2]

**Table 1 (continued)**

| Name of Protein | Organism Species | Amino Acid Sequence | SEQ ID NO. in Sequence Listing |
|---|---|---|---|
| DNA ligase I | *H. sapiens* | QRSIMSFF | 71 |
| | *M. musculus* | | |
| | *X. laevis* | QRTIKSFF | 72 |
| | *S. cerevisiae* | QATLARFF | 73 |
| | *S. pombe* | QSDISNFF | 74 |
| MSH3 | *H. sapiens* | QAVLSRFF | 75 |
| | *S. cerevisiae* | QPTISRFF | 76 |
| MSH6 | *H. sapiens* | QSTLYSFF | 77 |
| | *S. cerevisiae* | QSSLLSFF | 78 |
| UNG2 | *H. sapiens M. musculus* | QKTLYSFF | 79 |
| UNG | *S. cerevisiae* | QTTIEDFF | 80 |
| hMYH | *H. sapiens* | QQVLDNFF | 81 |
| XPG | *H. sapiens* | QLRIDSFF | 82 |
| | *M. musculus* | | |
| | *X. laevis* | | |
| | *C. elegans* | QMRLDRFF | 83 |
| | *S. cerevisiae* | QKRINEFF | 84 |
| | *S. pombe* | QSNLTQFF | 85 |
| Cac1 | *S. cerevisiae* | QSRIGNFF | 86 |
| hRECQ5 | *H. sapiens* | QNLIRHFF | 87 |
| Rrm3 | *S. cerevisiae* | QQTLSSFF | 88 |
| Cdc25C | *H sapiens* | QEELFNFF | 89 |
| p15 | *H. sapiens* | QKGIGEFF | 90 |
| DNA-dependent protein kinase | *H. sapiens* | QLIIRNFW | 91 |

Further, the PIP box used in the present invention includes, but not particularly limited to, those derived from proteins produced by thermophilic bacteria. Examples include preferably a PIP box derived from a replication factor C large subunit of thermophilic bacteria, and more preferably a PIP box derived from a replication factor C large subunit of *Pyrococcus furiosus.* Alternatively, it may be a functional equivalent having substantially same level of activity as those mentioned above.

In addition, these PIP boxes may exist in plurality within a fusion polypeptide of the present invention. Examples of the number of PIP boxes contained in the fusion polypeptide include, but not particularly limited to from 1 to 6, and preferably from 2 to 4. These plural PIP boxes may each have amino acid sequences different from each other, so long as they play their roles. In addition, between the plural PIP boxes themselves, other amino acid sequences, for example, a linker peptide mentioned later may be inserted.

Further, a "linker peptide" may be present at a C-terminal side of the above PIP box. The term "linker peptide" constituting the fusion polypeptide of the present invention refers to a peptide which is inserted between polypeptides that are fused together or between a peptide and a polypeptide in the fusion polypeptide of the present invention in order to avoid the inhibition of their functions or folding. The length of the linker peptide includes, but not particularly limited to, peptides of from 3 to 100 amino acids, preferably 5 to 50 amino acids. The kinds of the amino acids constituting the linker peptide are not particularly limited, and it is better to avoid a linker which itself forms a complicated conformation, and a peptide with a relatively small side chain richly containing amino acids, for example, serine or glycine, is well used. It is preferable that the linker peptide in the present invention is amino acids composed of serine and glycine.

The "polypeptide having a DNA polymerase activity" is present at a C terminal of the above linker peptide. As the "polypeptide having a DNA polymerase activity" which constitutes the fusion polypeptide of the present invention, a known DNA polymerase or a variant thereof can be used. When the fusion polypeptide of the present invention is used in PCR, a thermostable DNA polymerase and a variant thereof, preferably a thermostable family A (Pol I-type) DNA polymerase and a variant thereof, and more preferably a DNA polymerase derived from bacteria of the genus *Thermus* or a variant thereof is used as a "polypeptide having a DNA polymerase activity." According to the present invention, the performance of the Taq DNA polymerase can be dramatically improved, even though the present invention is not particularly limited thereby. In the present invention, "Taq polymerase" or "Taq DNA polymerase" refers to a Pol I-type DNA polymerase derived from *Thermus aquaticus.* The amino acid sequence of this DNA polymerase and the nucleotide sequence encoding the amino acid sequence are each shown as SEQ ID NOs: 1 and 2 which is a part of the present specification. Further, a Pol I-type DNA polymerase from *Thermus thermophilus* or *Thermus flavus* can be also used in the present invention. In the present specification, examples of the polypeptide having a DNA polymerase activity include a full-length polypeptide of a Pol I-type DNA polymerase or a fragment thereof, and preferably a full-length polypeptide of a Taq DNA polymerase or a fragment thereof. Here, the fragments of these polymerases may be a natural form or a variant form, so long as they have a DNA polymerase activity. In addition, the fragments of these polymerases may be fragments of polymerases not having a PIP box in a natural form, or may be fragments of polymerases having a PIP box in a natural form. Further, in a case of a fragment of a polymerase having a PIP box in a natural form, the PIP box may be removed.

Examples of one embodiment of the present invention are a fusion polypeptide containing a PIP box derived from a replication factor C large subunit from *P. furiosus* and Taq DNA polymerase. The amino acid sequences of the fusion polypeptides are shown in SEQ ID NOs: 4, 6, 8, 10, 12, 20, 22, 24, and 26.

It is preferable that the fusion polypeptide of the present invention has a dissociation constant (Kd) from a PCNA preferably within the range of from 1 × 10⁻⁸ to 25 × 10⁻⁷ M, preferably from 3 × 10⁻⁸ to 15 × 10⁻⁷ M, and more preferably from 5 × 10⁻⁸ to 10 × 10⁻⁷ M.

Further, the fusion polypeptide of the present invention can amplify a long-strand DNA, as compared to polypeptides having a DNA polymerase activity not having a peptide which binds to a PCNA. Especially, the fusion polypeptide can amplify a DNA having a length of 8 kb or more, preferably 12 kb or more, and more preferably 15 kb or more. Therefore, it can be said that the fusion polypeptide of the present invention is a DNA polymerase having excellent extensibility. Further, the fusion polypeptide of the present invention can amplify a DNA in a short time, as compared to a polypeptide having a DNA polymerase activity not having a peptide which binds to a PCNA. In other words, the fusion polypeptide of the present invention is a DNA polymerase having excellent speediness. Concretely, the fusion polypeptide of the present invention, as compared to a polypeptide before fusion, can shorten the time period that is required for DNA extension. For this reason, the fusion polypeptide is very useful in reactions for amplifying nucleic acids in which the DNA extension time is set shorter than a conventional method. Further, since the DNA extension time for each cycle can be shortened, it is possible to shorten the entire required time for the method for amplifying nucleic acids than a conventional method. For example, it is possible to amplify a DNA having a length of 8 kb by carrying out 30 cycles of shuttle PCR, wherein one cycle is 99°C for 5 seconds and 66°C for 4 minutes. It is possible to amplify a DNA having a length of preferably 12 kb or more, and more preferably 15 kb or more by carrying out 30 cycles of shuttle PCR, wherein one cycle is 99°C for 5 seconds and 66°C for 12 minutes.

### (2) Nucleic Acid Encoding Fusion Polypeptide of the Present Invention

The present invention provides a nucleic acid encoding a fusion polypeptide as defined in the above (1). A nucleic acid of the present invention is incorporated into a recombinant vector, whereby a fusion polypeptide of the present invention can be produced in accordance with a method well known to the person skilled in the art. In the nucleotide sequence of the nucleic acid of the present invention, a nucleotide may be substituted so as to have an optimal codon in expression of the fusion polypeptide in the host cells. Next, a nucleic acid of the present invention is inserted downstream of a promoter of an appropriate expression vector to generate an expression vector. It is necessary to incorporate the above nucleic acid into a vector so that a fusion polypeptide of the present invention is expressed in a host, and the vector can contain, besides the promoter, a ribosome-binding sequence (e.g., SD sequence: Shine-Dalgarno sequence), a cis element such as a terminator or an enhancer, a selection marker (e.g., dihydrofolate reductase gene, ampicillin-resistant gene, neomycin-resistant gene) or the like. The transformant capable of producing a fusion polypeptide of the present invention can be obtained by transducing an expression vector mentioned above to a host cell.

Also, the nucleic acid of the present invention mentioned above may further contain a nucleic acid encoding an affinity tag in order to facilitate purification of a protein expressed. The nucleic acid encoding an affinity tag is, for example, a nucleic acid encoding histidine (His) tag, a glutathione S-transferase (GST) tag, a maltose binding protein (MBP) tag, a Strep(II) tag consisting of eight amino acid residues (Trp-Ser-His-Pro-Gln-Phe-Glu-Lys), and the like, without intending to limit the present invention thereto. The position at which the tag is added may be either one of a 5'-terminal side or a 3'-terminal side of a nucleic acid encoding a fusion polypeptide of the present invention, and the tag may be properly added at a position that would not be a hindrance to expression and tag functions. Here, it is preferable that the tag can be cleaved in the purification stage of the expressed protein.

As the expression vector, a vector capable of autonomous replication or a vector capable of being incorporated into a host chromosome can be used. As the vector, for example, a plasmid vector, a phage vector, a virus vector or the like can be used. As the plasmid vector, a plasmid which is suitable for a host to be used, for example, a plasmid derived from *Escherichia coli,* a plasmid derived from bacteria of the genus *Bacillus,* and a plasmid derived from yeasts are well known to the person skilled in the art, and many of them are commercially available. In the present invention, these known plasmids or modified forms thereof can be used. As the phage vector, for example, a λ phage (e.g., Charon4A, Charon21A, EMBL3, EMBL4, λgt10, λgt11, λZAP) or the like can be used, and as the virus vector, for example, an animal virus such as a retrovirus or a vaccinia virus, or an insect virus such as a baculovirus can be used.

As host cells, any one of prokaryotic cells, yeasts, animal cells, insect cells, plant cells, and the like can be used so long as the fusion polypeptide of the present invention can be expressed.

When a prokaryotic cell is used as a host cell, for example, bacteria belonging to the *Escherichia* genus such as *Escherichia coli,* bacteria belonging to the *Bacillus* genus such as *Bacillus subtilis,* bacteria belonging to the *Pseudomonas* genus such as *Pseudomonas putida,* bacteria belonging to the *Rhizobium* genus such as *Rhizobium meliloti* can be used as host cells. *Escherichia coli* which can be used in the production of heterologous proteins is well known to the person skilled in the art, and many of them are commercially available (e.g., *Escherichia coli* BL21, *E. coli* XL1-Blue, *E. coli* XL2-Blue, *E. coli* DH1, *E. coli* JM109, *E. coli* HB101, etc.). Also, *Bacillus subtilis* MI114, *B. subtilis* 207-21 or the like, which is a bacterium belonging to the *Bacillus* genus, or *Brevibacillus choshinensis* or the like, which is a bacterium belonging to the *Brevibacillus* genus, has been known as a host for production of heterologous protein. These host cells can be combined with an appropriate expression vector and used in the production of a fusion polypeptide of the present invention. In this case, a promoter which is carried on an expression vector can be selected depending upon a host, and, for example, in *Escherichia coli,* a promoter derived from *Escherichia coli,* a phage etc., such as a trp promoter, a lac promoter, a PL promoter, or a PR promoter, or a modified product thereof can be used, without intending to limit to those mentioned above. Further, an expression system (e.g., pET expression system, etc.) in which a promoter derived from a phage and an RNA polymerase gene are combined may be utilized. Further, a heterologous protein expression system in which an yeast, an insect cell or a mammalian cell is used as a host has been numerously constructed, and has already been commercially available. In the production of a fusion polypeptide of the present invention, these expression systems may be used.

The method for introducing an expression vector into a host is not particularly limited, so long as the method is capable of introducing a nucleic acid into a host, and, for example, a method using calcium ions, an electroporation method, a spheroplast method, a lithium acetate method or the like can be used. The method for introducing a recombinant vector into an insect cell is not particularly limited, so long as the method is capable of introducing a DNA into an insect cell, and, for example, a calcium phosphate method, a lipofection method, an electroporation method, or the like can be used. The infection of a phage vector or a virus vector to a host cell is carried out in accordance with a method depending on these vectors, whereby a transformant which expresses a fusion polypeptide of the present invention can be obtained.

A transformant into which an expression vector incorporated with a DNA encoding a fusion polypeptide of the present invention is transduced is cultured. The transformant can be cultured in accordance with an ordinary method usable in the cultivation of host cells. Depending upon the kinds of the promoters carried on an expression vector, appropriate induction procedures (addition of an inducer or modification of culture temperature) are carried out.

The fusion polypeptide of the present invention can be collected from a cultured product of the transformant. Here, the term "cultured product" includes all of culture supernatant, cultured cells, cultured bacteria, disruptions of cells or bacteria. When the fusion polypeptide of the present invention is accumulated in the cells of the transformant, a cultured product is centrifuged to harvest the cells, the cells are washed, and the cells are then disrupted to extract an intended protein, to provide a starting material for purification. When the fusion polypeptide of the present invention is secreted outside of the cells of the transformant, a cultured product is used directly, or culture supernatant obtained by removing the cells from a cultured product by centrifugation or the like is used as a starting material for purification. The fusion polypeptide of the present invention can be purified from the above starting material by solvent extraction, salting out with ammonium sulfate or the like, precipitation with an organic solvent, various chromatographies (ion exchange chromatography, hydrophobic chromatography, gel filtration, affinity chromatography or the like) or the like.

### (3) Method for Amplifying Nucleic Acids Using Fusion Polypeptide of the Present Invention

The fusion polypeptide of the present invention can be used in amplifying long-strand length nucleic acids and amplifying nucleic acids with shortened reaction time period by combining the fusion polypeptide with a PCNA. The method for amplifying nucleic acids of the present invention can be used for any one of isothermal nucleic acid amplification method or temperature-changing nucleic acid amplification method. Any one of polymerase chain reaction (PCR), ligase chain reaction, MALBAC (Multiple Annealing and Looping-Based Amplification Cycles) method, MDA (Multiple Displacement Amplification) method, strand displacement DNA extension reaction (strand displacement amplification: SDA), rolling circle amplification (RCA) method, cross priming amplification method, loop-mediated isothermal amplification (LAMP) method, ICAN (isothermal and chimeric primer-initiated amplification of nucleic acids) method, or the like can be suitably used, without being particularly limited thereto. For example, a combination of the fusion polypeptide of the present invention with a PCNA can be expected to have high extensibility, so that it is effective in the preparation of a DNA having a long strand length for genome analysis or genome editing, and can be utilized in isothermal nucleic acid amplification method. Also, a combination of the fusion polypeptide of the present invention and a PCNA has excellent high-speed synthesis of DNA strand for improving PCR, so that it can be used for amplification of longer DNA with a shortened reaction time period. Although the present invention is not particularly limited thereto, a PCNA into which a mutation so as to lower stability of a ring-shaped structure is transduced is suitable to be combined with the fusion polypeptide of the present invention.

The PCNA used in the present invention includes a known PCNA or a variant thereof, and preferably a thermostable PCNA or a variant thereof is used. Examples are PCNA from *P. furiosus* or PCNA from *T. kodakarensis,* and the like, without particularly being limited thereto. Further, a variant PCNA can be also used in a composition for amplifying nucleic acids of the present invention. Examples of the variant PCNA are, for example, variant PCNAs described in International Publication Pamphlet WO 2007/004654, concretely variant PCNAs having a sequence in which an amino acid residue at 82nd, 84th, 109th, 139th, 143rd, or 147th position of PCNA from *P. furiosus* is substituted with another amino acid. An example of an especially preferred embodiment of the present invention is a variant PCNA having a sequence in which an amino acid residue at 143rd position is substituted from aspartic acid to asparagine (D143R). The variant PCNA of this embodiment exhibits especially excellent auxiliary actions with well-balanced extensibility and reaction speed of the DNA replication reaction, as shown in Examples set forth below.

Also, in the method for amplifying nucleic acids of the present invention, the fusion polypeptide of the present invention may be combined with a DNA polymerase that is different from that of the fusion polypeptide of the present invention. For example, a fusion polypeptide of the present invention which is generated using a Pol I-type DNA polymerase may be combined with an α-type DNA polymerase having 3'→5' exonuclease activity and used in the method for amplifying nucleic acids. Here, a technique of performing PCR with a reaction solution containing two kinds of DNA polymerases having different 3'→5' exonuclease activities has been known as LA-PCR (Long and Accurate PCR). Further, a combination of two kinds of fusion polypeptides of the present invention having different polypeptides having DNA polymerase activities may be used.

In addition, the oligonucleotide usable as a primer in the method for amplifying nucleic acids of the present invention has a sequence complementary to a nucleotide sequence of a nucleic acid used as a template, and the oligonucleotide is not particularly limited, so long as it hybridizes to a nucleic acid used as a template in the reaction conditions used. The strand length of the primer is preferably 6 nucleotides or more, and more preferably 10 nucleotides or more, from the viewpoint of specificity of hybridization, and the strand length is preferably 100 nucleotides or less, and more preferably 30 nucleotides or less, from the viewpoint of synthesis of the oligonucleotide. The above oligonucleotide can be chemically synthesized, for example, by a known method. In addition, the oligonucleotide may be an oligonucleotide derived from an organism sample, and, for example, an oligonucleotide may be prepared by isolating from a restriction endonuclease digest of a DNA prepared from a natural sample.

Furthermore, the method for amplifying nucleic acids of the present invention may be combined with a real-time detection technique. In the real-time detection, using an intercalator or a fluorescent-labeled probe, an amplified product is detected with the passage of time, concurrently with the amplification reaction. The intercalator includes SYBR(registered trademark) Green I and other nucleic acid-bindable pigments, and the fluorescent-labeled probe includes TaqMan(registered trademark) probe, CyCleave(registered trademark) probe, or molecular beacon probe, and the like, respectively.

### (4) Composition for Amplifying Nucleic Acids Containing Fusion Polypeptide of the Present Invention

The fusion polypeptide obtained by the present invention can be used as a component for a composition for amplifying nucleic acids which can be used in the above (3) Method for Amplifying Nucleic Acids. Further, the composition for amplifying nucleic acids may contain elements essential for the activity of a DNA polymerase, for example, a divalent metal salt (magnesium salt, etc.), dNTP, buffering components for maintaining a pH, and the like.

Preferably, the composition for amplifying nucleic acids of the present invention can further contain a PCNA, in addition to the fusion polypeptide of the present invention. As the PCNA contained in the composition for amplifying nucleic acids, the PCNA explained in the above (3) or a variant thereof can be used.

Also, the composition of the present invention may contain a DNA polymerase which is different from that of the fusion polypeptide of the present invention. For example, the fusion polypeptide of the present invention generated by using a Pol I-type DNA polymerase may be combined with an α-type DNA polymerase having a 3'→5' exonuclease activity to prepare a composition.

Examples of the divalent metal ions constituting the divalent metal salt contained in the composition of the present invention include magnesium ions, manganese ions, and cobalt ions. The divalent metal ions that are suitable for each of the DNA polymerases and concentrations thereof have been known in the art. The divalent metal ions can be supplied in the form of salts such as chlorides, sulfates, or acetates. Examples of the divalent metal ion concentration in the composition of the present invention are, for example, from 0.5 to 20 mM without particularly limiting the present invention thereto.

In the present invention, at least one member of dNTP, namely deoxyribonucleotide triphosphate (e.g., dATP, dCTP, dGTP, and dTTP) and derivatives thereof is used. Examples of the deoxyribonucleotide triphosphate contained in the composition of the present invention are preferably a mixture of four kinds dATP, dCTP, dGTP, and dTTP.

Also, the composition of the present invention may contain a buffering component. The component refers to, for example, a compound or a mixture having an action of moderating the fluctuations of a hydrogen ion concentration (pH) of a reaction solution, without particularly being limited thereto. In general, a mixed solution of a weak acid and a salt thereof or a weak base and a salt thereof has a strong buffering action, and is widely used for the purpose of a pH control as a reaction buffering agent. The pH of the composition of the present invention is appropriately set within an ordinary range for performing PCR, for example, within a pH range of from 8.0 to 9.5, without particularly limiting the present invention thereto.

Further, the composition of the present invention may contain a component for real-time detection. The composition can be combined with an intercalator or a fluorescent-labeled probe, without particularly being limited thereto.

### (5) Kit Containing Fusion Polypeptide of the Present Invention

The kit containing a fusion polypeptide of the present invention is one embodiment of the present invention. Preferably, examples of the kit include a kit further containing a PCNA, in addition to a fusion polypeptide described in the above (1). In particular, the preferable PCNA contained in the kit includes the above-described variant PCNA in which a ring-shaped structure is made labile. The kit of the present invention may further contain a component usable in the preparation of a composition for amplifying nucleic acids of the present invention, such as a divalent metal salt (magnesium salts, etc.), dNTP, or a buffering component for maintaining a pH, as an individual component, or the kit may contain a component in which plural of these components are combined and prepared.

Further, a component for real-time detection may be contained as a component. Examples include an intercalator, a fluorescent-labeled probe, and the like, without particularly being limited thereto.

### (6) Method for Producing DNA Complementary to Template DNA of the Present Invention

A composition containing a fusion polypeptide of the present invention and a PCNA can be used in a method for producing a DNA complementary to a template DNA. In the method for producing a DNA, the above (3) Method for Amplifying Nucleic Acids can be utilized. By utilizing a DNA produced by these nucleic acid amplification methods, it is possible to determine a nucleotide sequence of a target nucleic acid, to label a target nucleic acid, and to introduce a site-directed mutation to a target nucleic acid.

### EXAMPLES

The present invention will be specifically described hereinbelow by the Examples, without intending to limit the scope of the present invention thereto.

### Example 1 Purification of Taq DNA Polymerase to Which PIP Box Was Added

To an N-terminal or a C-terminal of a Taq DNA polymerase having the amino acid sequence shown in SEQ ID NO: 1 of the Sequence Listing was added a PIP box of replication factor C large subunit from *Pyrococcus furiosis* (hereinafter simply PfuRFCL) having the amino acid sequence shown in SEQ ID NO: 3 of the Sequence Listing via a linker peptide. The Taq DNA polymerases to which a PIP box was added to an N-terminal were five kinds of Taq81 to Taq85 (PIP-L5-Taq, PIP-L10-Taq, PIP-L15-Taq, PIP-L35-Taq, and PIP-L47-Taq), and the Taq DNA polymerases to which a PIP box was added to a C-terminal were three kinds of Taq92 to Taq94 (Taq-L5-PIP, Taq-L10-PIP, and Taq-L15-PIP). Further, a Taq DNA polymerase to which two to five PIP boxes were added to an N-terminal was prepared. Concretely, they were four kinds of Taq95 (PIP-L14-PIP-L15-Taq), Taq96 (PIP-L14-PIP-L14-PIP-L15-Taq), Taq97 (PIP-L14-PIP-L14-PIP-L14-PIP-L15-Taq), and Taq98 (PIP-L14-PIP-L14-PIP-L14-PIP-L14-PIP-L15-Taq). The names and structures of the Taq DNA polymerases to which PIP box or boxes were added in the present specification are shown in Table 2. The number following "L" in the column of "Structure" in Table 2 shows the length of a linker peptide (number of amino acid residues) composed of repeats of serine residues and glycine residues.

### [Table 2]

**Table 2**

| Name | Structure | Amino Acid Sequence | Nucleotide Sequence |
|---|---|---|---|
| Taq81 | PIP-L5-Taq | SEQ ID NO: 4 | SEQ ID NO: 5 |
| Taq82 | PIP-L10-Taq | SEQ ID NO: 6 | SEQ ID NO: 7 |
| Taq83 | PIP-L 15-Taq | SEQ ID NO: 8 | SEQ ID NO: 9 |
| Taq84 | PIP-L35-Taq | SEQ ID NO: 10 | SEQ ID NO: 11 |
| Taq85 | PIP-L47-Taq | SEQ ID NO: 12 | SEQ ID NO: 13 |
| Taq92 | Taq-L5-PIP | SEQ ID NO: 14 | SEQ ID NO: 15 |
| Taq93 | Taq-L10-PIP | SEQ ID NO: 16 | SEQ ID NO: 17 |
| Taq94 | Taq-L 15-PIP | SEQ ID NO: 18 | SEQ ID NO: 19 |
| Taq95 | PIP-L14-PIP-L15-Taq | SEQ ID NO: 20 | SEQ ID NO: 21 |
| Taq96 | PIP-L14-PIP-L14-PIP-L15-Taq | SEQ ID NO: 22 | SEQ ID NO: 23 |
| Taq97 | PIP-L14-PIP-L14-PIP-L14-PIP-L15-Taq | SEQ ID NO: 24 | SEQ ID NO: 25 |
| Taq98 | PIP-L14-PIP-L14-PIP-L14-PIP-L14-PIP-L15-Taq | SEQ ID NO: 26 | SEQ ID NO: 27 |

### (1) Taq81 (PIP-L5-Taq) Expression Plasmid

Using a Taq DNA polymerase gene having the nucleotide sequence shown in SEQ ID NO: 2 of the Sequence Listing as a template, PCR was carried out with a TaqNPIP-5 primer having the nucleotide sequence shown in SEQ ID NO: 28 of the Sequence Listing and a Taq-3 primer having the nucleotide sequence shown in SEQ ID NO: 29 of the Sequence Listing.

As the enzyme for PCR, KOD Plus Neo DNA polymerase (manufactured by TOYOBO CO, LTD.) was used, and the conditions for PCR were 30 cycles of reaction, wherein one cycle is 95°C for 30 seconds, 55°C for 30 seconds, and 72°C for 3 minutes. An amplified fragment was purified by agarose gel electrophoresis, and thereafter cleaved with restriction enzymes NdeI (manufactured by TAKARA BIO INC.) and NotI (manufactured by TAKARA BIO INC.). This fragment was ligated with pET21a (manufactured by Novagen) which was cleaved with the same restriction enzymes. *Escherichia coli* JM109 strain (manufactured by TAKARA BIO INC.) was transformed with the ligation product, and spread on an LB-ampicillin plate. A plasmid was purified from the colonies formed, nucleotide sequences were read off, and it was confirmed that the nucleotide sequence of SEQ ID NO: 5 was contained. This plasmid was named pTaq81.

### (2) Taq82 (PIP-L10-Taq) Expression Plasmid

A DNA (SEQ ID NO: 7) encoding a polypeptide in which five amino acids were inserted between PIP box and a linker peptide of Taq81 was generated by a site-directed mutagenesis using a QuickChange site-directed mutagenesis kit (manufactured by Agilent Technologies).

Using pTaq81 as a template and primers of taqN10-5 and taqN10-3 having the nucleotide sequences shown in SEQ ID NOs: 30 and 31 of the Sequence Listing, respectively, 14 cycles of PCR were carried out, wherein one cycle is 95°C for 30 seconds, 55°C for 60 seconds, and 68°C for 8 minutes. Subsequently, *Escherichia coli* JM109 strain was transformed with 1 µL of a reaction solution digested with DpnI, and the transformed cells were spread on an LB-ampicillin plate. A plasmid was purified from the colonies formed, nucleotide sequences were read off, and it was confirmed that the nucleotide sequence of SEQ ID NO: 7 was contained. This plasmid was named pTaq82.

### (3) Taq83 (PIP-L15-Taq) Expression Plasmid

Taq83 (PIP-L15-Taq) expression plasmid in which a sequence corresponding to 10 amino acids was inserted between PIP box and a linker peptide of Taq81 was constructed according to the mutagenesis method described in Example 1(2).

In this mutagenesis, PCR was carried out with pTaq81 as a template, and changing the used primers to taqN15-5 and taqN15-3 having the nucleotide sequences shown in SEQ ID NOs: 32 and 33 of the Sequence Listing, respectively, and a plasmid containing the nucleotide sequence of SEQ ID NO: 9 was obtained. This plasmid was named pTaq83.

### (4) Taq84 (PIP-L35-Taq) Expression Plasmid

A Taq84 (PIP-L35-Taq) expression plasmid in which a sequence corresponding to 20 amino acids was inserted between PIP box and a linker peptide of Taq83 was constructed according to the mutagenesis method described in Example 1(2).

In this mutagenesis, PCR was carried out with pTaq83 as a template, and two primers taq-plus20-5 and taq-plus20-3 having the nucleotide sequences shown in SEQ ID NOs: 34 and 35 of the Sequence Listing, respectively, and a plasmid containing the nucleotide sequence of SEQ ID NO: 11 was obtained. This plasmid was named pTaq84.

### (5) Taq85 (PIP-L47-Taq) Expression Plasmid

A Taq85 (PIP-L47-Taq) expression plasmid in which a sequence corresponding to 12 amino acids was inserted between 14th and 15th amino acids of a linker peptide of Taq84 was constructed according to the mutagenesis method described in Example 1(2).

In this mutagenesis, PCR was carried out with pTaq84 as a template, and two primers of Taq-plus12-5 and Taq-plus12-3 having the nucleotide sequences shown in SEQ ID NOs: 36 and 37 of the Sequence Listing, respectively, and a plasmid containing the nucleotide sequence of SEQ ID NO: 13 was obtained. This plasmid was named pTaq85.

### (6) Taq92 (Taq-L5-PIP) Expression Plasmid

Using a Taq DNA polymerase gene having the nucleotide sequence shown in SEQ ID NO: 2 of the Sequence Listing as a template, and two primers Taq-5 and Tq-L5-PIP-3 having the nucleotide sequences shown in SEQ ID NOs: 38 and 39 of the Sequence Listing, respectively, PCR, ligation, transformation, and plasmid purification were carried out according to the procedures described in Example 1(1). The plasmid thus obtained containing the nucleotide sequence of SEQ ID NO: 15 was named pTaq92.

### (7) Taq93 (Taq-L10-PIP) Expression Plasmid

Using a Taq DNA polymerase gene having the nucleotide sequence shown in SEQ ID NO: 2 of the Sequence Listing as a template, and two primers Taq-5 and Tq-L10-PIP-3 having the nucleotide sequences shown in SEQ ID NOs: 38 and 40 of the Sequence Listing, respectively, PCR, ligation, transformation, and plasmid purification were carried out under the conditions described in Example 1(1). The plasmid thus obtained containing the nucleotide sequence of SEQ ID NO: 17 was named pTaq93.

### (8) Taq94 (Taq-L15-PIP) Expression Plasmid

Using a Taq DNA polymerase gene having the nucleotide sequence shown in SEQ ID NO: 2 of the Sequence Listing as a template, and two primers Taq-5 and Tq-L15-PIP-3 having the nucleotide sequences shown in SEQ ID NOs: 38 and 41 of the Sequence Listing, respectively, PCR, ligation, transformation, and plasmid purification were carried out under the conditions described in Example 1(1). The plasmid thus obtained containing the nucleotide sequence of SEQ ID NO: 19 was named pTaq94.

### (9) Taq95 (PIP-L14-PIP-L15-Taq) Expression Plasmid

A Taq95 (PIP-L 14-PIP-L15-Taq) expression plasmid in which a sequence corresponding to PIP boxes and the linker peptides was inserted between PIP box and a linker peptide of Taq83 was constructed according to the mutagenesis method described in Example 1(2).

In this mutagenesis, PCR was carried out with pTaq83 as a template, and two primers Taq95-5 and Taq-3 having the nucleotide sequences shown in SEQ ID NOs: 48 and 29 of the Sequence Listing, respectively, and a plasmid containing the nucleotide sequence of SEQ ID NO: 21 was obtained. This plasmid was named pTaq95.

### (10) Taq96 (PIP-L14-PIP-L14-PIP-L15-Taq) Expression Plasmid

A Taq96 (PIP-L14-PIP-L14-PIP-L15-Taq) expression plasmid in which a sequence corresponding to PIP boxes and the linker peptides was inserted between PIP box and a linker peptide of Taq95 was constructed according to the mutagenesis method described in Example 1(2).

In this mutagenesis, PCR was carried out with pTaq95 as a template, and two primers Taq96-5 and Taq-3 having the nucleotide sequences shown in SEQ ID NOs: 49 and 29 of the Sequence Listing, respectively, and a plasmid containing the nucleotide sequence of SEQ ID NO: 23 was obtained. This plasmid was named pTaq96.

### (11) Taq97 (PIP-L14-PIP-L14-PIP-L14-PIP-L15-Taq) Expression Plasmid

A Taq97 (PIP-L14-PIP-L14-PIP-L14-PIP-L15-Taq) expression plasmid in which a sequence corresponding to PIP boxes and the linker peptides was inserted between PIP box and a linker peptide of Taq96 was constructed according to the mutagenesis method described in Example 1(2).

In this mutagenesis, PCR was carried out with pTaq96 as a template, and two primers Taq97-5 and Taq-3 having the nucleotide sequences shown in SEQ ID NOs: 50 and 29 of the Sequence Listing, respectively, and a plasmid containing the nucleotide sequence of SEQ ID NO: 25 was obtained. This plasmid was named pTaq97.

### (12) Taq98 (PIP-L14-PIP-L14-PIP-L14-PIP-L14-PIP-L15-Taq) Expression Plasmid

A Taq98 (PIP-L14-PIP-L14-PIP-L14-PIP-L14-PIP-L15-Taq) expression plasmid in which a sequence corresponding to PIP boxes and the linker peptides was inserted between PIP box and a linker peptide of Taq97 was constructed according to the mutagenesis method described in Example 1(2).

In this mutagenesis, PCR was carried out with pTaq97 as a template, and two primers Taq98-5 and Taq-3 having the nucleotide sequences shown in SEQ ID NOs: 51 and 29 of the Sequence Listing, respectively, and a plasmid containing the nucleotide sequence of SEQ ID NO: 27 was obtained. This plasmid was named pTaq98.

### (13) Preparation of Taq DNA Polymerase to Which PIP Box Was Added

*Escherichia coli* BL21-CodonPlus (DE3)-RIL (manufactured by Agilent Technologies) was transformed with each of the expression plasmids pTaq81 to pTaq85 and pTaq92 to pTaq94, and the transformant obtained was subjected to shaking culture in 1 L of an LB medium containing 50 µg/mL ampicillin and 34 µg/mL chloramphenicol. At a point where OD600 reached 0.2 to 0.3, IPTG was added so as to have a final concentration of 1 mM, and expression of a Taq DNA polymerase was induced. Thereafter, the reaction solution was further subjected to a shaking culture at 25°C for about 18 hours. After culture, the cultured bacteria were harvested, and the cultured bacteria were washed with a PBS solution (150 mM NaCl, 20 mM Na₂HPO₄, 2 mM NaH₂PO₄, at pH 7.5). Thereafter, the cultured bacteria were again harvested and stored at -80°C.

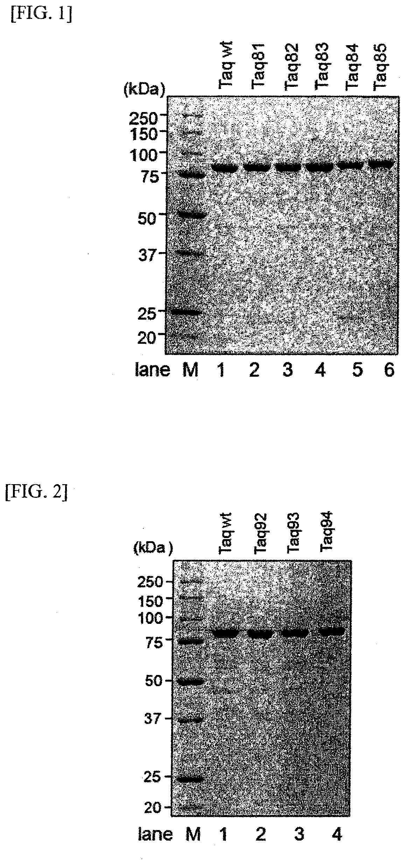

To the frozen cultured bacteria was added 20 mL of a solution A (50 mM Tris-HCl, 1 mM EDTA, pH 8.0) containing 1 mM PMSF (manufactured by nacalai tesque), and the mixture was subjected to ultrasonic disruption (on for 10 seconds, off for 10 seconds/on for a total of 10 minutes) on ice. The lysate was centrifuged (23,708 × g) at 4°C for 10 minutes, and the supernatant obtained was heat-treated at 75°C for 30 minutes, and the heat-treated mixture was again centrifuged (23,708 × g) at 4°C for 10 minutes. NaCl was added to the supernatant obtained so as to have a final concentration of 1 M, and further a 5% (w/v) polyethyleneimine solution (pH 8.0) was added thereto so as to have a final concentration of 0.15%, and the mixture was allowed to stand on ice for 20 minutes. Thereafter, the mixture was centrifuged (23,708 × g) at 4°C for 10 minutes. Ammonium sulfate was gradually added to the supernatant obtained at a low temperature so as to be 80% saturation, the mixture was allowed to stand overnight at 4°C, and centrifuged (23,708 × g) at 4°C for 10 minutes. The precipitates obtained were suspended in a solution B (50 mM Tris-HCl, 10% glycerol, pH 8.0) containing 1 M ammonium sulfate, and the suspension was subjected to a hydrophobic column HiTrap Phenyl HP 5 mL (manufactured by GE Healthcare) using AKTA Explorer (manufactured by GE Healthcare) to elute the protein by a 1 M to 0 M ammonium sulfate concentration gradient using the solution B. The eluted fraction obtained was dialyzed against a solution C (50 mM Tris-HCl, 50 mM NaCl, pH 8.0) overnight, the dialyzed solution was then subjected to affinity column HiTrap Heparin HP 5 mL (manufactured by GE Healthcare) to elute the protein by a 50 mM to 1 M sodium chloride concentration gradient, and this eluted fraction was used as a final purification product. The final purification product was subjected to 10% SDS-PAGE, and detected by CBB staining. The results are shown in FIGs. 1 and 2.

As shown in FIGs. 1 and 2, Taq DNA polymerase variants (Taq81 to Taq85 and Taq92 to Taq94), fusion proteins in which the wild-type Taq DNA polymerase and PIP box were fused, could be purified in a single band. Also, Taq95 to Taq98 could be purified in a single band in the same manner.

### Example 2 Physical Interaction Analysis Using SPR Method

### (1) Method

In the surface plasmon resonance (SPR) analysis, BIAcore J (manufactured by BIACORE) was used. A PCNA from *Pyrococcus furiosus* (J. Bacteriology, 181, 6591-6599, 1999: hereinafter referred to as PfuPCNA) was immobilized on a CM5 sensor chip (manufactured by GE Healthcare) by amine coupling, and the measurement was carried out under the conditions of 25°C, a flow rate of 30 µL/minutes, and a solution E (10 mM Tris-HCl, 50 mM KCl, 1.5 mM MgCl₂, pH 8.3). A sample solution of Taq81 to Taq85 diluted to 10, 20, 40, 80, 160, or 320 nM or a sample solution of Taq94 diluted to 125 nM, 250 nM, 500 nM, 1 µM, or 2 µM was each added for 2 minutes. The sensorgrams obtained were analyzed by BIA evaluation program, and the dissociation constants (Kd) between Taq81 to Taq85 and PfuPCNA and between Taq94 and PfuPCNA were calculated.

### (2) Results

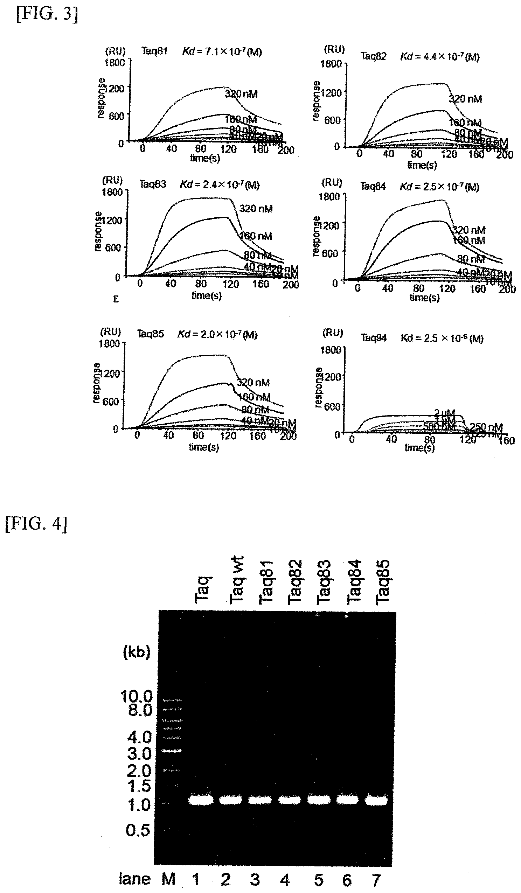

The results for the SPR analysis are shown in FIG. 3. The dissociation constants (Kd) with respect to the bindings between five kinds of Taq DNA polymerases to which PIP box was added at an N-terminal, each having a different length of a linker peptide (Taq81, Taq82, Taq83, Taq84, and Taq85), and PfuPCNA were calculated. As a result, the values were 7.1 × 10⁻⁷ M, 4.4 × 10⁻⁷ M, 2.4 × 10⁻⁷ M, 2.5 × 10⁻⁷ M, and 2.0 × 10⁻⁷ M, which were nearly of the same level regardless of the lengths of the linker peptides. The above results show that the strength of the interactions between the Taq DNA polymerase to which PIP box was added at an N-terminal and the PfuPCNA was nearly the same regardless of the length of the linker peptides.

On the other hand, the dissociation constant (Kd) between Taq94 and PfuPCNA was calculated to be 2.5 × 10⁻⁶ M. This is about 10 times of the dissociation constants of Taq81 to Taq85, and it could be confirmed from the results that the interaction was weak between the Taq DNA polymerase to which PIP box was added at a C-terminal and the PfuPCNA.

### Example 3 DNA Amplification by PCR

### (1) Amplification of 1 kb DNA

Using Taq81 to Taq85 prepared in Example 1 (13), PCR was carried out with a lambda DNA as a template. The reaction solution composition was 1 nM Taq81 to Taq85, 10 mM Tris-HCl (pH 8.3), 50 mM KCl, 1.5 mM MgCl₂, 0.2 mM dNTP, and 1 ng lambda DNA, and 0.4 µM of each primer, and a final volume of the reaction solution was 50 µL. The reaction was carried out in 30 cycles, wherein one cycle is 95°C for 30 seconds, 55°C for 30 seconds, and 72°C for 60 seconds.

In this PCR, two primers F1 and R2-2 having the nucleotide sequences shown in SEQ ID NOs: 42 and 43 of the Sequence Listing, respectively, were used. A product obtained was separated by 1% agarose gel, and stained with ethidium bromide. As a result, as shown in FIG. 4, all of a commercially available Taq DNA polymerase (lane 1), a wild-type Taq DNA polymerase (lane 2), and Taq81 to Taq85 (lanes 3 to 7) showed a band at a position of 1 kb. From the above results, it could be confirmed that the PIP box and the linker peptide added at an N-terminal do not affect PCR.

### (2) Amplification of 8 kb DNA

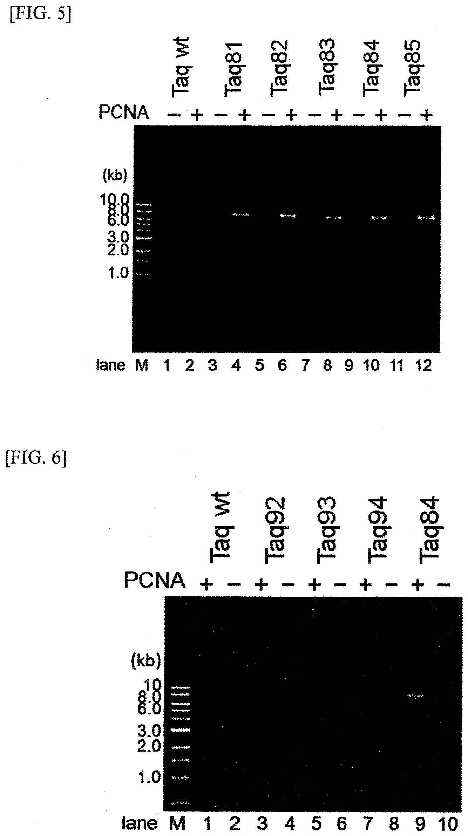

Next, PCR was carried out in the absence and in the presence of a PCNA. The reaction solution composition was 1 nM wild-type Taq DNA polymerase or Taq81 to Taq85, or 40 nM PufPCNA D143R variant (PCNA 13) prepared by a method described in Examples of International Publication No. WO 2007/004654, 10 mM Tris-HCl (pH 8.3), 50 mM KCl, 1.5 mM MgCl₂, 0.2 mM dNTP, 1 ng of lambda DNA, 0.4 µM of each primer, and a final volume of the reaction solution was 50 µL. First, a reaction solution was incubated at 95°C for 1 minute, and thereafter 30 cycles of shuttle PCR were carried out, wherein one cycle is 99°C for 5 seconds, and 66°C for 4 minutes. In this PCR, two primers LF-35 and LR8-35 having the nucleotide sequences shown in SEQ ID NOs: 44 and 45 of the Sequence Listing, respectively, were used. A product obtained was separated by 1% agarose gel, and stained with ethidium bromide. As a result, as shown in FIG. 5, contrary to the wild-type Taq DNA polymerases where hardly any bands were found at a position of 8 kb in either in the absence of a PCNA (lane 1) or in the presence of a PCNA (lane 2), Taq81 to Taq85 showed strong bands at a position of 8kb in the presence of a PCNA (lanes 4, 6, 8, 10, and 12), although hardly any bands were found in the absence of the PCNA (lanes 3, 5, 7, 9, and 11).

The above results show that the Taq81 to Taq85, which are Taq DNA polymerase to which a PIP box is previously added at an N-terminal, are capable of amplifying an 8 kb DNA in the presence of a PCNA.

On the other hand, as shown in FIG. 6, Taq92 to Taq93, Taq DNA polymerases to which PIP box was added at a C-terminal, were not capable of amplifying an 8 kb DNA even in the presence of a PCNA.

### (3) Amplification of 12 kb and 15 kb DNAs

The amplifications of 12 kb and 15 kb DNAs were studied. A reaction solution was prepared in the same manner as in Example 3-(2), except that in the amplification of 12 kb, two primers LF-35 and LR12-35 having the nucleotide sequences shown in SEQ ID NOs: 44 and 46 of the Sequence Listing, respectively, were used, or that in the amplification of 15 kb DNA, two primers LF-35 and LR15-35 having the nucleotide sequences shown in SEQ ID NOs: 44 and 47 of the Sequence Listing, respectively, were used.

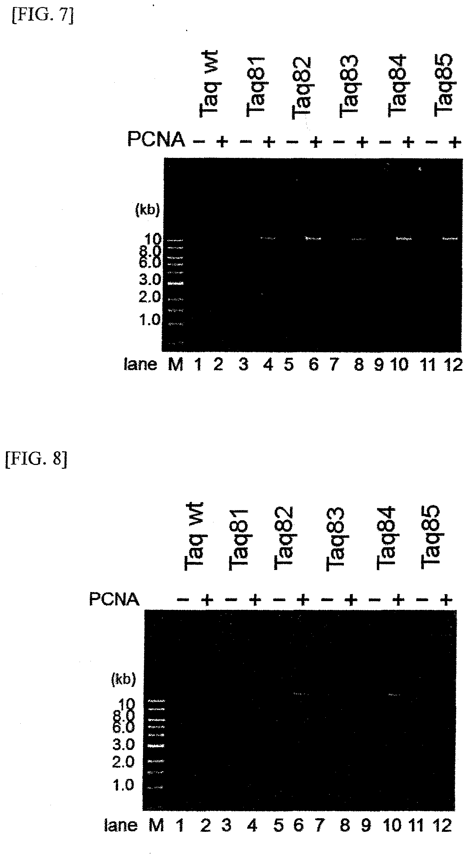

The conditions for PCR were as follows. First, a reaction solution was incubated for 95°C for 1 minute, and subsequently, 30 cycles of shuttle PCR were carried out, wherein one cycle is 99°C for 5 seconds, and 66°C for 12 minutes. The analyses of a product obtained were carried out in the same manner as in Example 3(2). The results are shown in FIGs. 7 and 8.

As shown in FIG. 7, in the amplification of a 12 kb DNA, although the wild-type Taq DNA polymerase showed no bands in the absence of a PCNA (lane 1) and in the presence of a PCNA (lane 2), Taq81 to Taq85 (lanes 4, 6, 8, 10, and 12) showed a band appearing at a position of 12 kb in the presence of a PCNA. Likewise, as shown in FIG. 8, even in the amplification of a 15kb DNA, bands could be confirmed in the presence of a PCNA in Taq81 to Taq85 (lanes 4, 6, 8, 10, and 12).

The above results show that Taq81 to Taq85 are capable of amplifying 12 kb and 15 kb DNAs in the presence of a PCNA.

In addition, the conditions for shuttle PCR employed in this Example are shorter in the time period needed for annealing of the primers and extension of complementary DNA, as compared to the conditions of conventional shuttle PCR. Nonetheless, it could be confirmed that a DNA of the same long-strand length as the conventional ones could be amplified, so that the shuttle PCR is excellent in speediness.

### Example 4 Taq DNA Polymerases to Which Plural PIP Boxes Were Added

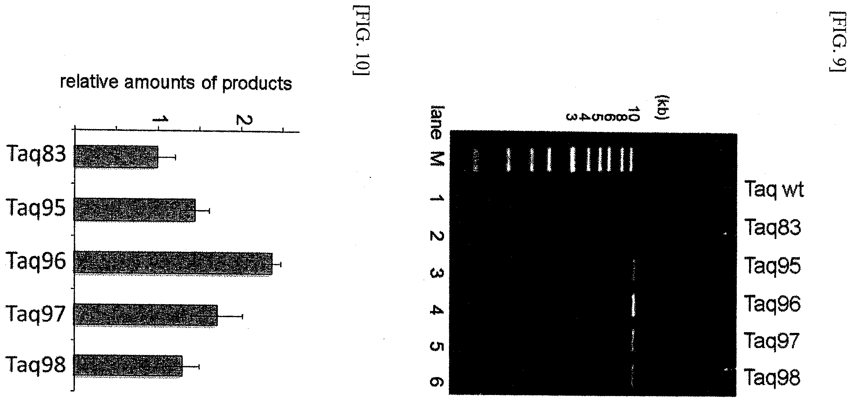

Using Taq95 (PIP-L14-PIP-L15-Taq), Taq96 (PIP-L14-PIP-L14-PIP-L15-Taq), Taq97 (PIP-L14-PIP-L14-PIP-L14-PIP-L15-Taq), and Taq98 (PIP-L14-PIP-L14-PIP-L14-PIP-L14-PIP-L15-Taq), each of which was prepared in Example 1(13), PCR was carried out for a 12 kb in the presence of a PCNA in the same manner as in Example 3(3). The results are shown in FIG. 9. Further, an amplified product was quantified using LAS-3000mini (manufactured by GE Healthcare). The procedures from PCR to quantification mentioned above were repeated 3 times, and a relative value was graphically shown, in which an amplified product of Taq83 (PIP-L15-Taq) was defined as 1. The results are shown in FIG. 10.

As shown in FIGs. 9 and 10, Taq95 to Taq98 to which plural PIP boxes were added could be confirmed to have larger amplified products as compared to Taq83 where PIP box was one. Especially, in Taq96 to which three PIP boxes were added, the amplified products were largest.

It is shown from the above that efficient DNA amplification can be made by adding plural PIP boxes to an N-terminal of the Taq DNA polymerase.

### Example 5 Taq DNA Polymerases to Which PIP Box Comprising Various Sequences Are Added

Using any one of peptides comprising amino acid sequences shown in SEQ ID NOs: 52 to 91 of the Sequence Listing listed in Table 1 (i.e., PIP box), in place of a PIP box comprising the amino acid sequence shown in SEQ ID NO: 3 of the Sequence Listing used in Example 1, Taq DNA polymerases to which any one of PIP boxes is added at an N-terminal are purified in accordance with the method described in Example 1. Using the Taq DNA polymerase, the amplification of a DNA is carried out in the presence of a PCNA in accordance with the method described in Example 3.

When any one of peptides listed in Table 1 are used in place of the PIP box comprising the amino acid sequence shown in SEQ ID NO: 3, the same effects as described above can be expected.

Further, PCR is carried out using a Taq DNA polymerase to which any one of peptides comprising amino acid sequences shown in SEQ ID NOs: 52 to 91 of the Sequence Listing listed in Table 1 (i.e., PIP box) are added at an N-terminal in plurality, in place of a PIP box comprising the amino acid sequence shown in SEQ ID NO: 3 of the Sequence Listing used in Example 1, in the presence of a PCNA, in accordance with the method described in Example 4.

When any one of peptides listed in Table 1 are used in place of the PIP box comprising the amino acid sequence shown in SEQ ID NO: 3, the same effects as described above can be expected.

Here, concrete primers to be used in the purification of a Taq DNA polymerase to which any of peptides comprising amino acid sequences shown in SEQ ID NOs: 52 to 91 are added, or concrete primers to be used in DNA amplification using the Taq DNA polymerase will be prepared referring to the description of the specification of the present application and the above Examples.

### INDUSTRIAL APPLICABILITY

According to the present invention, a family A (Pol I-type) DNA polymerase variant which is more convenient and easy-to-use, and has excellent extensibility and speediness, and a method for amplifying nucleic acids using the polymerase are provided. The DNA polymerase variant of the present invention is useful in amplifying nucleic acids especially in the presence of a PCNA.

### SEQUENCE LISTING FREE TEXT

SEQ ID NO: 1: Taq DNA polymerase amino acid sequence
SEQ ID NO: 2: Taq DNA polymerase nucleic acid sequence
SEQ ID NO: 3: *Pyrococcus furiosus* RFC-L PIP-box amino acid sequence
SEQ ID NO: 4: DNA polymerase variant Taq81 amino acid sequence
SEQ ID NO: 5: DNA polymerase variant Taq81 nucleic acid sequence
SEQ ID NO: 6: DNA polymerase variant Taq82 amino acid sequence
SEQ ID NO: 7: DNA polymerase variant Taq82 nucleic acid sequence
SEQ ID NO: 8: DNA polymerase variant Taq83 amino acid sequence
SEQ ID NO: 9: DNA polymerase variant Taq83 nucleic acid sequence
SEQ ID NO: 10: DNA polymerase variant Taq84 amino acid sequence
SEQ ID NO: 11: DNA polymerase variant Taq84 nucleic acid sequence
SEQ ID NO: 12: DNA polymerase variant Taq85 amino acid sequence
SEQ ID NO: 13: DNA polymerase variant Taq85 nucleic acid sequence
SEQ ID NO: 14: DNA polymerase variant Taq92 amino acid sequence
SEQ ID NO: 15: DNA polymerase variant Taq92 nucleic acid sequence
SEQ ID NO: 16: DNA polymerase variant Taq93 amino acid sequence
SEQ ID NO: 17: DNA polymerase variant Taq93 nucleic acid sequence
SEQ ID NO: 18: DNA polymerase variant Taq94 amino acid sequence
SEQ ID NO: 19: DNA polymerase variant Taq94 nucleic acid sequence
SEQ ID NO: 20: DNA polymerase variant Taq95 amino acid sequence
SEQ ID NO: 21: DNA polymerase variant Taq95 nucleic acid sequence
SEQ ID NO: 22: DNA polymerase variant Taq96 amino acid sequence
SEQ ID NO: 23: DNA polymerase variant Taq96 nucleic acid sequence
SEQ ID NO: 24: DNA polymerase variant Taq97 amino acid sequence
SEQ ID NO: 25: DNA polymerase variant Taq97 nucleic acid sequence
SEQ ID NO: 26: DNA polymerase variant Taq98 amino acid sequence
SEQ ID NO: 27: DNA polymerase variant Taq98 nucleic acid sequence
SEQ ID NO: 28: TaqNPIP-5 primer
SEQ ID NO: 29: Taq-3 primer
SEQ ID NO: 30: taqN10-5 primer
SEQ ID NO: 31: taqN10-3 primer
SEQ ID NO: 32: taqN15-5 primer
SEQ ID NO: 33: taqN15-3 primer
SEQ ID NO: 34: taq-plus20-5 primer
SEQ ID NO: 35: taq-plus20-3 primer
SEQ ID NO: 36: Taq-plus12-5 primer
SEQ ID NO: 37: Taq-plus12-3 primer
SEQ ID NO: 38: Taq-5 primer
SEQ ID NO: 39: Tq-L5-PIP-3 primer
SEQ ID NO: 40: Tq-L10-PIP-3 primer
SEQ ID NO: 41: Tq-L15-PIP-3 primer
SEQ ID NO: 42: F1 primer
SEQ ID NO: 43: R2-2 primer
SEQ ID NO: 44: LF-35 primer
SEQ ID NO: 45: LR8-35 primer
SEQ ID NO: 46: LR12-35 primer
SEQ ID NO: 47: LR15-35 primer
SEQ ID NO: 48: Taq95-5 primer
SEQ ID NO: 49: Taq96-5 primer
SEQ ID NO: 50: Taq97-5 primer
SEQ ID NO: 51: Taq98-5 primer
SEQ ID NO: 52: *P. furiosus* RFC-L PIP-box amino acid sequence
SEQ ID NO: 53: *M. jannaschii* RFC-L PIP-box amino acid sequence
SEQ ID NO: 54: *P. furiosus* PolBI PIP-box amino acid sequence
SEQ ID NO: 55: *T. litoralis* PolBI PIP-box amino acid sequence
SEQ ID NO: 56: *A. fulgidus* PolBI PIP-box amino acid sequence
SEQ ID NO: 57: *P. occultum* PolBII PIP-box amino acid sequence
SEQ ID NO: 58: *M. jannaschii* DP2 PIP-box amino acid sequence
SEQ ID NO: 59: *M. thermoautotrophicum* DP2 PIP-box amino acid sequence
SEQ ID NO: 60: *H. sapiens* Polδ p66 PIP-box amino acid sequence
SEQ ID NO: 61: *S. cerevisiae* Pol32 PIP-box amino acid sequence
SEQ ID NO: 62: *S. pombe* Cdc27 PIP-box amino acid sequence
SEQ ID NO: 63: *S. cerevisiae* Pol2 PIP-box amino acid sequence
SEQ ID NO: 64: *P. furiosus* Fen1 PIP-box amino acid sequence
SEQ ID NO: 65: *M. jannaschii* Fen1 PIP-box amino acid sequence
SEQ ID NO: 66: *A. fulgidus* Fen1 PIP-box amino acid sequence
SEQ ID NO: 67: *H. sapiens* Fen1 PIP-box amino acid sequence
SEQ ID NO: 68: *D. melanogaster* Fen1 PIP-box amino acid sequence
SEQ ID NO: 69: *S. cerevisiae* Fen1 PIP-box amino acid sequence
SEQ ID NO: 70: *S. pombe* Fen1 PIP-box amino acid sequence
SEQ ID NO: 71: *H. sapiens* DNA ligase I PIP-box amino acid sequence
SEQ ID NO: 72: *X. laevis* DNA ligase I PIP-box amino acid sequence
SEQ ID NO: 73: *S. cerevisiae* DNA ligase I PIP-box amino acid sequence
SEQ ID NO: 74: *S. pombe* DNA ligase I PIP-box amino acid sequence
SEQ ID NO: 75: *H. sapiens* MSH3 PIP-box amino acid sequence
SEQ ID NO: 76: *S. cerevisiae* MSH3 PIP-box amino acid sequence
SEQ ID NO: 77: *H. sapiens* MSH6 PIP-box amino acid sequence
SEQ ID NO: 78: *S. cerevisiae* MSH6 PIP-box amino acid sequence
SEQ ID NO: 79: *H. sapiens* UNG2 PIP-box amino acid sequence
SEQ ID NO: 80: *S. cerevisiae* UNG PIP-box amino acid sequence
SEQ ID NO: 81: *H. sapiens* hMYH PIP-box amino acid sequence
SEQ ID NO: 82: *H. sapiens* XPG PIP-box amino acid sequence
SEQ ID NO: 83: *C. elegans* XPG PIP-box amino acid sequence
SEQ ID NO: 84: *S. cerevisiae* XPG PIP-box amino acid sequence
SEQ ID NO: 85: *S. pombe* XPG PIP-box amino acid sequence
SEQ ID NO: 86: *S. cerevisiae* Cac1 PIP-box amino acid sequence
SEQ ID NO: 87: *H. sapiens* hRECQ5 PIP-box amino acid sequence
SEQ ID NO: 88: *S. cerevisiae* Rrm3 PIP-box amino acid sequence
SEQ ID NO: 89: *H. sapiens* Cdc25C PIP-box amino acid sequence
SEQ ID NO: 90: *H. sapiens* p15 PIP-box amino acid sequence
SEQ ID NO: 91: *H. sapiens* DNA-dependent protein kinase PIP-box amino acid sequence

## Claims

1. A fusion polypeptide comprising, in a direction of from an N-terminal side to a C-terminal side,
a) one or more peptides which bind to a PCNA, and
b) a polypeptide having a DNA polymerase activity.

2. The fusion polypeptide according to claim 1, **characterized in that** the peptide which bind to a PCNA is a peptide comprising a PIP box.

3. The fusion polypeptide according to claim 2, **characterized in that** the PIP box is a peptide consisting of an amino acid sequence shown in any one of SEQ ID NOs: 52 to 91 of the Sequence Listing.

4. The fusion polypeptide according to claim 1 or 2, **characterized in that** the peptide which bind to a PCNA is a peptide comprising a PIP box derived from a DNA polymerase-associated factor.

5. The fusion polypeptide according to any one of claims 1, 2 and 4, **characterized in that** the peptide which bind to a PCNA is a peptide comprising a PIP box derived from a replication factor C large subunit.

6. The fusion polypeptide according to any one of claims 1, 2, 4, and 5, **characterized in that** the peptide which bind to a PCNA is the amino acid sequence shown in SEQ ID NO: 3 of the Sequence Listing, or a peptide comprising the above amino acid sequence.

7. The fusion polypeptide according to any one of claims 1 to 6, **characterized in that** the fusion polypeptide comprises a 5 to 50 amino acid linker peptide between the peptide which bind to a PCNA and the polypeptide having a DNA polymerase activity.

8. The fusion polypeptide according to claim 7, **characterized in that** the linker peptide is an amino acid sequence composed of serine and glycine.

9. The fusion polypeptide according to any one of claims 1 to 8, **characterized in that** the polypeptide having a DNA polymerase activity is a Pol I-type DNA polymerase or a fragment thereof.

10. The fusion polypeptide according to any one of claims 1 to 9, **characterized in that** the polypeptide having a DNA polymerase activity is a Taq DNA polymerase or a fragment thereof.

11. A nucleic acid encoding a fusion polypeptide as defined in any one of claims 1 to 10.

12. A composition for amplifying nucleic acids comprising a fusion polypeptide as defined in any one of claims 1 to 10.

13. The composition for amplifying nucleic acids according to claim 12, further containing a PCNA.

14. A kit comprising a fusion polypeptide as defined in any one of claims 1 to 10.

15. The kit according to claim 14, further comprising a PCNA.

16. A method for producing a DNA complementary to a template DNA, **characterized by** the use of a composition comprising a fusion polypeptide as defined in any one of claims 1 to 10 and a PCNA.


SEQUENCE LISTING

<110>  Kyushu University, National University Corporation
       TAKARA BIO INC.

<120>  DNA polymerase variant

<130>  16-050-PCTJP

<150>  JP 2015-231974
<151>  2015-11-27

<160>  91

<170>  PatentIn version 3.5

<210>  1
<211>  832
<212>  PRT
<213>  Thermus aquaticus

<400>  1

Met Arg Gly Met Leu Pro Leu Phe Glu Pro Lys Gly Arg Val Leu Leu
1               5                   10                  15

Val Asp Gly His His Leu Ala Tyr Arg Thr Phe His Ala Leu Lys Gly
            20                  25                  30

Leu Thr Thr Ser Arg Gly Glu Pro Val Gln Ala Val Tyr Gly Phe Ala
        35                  40                  45

Lys Ser Leu Leu Lys Ala Leu Lys Glu Asp Gly Asp Ala Val Ile Val
    50                  55                  60

Val Phe Asp Ala Lys Ala Pro Ser Phe Arg His Glu Ala Tyr Gly Gly
65                  70                  75                  80

Tyr Lys Ala Gly Arg Ala Pro Thr Pro Glu Asp Phe Pro Arg Gln Leu
                85                  90                  95

Ala Leu Ile Lys Glu Leu Val Asp Leu Leu Gly Leu Ala Arg Leu Glu
            100                 105                 110

Val Pro Gly Tyr Glu Ala Asp Asp Val Leu Ala Ser Leu Ala Lys Lys
        115                 120                 125

Ala Glu Lys Glu Gly Tyr Glu Val Arg Ile Leu Thr Ala Asp Lys Asp
    130                 135                 140

Leu Tyr Gln Leu Leu Ser Asp Arg Ile His Ala Leu His Pro Glu Gly
145                 150                 155                 160

Tyr Leu Ile Thr Pro Ala Trp Leu Trp Glu Lys Tyr Gly Leu Arg Pro
                165                 170                 175

Asp Gln Trp Ala Asp Tyr Arg Ala Leu Thr Gly Asp Glu Ser Asp Asn
            180                 185                 190

Leu Pro Gly Val Lys Gly Ile Gly Glu Lys Thr Ala Arg Lys Leu Leu
        195                 200                 205

Glu Glu Trp Gly Ser Leu Glu Ala Leu Leu Lys Asn Leu Asp Arg Leu
    210                 215                 220

Lys Pro Ala Ile Arg Glu Lys Ile Leu Ala His Met Asp Asp Leu Lys
225                 230                 235                 240

Leu Ser Trp Asp Leu Ala Lys Val Arg Thr Asp Leu Pro Leu Glu Val
                245                 250                 255

Asp Phe Ala Lys Arg Arg Glu Pro Asp Arg Glu Arg Leu Arg Ala Phe
            260                 265                 270

Leu Glu Arg Leu Glu Phe Gly Ser Leu Leu His Glu Phe Gly Leu Leu
        275                 280                 285

Glu Ser Pro Lys Ala Leu Glu Glu Ala Pro Trp Pro Pro Pro Glu Gly
    290                 295                 300

Ala Phe Val Gly Phe Val Leu Ser Arg Lys Glu Pro Met Trp Ala Asp
305                 310                 315                 320

Leu Leu Ala Leu Ala Ala Ala Arg Gly Gly Arg Val His Arg Ala Pro
                325                 330                 335

Glu Pro Tyr Lys Ala Leu Arg Asp Leu Lys Glu Ala Arg Gly Leu Leu
            340                 345                 350

Ala Lys Asp Leu Ser Val Leu Ala Leu Arg Glu Gly Leu Gly Leu Pro
        355                 360                 365

Pro Gly Asp Asp Pro Met Leu Leu Ala Tyr Leu Leu Asp Pro Ser Asn
    370                 375                 380

Thr Thr Pro Glu Gly Val Ala Arg Arg Tyr Gly Gly Glu Trp Thr Glu
385                 390                 395                 400

Glu Ala Gly Glu Arg Ala Ala Leu Ser Glu Arg Leu Phe Ala Asn Leu
                405                 410                 415

Trp Gly Arg Leu Glu Gly Glu Glu Arg Leu Leu Trp Leu Tyr Arg Glu
            420                 425                 430

Val Glu Arg Pro Leu Ser Ala Val Leu Ala His Met Glu Ala Thr Gly
        435                 440                 445

Val Arg Leu Asp Val Ala Tyr Leu Arg Ala Leu Ser Leu Glu Val Ala
    450                 455                 460

Glu Glu Ile Ala Arg Leu Glu Ala Glu Val Phe Arg Leu Ala Gly His
465                 470                 475                 480

Pro Phe Asn Leu Asn Ser Arg Asp Gln Leu Glu Arg Val Leu Phe Asp
                485                 490                 495

Glu Leu Gly Leu Pro Ala Ile Gly Lys Thr Glu Lys Thr Gly Lys Arg
            500                 505                 510

Ser Thr Ser Ala Ala Val Leu Glu Ala Leu Arg Glu Ala His Pro Ile
        515                 520                 525

Val Glu Lys Ile Leu Gln Tyr Arg Glu Leu Thr Lys Leu Lys Ser Thr
    530                 535                 540

Tyr Ile Asp Pro Leu Pro Asp Leu Ile His Pro Arg Thr Gly Arg Leu
545                 550                 555                 560

His Thr Arg Phe Asn Gln Thr Ala Thr Ala Thr Gly Arg Leu Ser Ser
                565                 570                 575

Ser Asp Pro Asn Leu Gln Asn Ile Pro Val Arg Thr Pro Leu Gly Gln
            580                 585                 590

Arg Ile Arg Arg Ala Phe Ile Ala Glu Glu Gly Trp Leu Leu Val Ala
        595                 600                 605

Leu Asp Tyr Ser Gln Ile Glu Leu Arg Val Leu Ala His Leu Ser Gly
    610                 615                 620

Asp Glu Asn Leu Ile Arg Val Phe Gln Glu Gly Arg Asp Ile His Thr
625                 630                 635                 640

Glu Thr Ala Ser Trp Met Phe Gly Val Pro Arg Glu Ala Val Asp Pro
                645                 650                 655

Leu Met Arg Arg Ala Ala Lys Thr Ile Asn Phe Gly Val Leu Tyr Gly
            660                 665                 670

Met Ser Ala His Arg Leu Ser Gln Glu Leu Ala Ile Pro Tyr Glu Glu
        675                 680                 685

Ala Gln Ala Phe Ile Glu Arg Tyr Phe Gln Ser Phe Pro Lys Val Arg
    690                 695                 700

Ala Trp Ile Glu Lys Thr Leu Glu Glu Gly Arg Arg Arg Gly Tyr Val
705                 710                 715                 720

Glu Thr Leu Phe Gly Arg Arg Arg Tyr Val Pro Asp Leu Glu Ala Arg
                725                 730                 735

Val Lys Ser Val Arg Glu Ala Ala Glu Arg Met Ala Phe Asn Met Pro
            740                 745                 750

Val Gln Gly Thr Ala Ala Asp Leu Met Lys Leu Ala Met Val Lys Leu
        755                 760                 765

Phe Pro Arg Leu Glu Glu Met Gly Ala Arg Met Leu Leu Gln Val His
    770                 775                 780

Asp Glu Leu Val Leu Glu Ala Pro Lys Glu Arg Ala Glu Ala Val Ala
785                 790                 795                 800

Arg Leu Ala Lys Glu Val Met Glu Gly Val Tyr Pro Leu Ala Val Pro
                805                 810                 815

Leu Glu Val Glu Val Gly Ile Gly Glu Asp Trp Leu Ser Ala Lys Glu
            820                 825                 830

<210>  2
<211>  2499
<212>  DNA
<213>  Thermus aquaticus

<400>  2
atgcgtggta tgctgccgct gttcgaaccc aagggccggg tcctcctggt ggacggccac      60

cacctggcct accgcacctt ccacgccctg aagggcctca ccaccagccg gggggagccg     120

gtgcaggcgg tctacggctt cgccaagagc ctcctcaagg ccctcaagga ggacggggac     180

gcggtgatcg tggtctttga cgccaaggcc ccctccttcc gccacgaggc ctacgggggg     240

tacaaggcgg gccgggcccc cacgccggag gactttcccc ggcaactcgc cctcatcaag     300

gagctggtgg acctcctggg gctggcgcgc ctcgaggtcc cgggctacga ggcggacgac     360

gtcctggcca gcctggccaa gaaggcggaa aaggagggct acgaggtccg catcctcacc     420

gccgacaaag acctttacca gctcctttcc gaccgcatcc acgccctcca ccccgagggg     480

tacctcatca ccccggcctg gctttgggaa aagtacggcc tgaggcccga ccagtgggcc 540

gactaccggg ccctgaccgg ggacgagtcc gacaaccttc ccggggtcaa gggcatcggg 600

gagaagacgg cgaggaagct tctggaggag tgggggagcc tggaagccct cctcaagaac 660

ctggaccggc tgaagcccgc catccgggag aagatcctgg cccacatgga cgatctgaag 720

ctctcctggg acctggccaa ggtgcgcacc gacctgcccc tggaggtgga cttcgccaaa 780

aggcgggagc ccgaccggga gaggcttagg gcctttctgg agaggcttga gtttggcagc 840

ctcctccacg agttcggcct tctggaaagc cccaaggccc tggaggaggc cccctggccc 900

ccgccggaag gggccttcgt gggctttgtg ctttcccgca aggagcccat gtgggccgat 960

cttctggccc tggccgccgc cagggggggc cgggtccacc gggcccccga gccttataaa 1020

gccctcaggg acctgaagga ggcgcggggg cttctcgcca aagacctgag cgttctggcc 1080

ctgagggaag gccttggcct cccgcccggc gacgacccca tgctcctcgc ctacctcctg 1140

gacccttcca acaccacccc cgagggggtg gcccggcgct acggcgggga gtggacggag 1200

gaggcggggg agcgggccgc cctttccgag aggctcttcg ccaacctgtg ggggaggctt 1260

gagggggagg agaggctcct ttggctttac cgggaggtgg agaggcccct ttccgctgtc 1320

ctggcccaca tggaggccac gggggtgcgc ctggacgtgg cctatctcag ggccttgtcc 1380

ctggaggtgg ccgaggagat cgcccgcctc gaggccgagg tcttccgcct ggccggccac 1440

cccttcaacc tcaactcccg ggaccagctg gaaagggtcc tctttgacga gctagggctt 1500

cccgccatcg gcaagacgga gaagaccggc aagcgctcca ccagcgccgc cgtcctggag 1560

gccctccgcg aggcccaccc catcgtggag aagatcctgc agtaccggga gctcaccaag 1620

ctgaagagca cctacattga ccccttgccg gacctcatcc accccaggac gggccgcctc 1680

cacacccgct tcaaccagac ggccacggcc acgggcaggc taagtagctc cgatcccaac 1740

ctccagaaca tccccgtccg caccccgctt gggcagagga tccgccgggc cttcatcgcc 1800

gaggaggggt ggctattggt ggccctggac tatagccaga tagagctcag ggtgctggcc 1860

cacctctccg gcgacgagaa cctgatccgg gtcttccagg aggggcggga catccacacg 1920

gagaccgcca gctggatgtt cggcgtcccc cgggaggccg tggaccccct gatgcgccgg 1980

gcggccaaga ccatcaactt cggggtcctc tacggcatgt cggcccaccg cctctcccag 2040

gagctagcca tcccttacga ggaggcccag gccttcattg agcgctactt tcagagcttc 2100

cccaaggtgc gggcctggat tgagaagacc ctggaggagg gcaggaggcg ggggtacgtg 2160

gagaccctct tcggccgccg ccgctacgtg ccagacctag aggcccgggt gaagagcgtg 2220

cgggaggcgg ccgagcgcat ggccttcaac atgcccgtcc agggcaccgc cgccgacctc 2280

atgaagctgg ctatggtgaa gctcttcccc aggctggagg aaatgggggc caggatgctc 2340

cttcaggtcc acgacgagct ggtcctcgag gcccccaaag agagggcgga ggccgtggcc 2400

cggctggcca aggaggtcat ggagggggtg tatcccctgg ccgtgcccct ggaggtggag 2460

gtggggatag gggaggactg gctctccgcc aaggagtga 2499

<210> 3
<211> 11
<212> PRT
<213> Pyrococcus furiosus

<400> 3

Lys Gln Ala Thr Leu Phe Asp Phe Leu Lys Lys
1               5                   10

<210> 4
<211> 851
<212> PRT
<213> Artificial Sequence

<220>
<223> DNA polymerase variant Taq81

<400> 4

Met Ser Gly Lys Gln Ala Thr Leu Phe Asp Phe Leu Lys Lys Gly Ser
1               5                   10                  15

Gly Ser Gly Met Arg Gly Met Leu Pro Leu Phe Glu Pro Lys Gly Arg
            20                  25                  30

Val Leu Leu Val Asp Gly His His Leu Ala Tyr Arg Thr Phe His Ala
        35                  40                  45

Leu Lys Gly Leu Thr Thr Ser Arg Gly Glu Pro Val Gln Ala Val Tyr
    50                  55                  60

Gly Phe Ala Lys Ser Leu Leu Lys Ala Leu Lys Glu Asp Gly Asp Ala
65                  70                  75                  80

Val Ile Val Val Phe Asp Ala Lys Ala Pro Ser Phe Arg His Glu Ala
                85                  90                  95

Tyr Gly Gly Tyr Lys Ala Gly Arg Ala Pro Thr Pro Glu Asp Phe Pro
            100                 105                 110

Arg Gln Leu Ala Leu Ile Lys Glu Leu Val Asp Leu Leu Gly Leu Ala
        115                 120                 125

Arg Leu Glu Val Pro Gly Tyr Glu Ala Asp Asp Val Leu Ala Ser Leu
    130                 135                 140

Ala Lys Lys Ala Glu Lys Glu Gly Tyr Glu Val Arg Ile Leu Thr Ala
145                 150                 155                 160

Asp Lys Asp Leu Tyr Gln Leu Leu Ser Asp Arg Ile His Ala Leu His
                165                 170                 175

Pro Glu Gly Tyr Leu Ile Thr Pro Ala Trp Leu Trp Glu Lys Tyr Gly
            180                 185                 190

Leu Arg Pro Asp Gln Trp Ala Asp Tyr Arg Ala Leu Thr Gly Asp Glu
        195                 200                 205

Ser Asp Asn Leu Pro Gly Val Lys Gly Ile Gly Glu Lys Thr Ala Arg
    210                 215                 220

Lys Leu Leu Glu Glu Trp Gly Ser Leu Glu Ala Leu Leu Lys Asn Leu
225                 230                 235                 240

Asp Arg Leu Lys Pro Ala Ile Arg Glu Lys Ile Leu Ala His Met Asp
                245                 250                 255

Asp Leu Lys Leu Ser Trp Asp Leu Ala Lys Val Arg Thr Asp Leu Pro
            260                 265                 270

Leu Glu Val Asp Phe Ala Lys Arg Arg Glu Pro Asp Arg Glu Arg Leu
        275                 280                 285

Arg Ala Phe Leu Glu Arg Leu Glu Phe Gly Ser Leu Leu His Glu Phe
    290                 295                 300

Gly Leu Leu Glu Ser Pro Lys Ala Leu Glu Glu Ala Pro Trp Pro Pro
305                 310                 315                 320

Pro Glu Gly Ala Phe Val Gly Phe Val Leu Ser Arg Lys Glu Pro Met
                325                 330                 335

Trp Ala Asp Leu Leu Ala Leu Ala Ala Ala Arg Gly Gly Arg Val His
            340                 345                 350

Arg Ala Pro Glu Pro Tyr Lys Ala Leu Arg Asp Leu Lys Glu Ala Arg
        355                 360                 365

Gly Leu Leu Ala Lys Asp Leu Ser Val Leu Ala Leu Arg Glu Gly Leu
    370                 375                 380

Gly Leu Pro Pro Gly Asp Asp Pro Met Leu Leu Ala Tyr Leu Leu Asp
385                 390                 395                 400

Pro Ser Asn Thr Thr Pro Glu Gly Val Ala Arg Arg Tyr Gly Gly Glu
                405                 410                 415

Trp Thr Glu Glu Ala Gly Glu Arg Ala Ala Leu Ser Glu Arg Leu Phe
            420                 425                 430

Ala Asn Leu Trp Gly Arg Leu Glu Gly Glu Glu Arg Leu Leu Trp Leu
        435                 440                 445

Tyr Arg Glu Val Glu Arg Pro Leu Ser Ala Val Leu Ala His Met Glu
    450                 455                 460

Ala Thr Gly Val Arg Leu Asp Val Ala Tyr Leu Arg Ala Leu Ser Leu
465                 470                 475                 480

Glu Val Ala Glu Glu Ile Ala Arg Leu Glu Ala Glu Val Phe Arg Leu
                485                 490                 495

Ala Gly His Pro Phe Asn Leu Asn Ser Arg Asp Gln Leu Glu Arg Val
            500                 505                 510

Leu Phe Asp Glu Leu Gly Leu Pro Ala Ile Gly Lys Thr Glu Lys Thr
        515                 520                 525

Gly Lys Arg Ser Thr Ser Ala Ala Val Leu Glu Ala Leu Arg Glu Ala
    530                 535                 540

His Pro Ile Val Glu Lys Ile Leu Gln Tyr Arg Glu Leu Thr Lys Leu
545                 550                 555                 560

Lys Ser Thr Tyr Ile Asp Pro Leu Pro Asp Leu Ile His Pro Arg Thr
                565                 570                 575

Gly Arg Leu His Thr Arg Phe Asn Gln Thr Ala Thr Ala Thr Gly Arg
            580                 585                 590

Leu Ser Ser Ser Asp Pro Asn Leu Gln Asn Ile Pro Val Arg Thr Pro
        595                 600                 605

Leu Gly Gln Arg Ile Arg Arg Ala Phe Ile Ala Glu Glu Gly Trp Leu
    610                 615                 620

Leu Val Ala Leu Asp Tyr Ser Gln Ile Glu Leu Arg Val Leu Ala His
625                 630                 635                 640

Leu Ser Gly Asp Glu Asn Leu Ile Arg Val Phe Gln Glu Gly Arg Asp

645                 650                 655

Ile His Thr Glu Thr Ala Ser Trp Met Phe Gly Val Pro Arg Glu Ala
            660                 665                 670

Val Asp Pro Leu Met Arg Arg Ala Ala Lys Thr Ile Asn Phe Gly Val
        675                 680                 685

Leu Tyr Gly Met Ser Ala His Arg Leu Ser Gln Glu Leu Ala Ile Pro
    690                 695                 700

Tyr Glu Glu Ala Gln Ala Phe Ile Glu Arg Tyr Phe Gln Ser Phe Pro
705                 710                 715                 720

Lys Val Arg Ala Trp Ile Glu Lys Thr Leu Glu Glu Gly Arg Arg Arg
                725                 730                 735

Gly Tyr Val Glu Thr Leu Phe Gly Arg Arg Arg Tyr Val Pro Asp Leu
            740                 745                 750

Glu Ala Arg Val Lys Ser Val Arg Glu Ala Ala Glu Arg Met Ala Phe
        755                 760                 765

Asn Met Pro Val Gln Gly Thr Ala Ala Asp Leu Met Lys Leu Ala Met
    770                 775                 780

Val Lys Leu Phe Pro Arg Leu Glu Glu Met Gly Ala Arg Met Leu Leu
785                 790                 795                 800

Gln Val His Asp Glu Leu Val Leu Glu Ala Pro Lys Glu Arg Ala Glu
                805                 810                 815

Ala Val Ala Arg Leu Ala Lys Glu Val Met Glu Gly Val Tyr Pro Leu
            820                 825                 830

Ala Val Pro Leu Glu Val Glu Val Gly Ile Gly Glu Asp Trp Leu Ser
        835                 840                 845

Ala Lys Glu
    850

<210>  5
<211>  2556
<212>  DNA
<213>  Artificial Sequence

<220>
<223>  DNA polymerase variant Taq81

<400> 5
atgagcggca agcaggcgac gctgttcgac ttcctcaaga agggtagcgg ctccggcatg 60
cgtggtatgc tgccgctgtt cgaacccaag ggccgggtcc tcctggtgga cggccaccac 120
ctggcctacc gcaccttcca cgccctgaag ggcctcacca ccagccgggg ggagccggtg 180
caggcggtct acggcttcgc caagagcctc ctcaaggccc tcaaggagga cggggacgcg 240
gtgatcgtgg tctttgacgc caaggccccc tccttccgcc acgaggccta cggggggtac 300
aaggcgggcc gggcccccac gccggaggac tttccccggc aactcgccct catcaaggag 360
ctggtggacc tcctggggct ggcgcgcctc gaggtcccgg gctacgaggc ggacgacgtc 420
ctggccagcc tggccaagaa ggcggaaaag gagggctacg aggtccgcat cctcaccgcc 480
gacaaagacc tttaccagct cctttccgac cgcatccacg ccctccaccc cgaggggtac 540
ctcatcaccc cggcctggct ttgggaaaag tacggcctga ggcccgacca gtgggccgac 600
taccgggccc tgaccgggga cgagtccgac aaccttcccg gggtcaaggg catcggggag 660
aagacggcga ggaagcttct ggaggagtgg gggagcctgg aagccctcct caagaacctg 720
gaccggctga agcccgccat ccgggagaag atcctggccc acatggacga tctgaagctc 780
tcctgggacc tggccaaggt gcgcaccgac ctgcccctgg aggtggactt cgccaaaagg 840
cgggagcccg accgggagag gcttagggcc tttctggaga ggcttgagtt tggcagcctc 900
ctccacgagt tcggccttct ggaaagcccc aaggccctgg aggaggcccc ctggcccccg 960
ccggaagggg ccttcgtggg ctttgtgctt tcccgcaagg agcccatgtg ggccgatctt 1020
ctggccctgg ccgccgccag ggggggccgg gtccaccggg cccccgagcc ttataaagcc 1080
ctcagggacc tgaaggaggc gcgggggctt ctcgccaaag acctgagcgt tctggccctg 1140
agggaaggcc ttggcctccc gcccggcgac gaccccatgc tcctcgccta cctcctggac 1200
ccttccaaca ccacccccga gggggtggcc cggcgctacg gcggggagtg gacggaggag 1260
gcgggggagc gggccgccct ttccgagagg ctcttcgcca acctgtgggg gaggcttgag 1320
ggggaggaga ggctcctttg gctttaccgg gaggtggaga ggcccctttc cgctgtcctg 1380
gcccacatgg aggccacggg ggtgcgcctg gacgtggcct atctcagggc cttgtccctg 1440
gaggtggccg aggagatcgc ccgcctcgag gccgaggtct tccgcctggc cggccacccc 1500
ttcaacctca actcccggga ccagctggaa agggtcctct ttgacgagct agggcttccc 1560
gccatcggca agacggagaa gaccggcaag cgctccacca gcgccgccgt cctggaggcc 1620
ctccgcgagg cccaccccat cgtggagaag atcctgcagt accgggagct caccaagctg 1680
aagagcacct acattgaccc cttgccggac ctcatccacc ccaggacggg ccgcctccac 1740
acccgcttca accagacggc cacggccacg ggcaggctaa gtagctccga tcccaacctc 1800
cagaacatcc ccgtccgcac cccgcttggg cagaggatcc gccgggcctt catcgccgag 1860

gaggggtggc tattggtggc cctggactat agccagatag agctcagggt gctggcccac 1920

ctctccggcg acgagaacct gatccgggtc ttccaggagg ggcgggacat ccacacggag 1980

accgccagct ggatgttcgg cgtcccccgg gaggccgtgg accccctgat gcgccgggcg 2040

gccaagacca tcaacttcgg ggtcctctac ggcatgtcgg cccaccgcct ctcccaggag 2100

ctagccatcc cttacgagga ggcccaggcc ttcattgagc gctactttca gagcttcccc 2160

aaggtgcggg cctggattga gaagaccctg gaggagggca ggaggcgggg gtacgtggag 2220

accctcttcg gccgccgccg ctacgtgcca gacctagagg cccgggtgaa gagcgtgcgg 2280

gaggcggccg agcgcatggc cttcaacatg cccgtccagg gcaccgccgc cgacctcatg 2340

aagctggcta tggtgaagct cttccccagg ctggaggaaa tgggggccag gatgctcctt 2400

caggtccacg acgagctggt cctcgaggcc ccaaaagaga gggcggaggc cgtggcccgg 2460

ctggccaagg aggtcatgga gggggtgtat cccctggccg tgcccctgga ggtggaggtg 2520

gggatagggg aggactggct ctccgccaag gagtga 2556

<210> 6
<211> 856
<212> PRT
<213> Artificial Sequence

<220>
<223> DNA polymerase variant Taq82

<400> 6

Met Ser Gly Lys Gln Ala Thr Leu Phe Asp Phe Leu Lys Lys Ser Gly
1               5                   10                  15

Ser Gly Ser Gly Ser Gly Ser Gly Met Arg Gly Met Leu Pro Leu Phe
            20                  25                  30

Glu Pro Lys Gly Arg Val Leu Leu Val Asp Gly His His Leu Ala Tyr
        35                  40                  45

Arg Thr Phe His Ala Leu Lys Gly Leu Thr Thr Ser Arg Gly Glu Pro
    50                  55                  60

Val Gln Ala Val Tyr Gly Phe Ala Lys Ser Leu Leu Lys Ala Leu Lys
65                  70                  75                  80

Glu Asp Gly Asp Ala Val Ile Val Val Phe Asp Ala Lys Ala Pro Ser
                85                  90                  95

Phe Arg His Glu Ala Tyr Gly Gly Tyr Lys Ala Gly Arg Ala Pro Thr
            100                 105                 110

Pro Glu Asp Phe Pro Arg Gln Leu Ala Leu Ile Lys Glu Leu Val Asp
        115                 120                 125

Leu Leu Gly Leu Ala Arg Leu Glu Val Pro Gly Tyr Glu Ala Asp Asp
    130                 135                 140

Val Leu Ala Ser Leu Ala Lys Lys Ala Glu Lys Glu Gly Tyr Glu Val
145                 150                 155                 160

Arg Ile Leu Thr Ala Asp Lys Asp Leu Tyr Gln Leu Leu Ser Asp Arg
                165                 170                 175

Ile His Ala Leu His Pro Glu Gly Tyr Leu Ile Thr Pro Ala Trp Leu
            180                 185                 190

Trp Glu Lys Tyr Gly Leu Arg Pro Asp Gln Trp Ala Asp Tyr Arg Ala
        195                 200                 205

Leu Thr Gly Asp Glu Ser Asp Asn Leu Pro Gly Val Lys Gly Ile Gly
    210                 215                 220

Glu Lys Thr Ala Arg Lys Leu Leu Glu Glu Trp Gly Ser Leu Glu Ala
225                 230                 235                 240

Leu Leu Lys Asn Leu Asp Arg Leu Lys Pro Ala Ile Arg Glu Lys Ile
                245                 250                 255

Leu Ala His Met Asp Asp Leu Lys Leu Ser Trp Asp Leu Ala Lys Val
            260                 265                 270

Arg Thr Asp Leu Pro Leu Glu Val Asp Phe Ala Lys Arg Arg Glu Pro
        275                 280                 285

Asp Arg Glu Arg Leu Arg Ala Phe Leu Glu Arg Leu Glu Phe Gly Ser
    290                 295                 300

Leu Leu His Glu Phe Gly Leu Leu Glu Ser Pro Lys Ala Leu Glu Glu
305                 310                 315                 320

Ala Pro Trp Pro Pro Pro Glu Gly Ala Phe Val Gly Phe Val Leu Ser
                325                 330                 335

Arg Lys Glu Pro Met Trp Ala Asp Leu Leu Ala Leu Ala Ala Ala Arg
            340                 345                 350

Gly Gly Arg Val His Arg Ala Pro Glu Pro Tyr Lys Ala Leu Arg Asp
        355                 360                 365

Leu Lys Glu Ala Arg Gly Leu Leu Ala Lys Asp Leu Ser Val Leu Ala
    370                 375                 380

Leu Arg Glu Gly Leu Gly Leu Pro Pro Gly Asp Asp Pro Met Leu Leu
385                 390                 395                 400

Ala Tyr Leu Leu Asp Pro Ser Asn Thr Thr Pro Glu Gly Val Ala Arg
                405                 410                 415

Arg Tyr Gly Gly Glu Trp Thr Glu Glu Ala Gly Glu Arg Ala Ala Leu
            420                 425                 430

Ser Glu Arg Leu Phe Ala Asn Leu Trp Gly Arg Leu Glu Gly Glu Glu
        435                 440                 445

Arg Leu Leu Trp Leu Tyr Arg Glu Val Glu Arg Pro Leu Ser Ala Val
    450                 455                 460

Leu Ala His Met Glu Ala Thr Gly Val Arg Leu Asp Val Ala Tyr Leu
465                 470                 475                 480

Arg Ala Leu Ser Leu Glu Val Ala Glu Glu Ile Ala Arg Leu Glu Ala
                485                 490                 495

Glu Val Phe Arg Leu Ala Gly His Pro Phe Asn Leu Asn Ser Arg Asp
            500                 505                 510

Gln Leu Glu Arg Val Leu Phe Asp Glu Leu Gly Leu Pro Ala Ile Gly
        515                 520                 525

Lys Thr Glu Lys Thr Gly Lys Arg Ser Thr Ser Ala Ala Val Leu Glu
    530                 535                 540

Ala Leu Arg Glu Ala His Pro Ile Val Glu Lys Ile Leu Gln Tyr Arg
545                 550                 555                 560

Glu Leu Thr Lys Leu Lys Ser Thr Tyr Ile Asp Pro Leu Pro Asp Leu
                565                 570                 575

Ile His Pro Arg Thr Gly Arg Leu His Thr Arg Phe Asn Gln Thr Ala
            580                 585                 590

Thr Ala Thr Gly Arg Leu Ser Ser Ser Asp Pro Asn Leu Gln Asn Ile
        595                 600                 605

Pro Val Arg Thr Pro Leu Gly Gln Arg Ile Arg Arg Ala Phe Ile Ala
    610                 615                 620

Glu Glu Gly Trp Leu Leu Val Ala Leu Asp Tyr Ser Gln Ile Glu Leu
625                 630                 635                 640

Arg Val Leu Ala His Leu Ser Gly Asp Glu Asn Leu Ile Arg Val Phe
                645                 650                 655

Gln Glu Gly Arg Asp Ile His Thr Glu Thr Ala Ser Trp Met Phe Gly
            660                 665                 670

Val Pro Arg Glu Ala Val Asp Pro Leu Met Arg Arg Ala Ala Lys Thr
        675                 680                 685

Ile Asn Phe Gly Val Leu Tyr Gly Met Ser Ala His Arg Leu Ser Gln
    690                 695                 700

Glu Leu Ala Ile Pro Tyr Glu Glu Ala Gln Ala Phe Ile Glu Arg Tyr
705                 710                 715                 720

Phe Gln Ser Phe Pro Lys Val Arg Ala Trp Ile Glu Lys Thr Leu Glu
                725                 730                 735

Glu Gly Arg Arg Arg Gly Tyr Val Glu Thr Leu Phe Gly Arg Arg Arg
            740                 745                 750

Tyr Val Pro Asp Leu Glu Ala Arg Val Lys Ser Val Arg Glu Ala Ala
        755                 760                 765

Glu Arg Met Ala Phe Asn Met Pro Val Gln Gly Thr Ala Ala Asp Leu
    770                 775                 780

Met Lys Leu Ala Met Val Lys Leu Phe Pro Arg Leu Glu Glu Met Gly
785                 790                 795                 800

Ala Arg Met Leu Leu Gln Val His Asp Glu Leu Val Leu Glu Ala Pro
                805                 810                 815

Lys Glu Arg Ala Glu Ala Val Ala Arg Leu Ala Lys Glu Val Met Glu
            820                 825                 830

Gly Val Tyr Pro Leu Ala Val Pro Leu Glu Val Glu Val Gly Ile Gly
        835                 840                 845

Glu Asp Trp Leu Ser Ala Lys Glu
    850                 855

<210>  7

<211>  2571
<212>  DNA
<213>  Artificial Sequence

<220>
<223>  DNA polymerase variant Taq82

<400>  7
atgagcggca agcaggcgac gctgttcgac ttcctcaaga agagcggttc tggctccggt      60

agcggctccg gcatgcgtgg tatgctgccg ctgttcgaac ccaagggccg ggtcctcctg     120

gtggacggcc accacctggc ctaccgcacc ttccacgccc tgaagggcct caccaccagc     180

cggggggagc cggtgcaggc ggtctacggc ttcgccaaga gcctcctcaa ggccctcaag     240

gaggacgggg acgcggtgat cgtggtcttt gacgccaagg ccccctcctt ccgccacgag     300

gcctacgggg ggtacaaggc gggccgggcc cccacgccgg aggactttcc ccggcaactc     360

gccctcatca aggagctggt ggacctcctg gggctggcgc gcctcgaggt cccgggctac     420

gaggcggacg acgtcctggc cagcctggcc aagaaggcgg aaaaggaggg ctacgaggtc     480

cgcatcctca ccgccgacaa agacctttac cagctccttt ccgaccgcat ccacgccctc     540

caccccgagg ggtacctcat caccccggcc tggctttggg aaaagtacgg cctgaggccc     600

gaccagtggg ccgactaccg ggccctgacc ggggacgagt ccgacaacct tcccggggtc     660

aagggcatcg gggagaagac ggcgaggaag cttctggagg agtgggggag cctggaagcc     720

ctcctcaaga acctggaccg gctgaagccc gccatccggg agaagatcct ggcccacatg     780

gacgatctga agctctcctg ggacctggcc aaggtgcgca ccgacctgcc cctggaggtg     840

gacttcgcca aaaggcggga gcccgaccgg gagaggctta gggcctttct ggagaggctt     900

gagtttggca gcctcctcca cgagttcggc cttctggaaa gccccaaggc cctggaggag     960

gccccctggc ccccgccgga aggggccttc gtgggctttg tgctttcccg caaggagccc    1020

atgtgggccg atcttctggc cctggccgcc gccagggggg gccgggtcca ccgggccccc    1080

gagccttata aagccctcag ggacctgaag gaggcgcggg ggcttctcgc caaagacctg    1140

agcgttctgg ccctgaggga aggccttggc ctcccgcccg gcgacgaccc catgctcctc    1200

gcctacctcc tggacccttc caacaccacc cccgaggggg tggcccggcg ctacggcggg    1260

gagtggacgg aggaggcggg ggagcgggcc gccctttccg agaggctctt cgccaacctg    1320

tgggggaggc ttgaggggga ggagaggctc ctttggcttt accgggaggt ggagaggccc    1380

ctttccgctg tcctggccca catggaggcc acgggggtgc gcctggacgt ggcctatctc    1440

agggccttgt ccctggaggt ggccgaggag atcgcccgcc tcgaggccga ggtcttccgc    1500

ctggccggcc accccttcaa cctcaactcc cgggaccagc tggaaagggt cctctttgac    1560

gagctagggc ttcccgccat cggcaagacg gagaagaccg gcaagcgctc caccagcgcc    1620

gccgtcctgg aggccctccg cgaggcccac cccatcgtgg agaagatcct gcagtaccgg    1680

gagctcacca agctgaagag cacctacatt gaccccttgc cggacctcat ccaccccagg 1740

acgggccgcc tccacacccg cttcaaccag acggccacgg ccacgggcag gctaagtagc 1800

tccgatccca acctccagaa catccccgtc cgcaccccgc ttgggcagag gatccgccgg 1860

gccttcatcg ccgaggaggg gtggctattg gtggccctgg actatagcca gatagagctc 1920

agggtgctgg cccacctctc cggcgacgag aacctgatcc gggtcttcca ggaggggcgg 1980

gacatccaca cggagaccgc cagctggatg ttcggcgtcc cccgggaggc cgtggacccc 2040

ctgatgcgcc gggcggccaa gaccatcaac ttcggggtcc tctacggcat gtcggcccac 2100

cgcctctccc aggagctagc catcccttac gaggaggccc aggccttcat tgagcgctac 2160

tttcagagct tccccaaggt gcgggcctgg attgagaaga ccctggagga gggcaggagg 2220

cgggggtacg tggagaccct cttcggccgc cgccgctacg tgccagacct agaggcccgg 2280

gtgaagagcg tgcgggaggc ggccgagcgc atggccttca acatgcccgt ccagggcacc 2340

gccgccgacc tcatgaagct ggctatggtg aagctcttcc ccaggctgga ggaaatgggg 2400

gccaggatgc tccttcaggt ccacgacgag ctggtcctcg aggccccaaa agagagggcg 2460

gaggccgtgg cccggctggc caaggaggtc atggaggggg tgtatcccct ggccgtgccc 2520

ctggaggtgg aggtggggat aggggaggac tggctctccg ccaaggagtg a 2571

<210> 8
<211> 861
<212> PRT
<213> Artificial Sequence

<220>
<223> DNA polymerase variant Taq83

<400> 8

Met Ser Gly Lys Gln Ala Thr Leu Phe Asp Phe Leu Lys Lys Gly Ser
1               5                   10                  15

Gly Ser Gly Ser Gly Ser Gly Ser Gly Ser Gly Ser Gly Met Arg Gly
            20                  25                  30

Met Leu Pro Leu Phe Glu Pro Lys Gly Arg Val Leu Leu Val Asp Gly
        35                  40                  45

His His Leu Ala Tyr Arg Thr Phe His Ala Leu Lys Gly Leu Thr Thr
    50                  55                  60

Ser Arg Gly Glu Pro Val Gln Ala Val Tyr Gly Phe Ala Lys Ser Leu
65                  70                  75                  80

Leu Lys Ala Leu Lys Glu Asp Gly Asp Ala Val Ile Val Val Phe Asp

                85                  90                  95

Ala Lys Ala Pro Ser Phe Arg His Glu Ala Tyr Gly Gly Tyr Lys Ala
            100                 105                 110

Gly Arg Ala Pro Thr Pro Glu Asp Phe Pro Arg Gln Leu Ala Leu Ile
        115                 120                 125

Lys Glu Leu Val Asp Leu Leu Gly Leu Ala Arg Leu Glu Val Pro Gly
    130                 135                 140

Tyr Glu Ala Asp Asp Val Leu Ala Ser Leu Ala Lys Lys Ala Glu Lys
145                 150                 155                 160

Glu Gly Tyr Glu Val Arg Ile Leu Thr Ala Asp Lys Asp Leu Tyr Gln
                165                 170                 175

Leu Leu Ser Asp Arg Ile His Ala Leu His Pro Glu Gly Tyr Leu Ile
            180                 185                 190

Thr Pro Ala Trp Leu Trp Glu Lys Tyr Gly Leu Arg Pro Asp Gln Trp
        195                 200                 205

Ala Asp Tyr Arg Ala Leu Thr Gly Asp Glu Ser Asp Asn Leu Pro Gly
    210                 215                 220

Val Lys Gly Ile Gly Glu Lys Thr Ala Arg Lys Leu Leu Glu Glu Trp
225                 230                 235                 240

Gly Ser Leu Glu Ala Leu Leu Lys Asn Leu Asp Arg Leu Lys Pro Ala
                245                 250                 255

Ile Arg Glu Lys Ile Leu Ala His Met Asp Asp Leu Lys Leu Ser Trp
            260                 265                 270

Asp Leu Ala Lys Val Arg Thr Asp Leu Pro Leu Glu Val Asp Phe Ala
        275                 280                 285

Lys Arg Arg Glu Pro Asp Arg Glu Arg Leu Arg Ala Phe Leu Glu Arg
    290                 295                 300

Leu Glu Phe Gly Ser Leu Leu His Glu Phe Gly Leu Leu Glu Ser Pro
305                 310                 315                 320

Lys Ala Leu Glu Glu Ala Pro Trp Pro Pro Pro Glu Gly Ala Phe Val
                325                 330                 335

Gly Phe Val Leu Ser Arg Lys Glu Pro Met Trp Ala Asp Leu Leu Ala
        340                 345                 350

Leu Ala Ala Ala Arg Gly Gly Arg Val His Arg Ala Pro Glu Pro Tyr
    355                 360                 365

Lys Ala Leu Arg Asp Leu Lys Glu Ala Arg Gly Leu Leu Ala Lys Asp
    370                 375                 380

Leu Ser Val Leu Ala Leu Arg Glu Gly Leu Gly Leu Pro Pro Gly Asp
385                 390                 395                 400

Asp Pro Met Leu Leu Ala Tyr Leu Leu Asp Pro Ser Asn Thr Thr Pro
                405                 410                 415

Glu Gly Val Ala Arg Arg Tyr Gly Gly Glu Trp Thr Glu Glu Ala Gly
        420                 425                 430

Glu Arg Ala Ala Leu Ser Glu Arg Leu Phe Ala Asn Leu Trp Gly Arg
    435                 440                 445

Leu Glu Gly Glu Glu Arg Leu Leu Trp Leu Tyr Arg Glu Val Glu Arg
    450                 455                 460

Pro Leu Ser Ala Val Leu Ala His Met Glu Ala Thr Gly Val Arg Leu
465                 470                 475                 480

Asp Val Ala Tyr Leu Arg Ala Leu Ser Leu Glu Val Ala Glu Glu Ile
                485                 490                 495

Ala Arg Leu Glu Ala Glu Val Phe Arg Leu Ala Gly His Pro Phe Asn
        500                 505                 510

Leu Asn Ser Arg Asp Gln Leu Glu Arg Val Leu Phe Asp Glu Leu Gly
    515                 520                 525

Leu Pro Ala Ile Gly Lys Thr Glu Lys Thr Gly Lys Arg Ser Thr Ser
    530                 535                 540

Ala Ala Val Leu Glu Ala Leu Arg Glu Ala His Pro Ile Val Glu Lys
545                 550                 555                 560

Ile Leu Gln Tyr Arg Glu Leu Thr Lys Leu Lys Ser Thr Tyr Ile Asp
                565                 570                 575

Pro Leu Pro Asp Leu Ile His Pro Arg Thr Gly Arg Leu His Thr Arg
        580                 585                 590

Phe Asn Gln Thr Ala Thr Ala Thr Gly Arg Leu Ser Ser Ser Asp Pro
        595                 600                 605

Asn Leu Gln Asn Ile Pro Val Arg Thr Pro Leu Gly Gln Arg Ile Arg
    610                 615                 620

Arg Ala Phe Ile Ala Glu Glu Gly Trp Leu Leu Val Ala Leu Asp Tyr
625                 630                 635                 640

Ser Gln Ile Glu Leu Arg Val Leu Ala His Leu Ser Gly Asp Glu Asn
                645                 650                 655

Leu Ile Arg Val Phe Gln Glu Gly Arg Asp Ile His Thr Glu Thr Ala
            660                 665                 670

Ser Trp Met Phe Gly Val Pro Arg Glu Ala Val Asp Pro Leu Met Arg
        675                 680                 685

Arg Ala Ala Lys Thr Ile Asn Phe Gly Val Leu Tyr Gly Met Ser Ala
    690                 695                 700

His Arg Leu Ser Gln Glu Leu Ala Ile Pro Tyr Glu Glu Ala Gln Ala
705                 710                 715                 720

Phe Ile Glu Arg Tyr Phe Gln Ser Phe Pro Lys Val Arg Ala Trp Ile
                725                 730                 735

Glu Lys Thr Leu Glu Glu Gly Arg Arg Arg Gly Tyr Val Glu Thr Leu
            740                 745                 750

Phe Gly Arg Arg Arg Tyr Val Pro Asp Leu Glu Ala Arg Val Lys Ser
        755                 760                 765

Val Arg Glu Ala Ala Glu Arg Met Ala Phe Asn Met Pro Val Gln Gly
    770                 775                 780

Thr Ala Ala Asp Leu Met Lys Leu Ala Met Val Lys Leu Phe Pro Arg
785                 790                 795                 800

Leu Glu Glu Met Gly Ala Arg Met Leu Leu Gln Val His Asp Glu Leu
                805                 810                 815

Val Leu Glu Ala Pro Lys Glu Arg Ala Glu Ala Val Ala Arg Leu Ala
            820                 825                 830

Lys Glu Val Met Glu Gly Val Tyr Pro Leu Ala Val Pro Leu Glu Val
        835                 840                 845

Glu Val Gly Ile Gly Glu Asp Trp Leu Ser Ala Lys Glu
    850                 855                 860

<210>  9
<211>  2586
<212>  DNA
<213>  Artificial Sequence

<220>
<223>  DNA polymerase variant Taq83

<400>  9
atgagcggca agcaggcgac gctgttcgac ttcctcaaga agggctctgg tagcggtagc      60

ggttctggct ccggtagcgg ctccggcatg cgtggtatgc tgccgctgtt cgaacccaag     120

ggccgggtcc tcctggtgga cggccaccac ctggcctacc gcaccttcca cgccctgaag     180

ggcctcacca ccagccgggg ggagccggtg caggcggtct acggcttcgc caagagcctc     240

ctcaaggccc tcaaggagga cggggacgcg gtgatcgtgg tctttgacgc caaggccccc     300

tccttccgcc acgaggccta cggggggtac aaggcgggcc gggcccccac gccggaggac     360

tttccccggc aactcgccct catcaaggag ctggtggacc tcctggggct ggcgcgcctc     420

gaggtcccgg gctacgaggc ggacgacgtc ctggccagcc tggccaagaa ggcggaaaag     480

gagggctacg aggtccgcat cctcaccgcc gacaaagacc tttaccagct cctttccgac     540

cgcatccacg ccctccaccc cgaggggtac ctcatcaccc cggcctggct ttgggaaaag     600

tacggcctga ggcccgacca gtgggccgac taccgggccc tgaccgggga cgagtccgac     660

aaccttcccg gggtcaaggg catcggggag aagacggcga ggaagcttct ggaggagtgg     720

gggagcctgg aagccctcct caagaacctg gaccggctga agcccgccat ccgggagaag     780

atcctggccc acatggacga tctgaagctc tcctgggacc tggccaaggt gcgcaccgac     840

ctgcccctgg aggtggactt cgccaaaagg cgggagcccg accgggagag gcttagggcc     900

tttctggaga ggcttgagtt tggcagcctc ctccacgagt tcggccttct ggaaagcccc     960

aaggccctgg aggaggcccc ctggcccccg ccggaagggg ccttcgtggg ctttgtgctt    1020

tcccgcaagg agcccatgtg ggccgatctt ctggccctgg ccgccgccag ggggggccgg    1080

gtccaccggg cccccgagcc ttataaagcc ctcagggacc tgaaggaggc gcgggggctt    1140

ctcgccaaag acctgagcgt tctggccctg agggaaggcc ttggcctccc gcccggcgac    1200

gaccccatgc tcctcgccta cctcctggac ccttccaaca ccacccccga gggggtggcc    1260

cggcgctacg gcggggagtg gacggaggag gcgggggagc gggccgccct ttccgagagg    1320

ctcttcgcca acctgtgggg gaggcttgag ggggaggaga ggctcctttg gctttaccgg    1380

gaggtggaga ggcccctttc cgctgtcctg gcccacatgg aggccacggg ggtgcgcctg    1440

gacgtggcct atctcagggc cttgtccctg gaggtggccg aggagatcgc ccgcctcgag 1500

gccgaggtct tccgcctggc cggccacccc ttcaacctca actcccggga ccagctggaa 1560

agggtcctct ttgacgagct agggcttccc gccatcggca agacggagaa gaccggcaag 1620

cgctccacca gcgccgccgt cctggaggcc ctccgcgagg cccaccccat cgtggagaag 1680

atcctgcagt accgggagct caccaagctg aagagcacct acattgaccc cttgccggac 1740

ctcatccacc ccaggacggg ccgcctccac acccgcttca accagacggc cacggccacg 1800

ggcaggctaa gtagctccga tcccaacctc cagaacatcc ccgtccgcac cccgcttggg 1860

cagaggatcc gccgggcctt catcgccgag gaggggtggc tattggtggc cctggactat 1920

agccagatag agctcagggt gctggcccac ctctccggcg acgagaacct gatccgggtc 1980

ttccaggagg ggcgggacat ccacacggag accgccagct ggatgttcgg cgtcccccgg 2040

gaggccgtgg accccctgat gcgccgggcg gccaagacca tcaacttcgg ggtcctctac 2100

ggcatgtcgg cccaccgcct ctcccaggag ctagccatcc cttacgagga ggcccaggcc 2160

ttcattgagc gctactttca gagcttcccc aaggtgcggg cctggattga gaagaccctg 2220

gaggagggca ggaggcgggg gtacgtggag accctcttcg gccgccgccg ctacgtgcca 2280

gacctagagg cccgggtgaa gagcgtgcgg gaggcggccg agcgcatggc cttcaacatg 2340

cccgtccagg gcaccgccgc cgacctcatg aagctggcta tggtgaagct cttccccagg 2400

ctggaggaaa tgggggccag gatgctcctt caggtccacg acgagctggt cctcgaggcc 2460

ccaaaagaga gggcggaggc cgtggcccgg ctggccaagg aggtcatgga gggggtgtat 2520

cccctggccg tgcccctgga ggtggaggtg gggatagggg aggactggct ctccgccaag 2580

gagtga 2586

<210> 10
<211> 881
<212> PRT
<213> Artificial Sequence

<220>
<223> DNA polymerase variant Taq84

<400> 10

Met Ser Gly Lys Gln Ala Thr Leu Phe Asp Phe Leu Lys Lys Gly Ser
1               5                   10                  15

Gly Ser Gly Ser Gly Ser Gly Ser Gly Ser Gly Ser Gly Ser Gly Ser
            20                  25                  30

Gly Ser Gly Ser Gly Ser Gly Ser Gly Ser Gly Ser Gly Ser Gly Ser
        35                  40                  45

Gly Met Arg Gly Met Leu Pro Leu Phe Glu Pro Lys Gly Arg Val Leu
    50                  55                  60

Leu Val Asp Gly His His Leu Ala Tyr Arg Thr Phe His Ala Leu Lys
65                  70                  75                  80

Gly Leu Thr Thr Ser Arg Gly Glu Pro Val Gln Ala Val Tyr Gly Phe
                85                  90                  95

Ala Lys Ser Leu Leu Lys Ala Leu Lys Glu Asp Gly Asp Ala Val Ile
            100                 105                 110

Val Val Phe Asp Ala Lys Ala Pro Ser Phe Arg His Glu Ala Tyr Gly
        115                 120                 125

Gly Tyr Lys Ala Gly Arg Ala Pro Thr Pro Glu Asp Phe Pro Arg Gln
    130                 135                 140

Leu Ala Leu Ile Lys Glu Leu Val Asp Leu Leu Gly Leu Ala Arg Leu
145                 150                 155                 160

Glu Val Pro Gly Tyr Glu Ala Asp Asp Val Leu Ala Ser Leu Ala Lys
                165                 170                 175

Lys Ala Glu Lys Glu Gly Tyr Glu Val Arg Ile Leu Thr Ala Asp Lys
            180                 185                 190

Asp Leu Tyr Gln Leu Leu Ser Asp Arg Ile His Ala Leu His Pro Glu
        195                 200                 205

Gly Tyr Leu Ile Thr Pro Ala Trp Leu Trp Glu Lys Tyr Gly Leu Arg
    210                 215                 220

Pro Asp Gln Trp Ala Asp Tyr Arg Ala Leu Thr Gly Asp Glu Ser Asp
225                 230                 235                 240

Asn Leu Pro Gly Val Lys Gly Ile Gly Glu Lys Thr Ala Arg Lys Leu
                245                 250                 255

Leu Glu Glu Trp Gly Ser Leu Glu Ala Leu Leu Lys Asn Leu Asp Arg
            260                 265                 270

Leu Lys Pro Ala Ile Arg Glu Lys Ile Leu Ala His Met Asp Asp Leu
        275                 280                 285

Lys Leu Ser Trp Asp Leu Ala Lys Val Arg Thr Asp Leu Pro Leu Glu
    290                 295                 300

Val Asp Phe Ala Lys Arg Arg Glu Pro Asp Arg Glu Arg Leu Arg Ala
305                 310                 315                 320

Phe Leu Glu Arg Leu Glu Phe Gly Ser Leu Leu His Glu Phe Gly Leu
                325                 330                 335

Leu Glu Ser Pro Lys Ala Leu Glu Glu Ala Pro Trp Pro Pro Pro Glu
            340                 345                 350

Gly Ala Phe Val Gly Phe Val Leu Ser Arg Lys Glu Pro Met Trp Ala
        355                 360                 365

Asp Leu Leu Ala Leu Ala Ala Ala Arg Gly Gly Arg Val His Arg Ala
    370                 375                 380

Pro Glu Pro Tyr Lys Ala Leu Arg Asp Leu Lys Glu Ala Arg Gly Leu
385                 390                 395                 400

Leu Ala Lys Asp Leu Ser Val Leu Ala Leu Arg Glu Gly Leu Gly Leu
                405                 410                 415

Pro Pro Gly Asp Asp Pro Met Leu Leu Ala Tyr Leu Leu Asp Pro Ser
            420                 425                 430

Asn Thr Thr Pro Glu Gly Val Ala Arg Arg Tyr Gly Gly Glu Trp Thr
        435                 440                 445

Glu Glu Ala Gly Glu Arg Ala Ala Leu Ser Glu Arg Leu Phe Ala Asn
    450                 455                 460

Leu Trp Gly Arg Leu Glu Gly Glu Glu Arg Leu Leu Trp Leu Tyr Arg
465                 470                 475                 480

Glu Val Glu Arg Pro Leu Ser Ala Val Leu Ala His Met Glu Ala Thr
                485                 490                 495

Gly Val Arg Leu Asp Val Ala Tyr Leu Arg Ala Leu Ser Leu Glu Val
            500                 505                 510

Ala Glu Glu Ile Ala Arg Leu Glu Ala Glu Val Phe Arg Leu Ala Gly
        515                 520                 525

His Pro Phe Asn Leu Asn Ser Arg Asp Gln Leu Glu Arg Val Leu Phe
    530                 535                 540

Asp Glu Leu Gly Leu Pro Ala Ile Gly Lys Thr Glu Lys Thr Gly Lys
545                 550                 555                 560

Arg Ser Thr Ser Ala Ala Val Leu Glu Ala Leu Arg Glu Ala His Pro
                565                 570                 575

Ile Val Glu Lys Ile Leu Gln Tyr Arg Glu Leu Thr Lys Leu Lys Ser
            580                 585                 590

Thr Tyr Ile Asp Pro Leu Pro Asp Leu Ile His Pro Arg Thr Gly Arg
        595                 600                 605

Leu His Thr Arg Phe Asn Gln Thr Ala Thr Ala Thr Gly Arg Leu Ser
    610                 615                 620

Ser Ser Asp Pro Asn Leu Gln Asn Ile Pro Val Arg Thr Pro Leu Gly
625                 630                 635                 640

Gln Arg Ile Arg Arg Ala Phe Ile Ala Glu Glu Gly Trp Leu Leu Val
                645                 650                 655

Ala Leu Asp Tyr Ser Gln Ile Glu Leu Arg Val Leu Ala His Leu Ser
            660                 665                 670

Gly Asp Glu Asn Leu Ile Arg Val Phe Gln Glu Gly Arg Asp Ile His
        675                 680                 685

Thr Glu Thr Ala Ser Trp Met Phe Gly Val Pro Arg Glu Ala Val Asp
    690                 695                 700

Pro Leu Met Arg Arg Ala Ala Lys Thr Ile Asn Phe Gly Val Leu Tyr
705                 710                 715                 720

Gly Met Ser Ala His Arg Leu Ser Gln Glu Leu Ala Ile Pro Tyr Glu
                725                 730                 735

Glu Ala Gln Ala Phe Ile Glu Arg Tyr Phe Gln Ser Phe Pro Lys Val
            740                 745                 750

Arg Ala Trp Ile Glu Lys Thr Leu Glu Glu Gly Arg Arg Arg Gly Tyr
        755                 760                 765

Val Glu Thr Leu Phe Gly Arg Arg Arg Tyr Val Pro Asp Leu Glu Ala
    770                 775                 780

Arg Val Lys Ser Val Arg Glu Ala Ala Glu Arg Met Ala Phe Asn Met
785                 790                 795                 800

Pro Val Gln Gly Thr Ala Ala Asp Leu Met Lys Leu Ala Met Val Lys

805                 810                 815

Leu Phe Pro Arg Leu Glu Glu Met Gly Ala Arg Met Leu Leu Gln Val
            820                 825                 830

His Asp Glu Leu Val Leu Glu Ala Pro Lys Glu Arg Ala Glu Ala Val
        835                 840                 845

Ala Arg Leu Ala Lys Glu Val Met Glu Gly Val Tyr Pro Leu Ala Val
    850                 855                 860

Pro Leu Glu Val Glu Val Gly Ile Gly Glu Asp Trp Leu Ser Ala Lys
865                 870                 875                 880

Glu

<210>  11
<211>  2646
<212>  DNA
<213>  Artificial Sequence

<220>
<223>  DNA polymerase variant Taq84

<400>  11
atgagcggca agcaggcgac gctgttcgac ttcctcaaga agggttctgg ttccggctcc      60

ggcagcggct ctggctccgg ctccggtagc ggttccggta gcggctctgg tagcggtagc     120

ggttctggct ccggtagcgg ctccggcatg cgtggtatgc tgccgctgtt cgaacccaag     180

ggccgggtcc tcctggtgga cggccaccac ctggcctacc gcaccttcca cgccctgaag     240

ggcctcacca ccagccgggg ggagccggtg caggcggtct acggcttcgc caagagcctc     300

ctcaaggccc tcaaggagga cggggacgcg gtgatcgtgg tctttgacgc caaggccccc     360

tccttccgcc acgaggccta cggggggtac aaggcgggcc gggcccccac gccggaggac     420

tttccccggc aactcgccct catcaaggag ctggtggacc tcctggggct ggcgcgcctc     480

gaggtcccgg gctacgaggc ggacgacgtc ctggccagcc tggccaagaa ggcggaaaag     540

gagggctacg aggtccgcat cctcaccgcc gacaaagacc tttaccagct cctttccgac     600

cgcatccacg ccctccaccc cgaggggtac ctcatcaccc cggcctggct ttgggaaaag     660

tacggcctga ggcccgacca gtgggccgac taccgggccc tgaccgggga cgagtccgac     720

aaccttcccg gggtcaaggg catcggggag aagacggcga ggaagcttct ggaggagtgg     780

gggagcctgg aagccctcct caagaacctg gaccggctga agcccgccat ccgggagaag     840

atcctggccc acatggacga tctgaagctc tcctgggacc tggccaaggt gcgcaccgac     900

ctgcccctgg aggtggactt cgccaaaagg cgggagcccg accgggagag gcttagggcc     960

tttctggaga ggcttgagtt tggcagcctc ctccacgagt tcggccttct ggaaagcccc 1020

aaggccctgg aggaggcccc ctggcccccg ccggaagggg ccttcgtggg ctttgtgctt 1080

tcccgcaagg agcccatgtg ggccgatctt ctggccctgg ccgccgccag ggggggccgg 1140

gtccaccggg cccccgagcc ttataaagcc ctcagggacc tgaaggaggc gcgggggctt 1200

ctcgccaaag acctgagcgt tctggccctg agggaaggcc ttggcctccc gcccggcgac 1260

gaccccatgc tcctcgccta cctcctggac ccttccaaca ccacccccga gggggtggcc 1320

cggcgctacg gcggggagtg gacggaggag gcgggggagc gggccgccct ttccgagagg 1380

ctcttcgcca acctgtgggg gaggcttgag ggggaggaga ggctcctttg gctttaccgg 1440

gaggtggaga ggcccctttc cgctgtcctg gcccacatgg aggccacggg ggtgcgcctg 1500

gacgtggcct atctcagggc cttgtccctg gaggtggccg aggagatcgc ccgcctcgag 1560

gccgaggtct tccgcctggc cggccacccc ttcaacctca actcccggga ccagctggaa 1620

agggtcctct ttgacgagct agggcttccc gccatcggca agacggagaa gaccggcaag 1680

cgctccacca gcgccgccgt cctggaggcc ctccgcgagg cccaccccat cgtggagaag 1740

atcctgcagt accgggagct caccaagctg aagagcacct acattgaccc cttgccggac 1800

ctcatccacc ccaggacggg ccgcctccac acccgcttca accagacggc cacggccacg 1860

ggcaggctaa gtagctccga tcccaacctc cagaacatcc ccgtccgcac cccgcttggg 1920

cagaggatcc gccgggcctt catcgccgag gaggggtggc tattggtggc cctggactat 1980

agccagatag agctcagggt gctggcccac ctctccggcg acgagaacct gatccgggtc 2040

ttccaggagg ggcgggacat ccacacggag accgccagct ggatgttcgg cgtcccccgg 2100

gaggccgtgg accccctgat gcgccgggcg gccaagacca tcaacttcgg ggtcctctac 2160

ggcatgtcgg cccaccgcct ctcccaggag ctagccatcc cttacgagga ggcccaggcc 2220

ttcattgagc gctactttca gagcttcccc aaggtgcggg cctggattga gaagaccctg 2280

gaggagggca ggaggcgggg gtacgtggag accctcttcg gccgccgccg ctacgtgcca 2340

gacctagagg cccgggtgaa gagcgtgcgg gaggcggccg agcgcatggc cttcaacatg 2400

cccgtccagg gcaccgccgc cgacctcatg aagctggcta tggtgaagct cttccccagg 2460

ctggaggaaa tgggggccag gatgctcctt caggtccacg acgagctggt cctcgaggcc 2520

ccaaaagaga gggcggaggc cgtggcccgg ctggccaagg aggtcatgga gggggtgtat 2580

cccctggccg tgcccctgga ggtggaggtg gggatagggg aggactggct ctccgccaag 2640

gagtga 2646

<210> 12
<211> 893
<212> PRT

<213>  Artificial Sequence

<220>
<223>  DNA polymerase variant Taq85

<400>  12

Met Ser Gly Lys Gln Ala Thr Leu Phe Asp Phe Leu Lys Lys Gly Ser
1               5                   10                  15

Gly Ser Gly Ser Gly Ser Gly Ser Gly Ser Gly Ser Gly Ser Gly Ser
            20                  25                  30

Gly Ser Gly Ser Gly Ser Gly Ser Gly Ser Gly Ser Gly Ser Gly Ser
        35                  40                  45

Gly Ser Gly Ser Gly Ser Gly Ser Gly Ser Gly Ser Gly Met Arg Gly
    50                  55                  60

Met Leu Pro Leu Phe Glu Pro Lys Gly Arg Val Leu Leu Val Asp Gly
65                  70                  75                  80

His His Leu Ala Tyr Arg Thr Phe His Ala Leu Lys Gly Leu Thr Thr
                85                  90                  95

Ser Arg Gly Glu Pro Val Gln Ala Val Tyr Gly Phe Ala Lys Ser Leu
            100                 105                 110

Leu Lys Ala Leu Lys Glu Asp Gly Asp Ala Val Ile Val Val Phe Asp
        115                 120                 125

Ala Lys Ala Pro Ser Phe Arg His Glu Ala Tyr Gly Gly Tyr Lys Ala
    130                 135                 140

Gly Arg Ala Pro Thr Pro Glu Asp Phe Pro Arg Gln Leu Ala Leu Ile
145                 150                 155                 160

Lys Glu Leu Val Asp Leu Leu Gly Leu Ala Arg Leu Glu Val Pro Gly
                165                 170                 175

Tyr Glu Ala Asp Asp Val Leu Ala Ser Leu Ala Lys Lys Ala Glu Lys
            180                 185                 190

Glu Gly Tyr Glu Val Arg Ile Leu Thr Ala Asp Lys Asp Leu Tyr Gln
        195                 200                 205

Leu Leu Ser Asp Arg Ile His Ala Leu His Pro Glu Gly Tyr Leu Ile
    210                 215                 220

Thr Pro Ala Trp Leu Trp Glu Lys Tyr Gly Leu Arg Pro Asp Gln Trp
225                 230                 235                 240

Ala Asp Tyr Arg Ala Leu Thr Gly Asp Glu Ser Asp Asn Leu Pro Gly
                245                 250                 255

Val Lys Gly Ile Gly Glu Lys Thr Ala Arg Lys Leu Leu Glu Glu Trp
            260                 265                 270

Gly Ser Leu Glu Ala Leu Leu Lys Asn Leu Asp Arg Leu Lys Pro Ala
        275                 280                 285

Ile Arg Glu Lys Ile Leu Ala His Met Asp Asp Leu Lys Leu Ser Trp
    290                 295                 300

Asp Leu Ala Lys Val Arg Thr Asp Leu Pro Leu Glu Val Asp Phe Ala
305                 310                 315                 320

Lys Arg Arg Glu Pro Asp Arg Glu Arg Leu Arg Ala Phe Leu Glu Arg
                325                 330                 335

Leu Glu Phe Gly Ser Leu Leu His Glu Phe Gly Leu Leu Glu Ser Pro
            340                 345                 350

Lys Ala Leu Glu Glu Ala Pro Trp Pro Pro Pro Glu Gly Ala Phe Val
        355                 360                 365

Gly Phe Val Leu Ser Arg Lys Glu Pro Met Trp Ala Asp Leu Leu Ala
    370                 375                 380

Leu Ala Ala Ala Arg Gly Gly Arg Val His Arg Ala Pro Glu Pro Tyr
385                 390                 395                 400

Lys Ala Leu Arg Asp Leu Lys Glu Ala Arg Gly Leu Leu Ala Lys Asp
                405                 410                 415

Leu Ser Val Leu Ala Leu Arg Glu Gly Leu Gly Leu Pro Pro Gly Asp
            420                 425                 430

Asp Pro Met Leu Leu Ala Tyr Leu Leu Asp Pro Ser Asn Thr Thr Pro
        435                 440                 445

Glu Gly Val Ala Arg Arg Tyr Gly Gly Glu Trp Thr Glu Glu Ala Gly
    450                 455                 460

Glu Arg Ala Ala Leu Ser Glu Arg Leu Phe Ala Asn Leu Trp Gly Arg
465                 470                 475                 480

Leu Glu Gly Glu Glu Arg Leu Leu Trp Leu Tyr Arg Glu Val Glu Arg
                485                 490                 495

Pro Leu Ser Ala Val Leu Ala His Met Glu Ala Thr Gly Val Arg Leu
            500                 505                 510

Asp Val Ala Tyr Leu Arg Ala Leu Ser Leu Glu Val Ala Glu Glu Ile
        515                 520                 525

Ala Arg Leu Glu Ala Glu Val Phe Arg Leu Ala Gly His Pro Phe Asn
    530                 535                 540

Leu Asn Ser Arg Asp Gln Leu Glu Arg Val Leu Phe Asp Glu Leu Gly
545                 550                 555                 560

Leu Pro Ala Ile Gly Lys Thr Glu Lys Thr Gly Lys Arg Ser Thr Ser
                565                 570                 575

Ala Ala Val Leu Glu Ala Leu Arg Glu Ala His Pro Ile Val Glu Lys
            580                 585                 590

Ile Leu Gln Tyr Arg Glu Leu Thr Lys Leu Lys Ser Thr Tyr Ile Asp
        595                 600                 605

Pro Leu Pro Asp Leu Ile His Pro Arg Thr Gly Arg Leu His Thr Arg
    610                 615                 620

Phe Asn Gln Thr Ala Thr Ala Thr Gly Arg Leu Ser Ser Ser Asp Pro
625                 630                 635                 640

Asn Leu Gln Asn Ile Pro Val Arg Thr Pro Leu Gly Gln Arg Ile Arg
                645                 650                 655

Arg Ala Phe Ile Ala Glu Glu Gly Trp Leu Leu Val Ala Leu Asp Tyr
            660                 665                 670

Ser Gln Ile Glu Leu Arg Val Leu Ala His Leu Ser Gly Asp Glu Asn
        675                 680                 685

Leu Ile Arg Val Phe Gln Glu Gly Arg Asp Ile His Thr Glu Thr Ala
    690                 695                 700

Ser Trp Met Phe Gly Val Pro Arg Glu Ala Val Asp Pro Leu Met Arg
705                 710                 715                 720

Arg Ala Ala Lys Thr Ile Asn Phe Gly Val Leu Tyr Gly Met Ser Ala
                725                 730                 735

His Arg Leu Ser Gln Glu Leu Ala Ile Pro Tyr Glu Glu Ala Gln Ala
            740                 745                 750

Phe Ile Glu Arg Tyr Phe Gln Ser Phe Pro Lys Val Arg Ala Trp Ile
        755                 760                 765

Glu Lys Thr Leu Glu Glu Gly Arg Arg Arg Gly Tyr Val Glu Thr Leu
    770                 775                 780

Phe Gly Arg Arg Arg Tyr Val Pro Asp Leu Glu Ala Arg Val Lys Ser
785                 790                 795                 800

Val Arg Glu Ala Ala Glu Arg Met Ala Phe Asn Met Pro Val Gln Gly
                805                 810                 815

Thr Ala Ala Asp Leu Met Lys Leu Ala Met Val Lys Leu Phe Pro Arg
            820                 825                 830

Leu Glu Glu Met Gly Ala Arg Met Leu Leu Gln Val His Asp Glu Leu
        835                 840                 845

Val Leu Glu Ala Pro Lys Glu Arg Ala Glu Ala Val Ala Arg Leu Ala
    850                 855                 860

Lys Glu Val Met Glu Gly Val Tyr Pro Leu Ala Val Pro Leu Glu Val
865                 870                 875                 880

Glu Val Gly Ile Gly Glu Asp Trp Leu Ser Ala Lys Glu
                885                 890

<210>  13
<211>  2682
<212>  DNA
<213>  Artificial Sequence

<220>
<223>  DNA polymerase variant Taq85

<400>  13
atgagcggca agcaggcgac gctgttcgac ttcctcaaga agggttctgg ttccggctcc      60

ggcagcggct ctggctccgg ctccggtagc ggttccggta gcggctctgg tagcggctct     120

ggtagcggtt ccggtagcgg ctctggtagc ggtagcggtt ctggctccgg tagcggctcc     180

ggcatgcgtg gtatgctgcc gctgttcgaa cccaagggcc gggtcctcct ggtggacggc     240

caccacctgg cctaccgcac cttccacgcc ctgaagggcc tcaccaccag ccgcggggag      300

ccggtgcagg cggtctacgg cttcgccaag agcctcctca aggccctcaa ggaggacggg     360

gacgcggtga tcgtggtctt tgacgccaag gccccctcct tccgccacga ggcctacggg 420

gggtacaagg cgggccgggc ccccacgccg gaggactttc cccggcaact cgccctcatc 480

aaggagctgg tggacctcct ggggctggcg cgcctcgagg tcccgggcta cgaggcggac 540

gacgtcctgg ccagcctggc caagaaggcg gaaaaggagg gctacgaggt ccgcatcctc 600

accgccgaca aagaccttta ccagctcctt tccgaccgca tccacgccct ccaccccgag 660

gggtacctca tcaccccggc ctggctttgg gaaaagtacg gcctgaggcc cgaccagtgg 720

gccgactacc gggccctgac cggggacgag tccgacaacc ttcccggggt caagggcatc 780

ggggagaaga cggcgaggaa gcttctggag gagtggggga gcctggaagc cctcctcaag 840

aacctggacc ggctgaagcc cgccatccgg gagaagatcc tggcccacat ggacgatctg 900

aagctctcct gggacctggc caaggtgcgc accgacctgc ccctggaggt ggacttcgcc 960

aaaaggcggg agcccgaccg ggagaggctt agggcctttc tggagaggct tgagtttggc 1020

agcctcctcc acgagttcgg ccttctggaa agccccaagg ccctggagga ggccccctgg 1080

cccccgccgg aaggggcctt cgtgggcttt gtgctttccc gcaaggagcc catgtgggcc 1140

gatcttctgg ccctggccgc cgccaggggg ggccgggtcc accgggcccc cgagccttat 1200

aaagccctca gggacctgaa ggaggcgcgg gggcttctcg ccaaagacct gagcgttctg 1260

gccctgaggg aaggccttgg cctcccgccc ggcgacgacc ccatgctcct cgcctacctc 1320

ctggaccctt ccaacaccac ccccgagggg gtggcccggc gctacggcgg ggagtggacg 1380

gaggaggcgg gggagcgggc cgccctttcc gagaggctct tcgccaacct gtgggggagg 1440

cttgaggggg aggagaggct cctttggctt taccgggagg tggagaggcc cctttccgct 1500

gtcctggccc acatggaggc cacgggggtg cgcctggacg tggcctatct cagggccttg 1560

tccctggagg tggccgagga gatcgcccgc ctcgaggccg aggtcttccg cctggccggc 1620

caccccttca acctcaactc ccgggaccag ctggaaaggg tcctctttga cgagctaggg 1680

cttcccgcca tcggcaagac ggagaagacc ggcaagcgct ccaccagcgc cgccgtcctg 1740

gaggccctcc gcgaggccca ccccatcgtg gagaagatcc tgcagtaccg ggagctcacc 1800

aagctgaaga gcacctacat tgaccccttg ccggacctca tccaccccag gacgggccgc 1860

ctccacaccc gcttcaacca gacggccacg gccacgggca ggctaagtag ctccgatccc 1920

aacctccaga acatccccgt ccgcaccccg cttgggcaga ggatccgccg ggccttcatc 1980

gccgaggagg ggtggctatt ggtggccctg gactatagcc agatagagct cagggtgctg 2040

gcccacctct ccggcgacga gaacctgatc cgggtcttcc aggaggggcg ggacatccac 2100

acggagaccg ccagctggat gttcggcgtc ccccgggagg ccgtggaccc cctgatgcgc 2160

cgggcggcca agaccatcaa cttcggggtc ctctacggca tgtcggccca ccgcctctcc 2220

caggagctag ccatccctta cgaggaggcc caggccttca ttgagcgcta ctttcagagc 2280

ttccccaagg tgcgggcctg gattgagaag accctggagg agggcaggag gcgggggtac 2340

gtggagaccc tcttcggccg ccgccgctac gtgccagacc tagaggcccg ggtgaagagc 2400

gtgcgggagg cggccgagcg catggccttc aacatgcccg tccagggcac cgccgccgac 2460

ctcatgaagc tggctatggt gaagctcttc cccaggctgg aggaaatggg ggccaggatg 2520

ctccttcagg tccacgacga gctggtcctc gaggccccaa aagagagggc ggaggccgtg 2580

gcccggctgg ccaaggaggt catggagggg gtgtatcccc tggccgtgcc cctggaggtg 2640

gaggtgggga taggggagga ctggctctcc gccaaggagt ga 2682

<210> 14
<211> 848
<212> PRT
<213> Artificial Sequence

<220>
<223> DNA polymerase variant Taq92

<400> 14

Met Arg Gly Met Leu Pro Leu Phe Glu Pro Lys Gly Arg Val Leu Leu
1               5                   10                  15

Val Asp Gly His His Leu Ala Tyr Arg Thr Phe His Ala Leu Lys Gly
            20                  25                  30

Leu Thr Thr Ser Arg Gly Glu Pro Val Gln Ala Val Tyr Gly Phe Ala
        35                  40                  45

Lys Ser Leu Leu Lys Ala Leu Lys Glu Asp Gly Asp Ala Val Ile Val
    50                  55                  60

Val Phe Asp Ala Lys Ala Pro Ser Phe Arg His Glu Ala Tyr Gly Gly
65                  70                  75                  80

Tyr Lys Ala Gly Arg Ala Pro Thr Pro Glu Asp Phe Pro Arg Gln Leu
                85                  90                  95

Ala Leu Ile Lys Glu Leu Val Asp Leu Leu Gly Leu Ala Arg Leu Glu
            100                 105                 110

Val Pro Gly Tyr Glu Ala Asp Asp Val Leu Ala Ser Leu Ala Lys Lys
        115                 120                 125

Ala Glu Lys Glu Gly Tyr Glu Val Arg Ile Leu Thr Ala Asp Lys Asp
    130                 135                 140

Leu Tyr Gln Leu Leu Ser Asp Arg Ile His Ala Leu His Pro Glu Gly

145                 150                 155                 160

Tyr Leu Ile Thr Pro Ala Trp Leu Trp Glu Lys Tyr Gly Leu Arg Pro
                165                 170                 175

Asp Gln Trp Ala Asp Tyr Arg Ala Leu Thr Gly Asp Glu Ser Asp Asn
            180                 185                 190

Leu Pro Gly Val Lys Gly Ile Gly Glu Lys Thr Ala Arg Lys Leu Leu
        195                 200                 205

Glu Glu Trp Gly Ser Leu Glu Ala Leu Leu Lys Asn Leu Asp Arg Leu
    210                 215                 220

Lys Pro Ala Ile Arg Glu Lys Ile Leu Ala His Met Asp Asp Leu Lys
225                 230                 235                 240

Leu Ser Trp Asp Leu Ala Lys Val Arg Thr Asp Leu Pro Leu Glu Val
                245                 250                 255

Asp Phe Ala Lys Arg Arg Glu Pro Asp Arg Glu Arg Leu Arg Ala Phe
            260                 265                 270

Leu Glu Arg Leu Glu Phe Gly Ser Leu Leu His Glu Phe Gly Leu Leu
        275                 280                 285

Glu Ser Pro Lys Ala Leu Glu Glu Ala Pro Trp Pro Pro Pro Glu Gly
    290                 295                 300

Ala Phe Val Gly Phe Val Leu Ser Arg Lys Glu Pro Met Trp Ala Asp
305                 310                 315                 320

Leu Leu Ala Leu Ala Ala Ala Arg Gly Gly Arg Val His Arg Ala Pro
                325                 330                 335

Glu Pro Tyr Lys Ala Leu Arg Asp Leu Lys Glu Ala Arg Gly Leu Leu
            340                 345                 350

Ala Lys Asp Leu Ser Val Leu Ala Leu Arg Glu Gly Leu Gly Leu Pro
        355                 360                 365

Pro Gly Asp Asp Pro Met Leu Leu Ala Tyr Leu Leu Asp Pro Ser Asn
    370                 375                 380

Thr Thr Pro Glu Gly Val Ala Arg Arg Tyr Gly Gly Glu Trp Thr Glu
385                 390                 395                 400

Glu Ala Gly Glu Arg Ala Ala Leu Ser Glu Arg Leu Phe Ala Asn Leu
                405                 410                 415

Trp Gly Arg Leu Glu Gly Glu Glu Arg Leu Leu Trp Leu Tyr Arg Glu
            420                 425                 430

Val Glu Arg Pro Leu Ser Ala Val Leu Ala His Met Glu Ala Thr Gly
        435                 440                 445

Val Arg Leu Asp Val Ala Tyr Leu Arg Ala Leu Ser Leu Glu Val Ala
    450                 455                 460

Glu Glu Ile Ala Arg Leu Glu Ala Glu Val Phe Arg Leu Ala Gly His
465                 470                 475                 480

Pro Phe Asn Leu Asn Ser Arg Asp Gln Leu Glu Arg Val Leu Phe Asp
                485                 490                 495

Glu Leu Gly Leu Pro Ala Ile Gly Lys Thr Glu Lys Thr Gly Lys Arg
            500                 505                 510

Ser Thr Ser Ala Ala Val Leu Glu Ala Leu Arg Glu Ala His Pro Ile
        515                 520                 525

Val Glu Lys Ile Leu Gln Tyr Arg Glu Leu Thr Lys Leu Lys Ser Thr
    530                 535                 540

Tyr Ile Asp Pro Leu Pro Asp Leu Ile His Pro Arg Thr Gly Arg Leu
545                 550                 555                 560

His Thr Arg Phe Asn Gln Thr Ala Thr Ala Thr Gly Arg Leu Ser Ser
                565                 570                 575

Ser Asp Pro Asn Leu Gln Asn Ile Pro Val Arg Thr Pro Leu Gly Gln
            580                 585                 590

Arg Ile Arg Arg Ala Phe Ile Ala Glu Glu Gly Trp Leu Leu Val Ala
        595                 600                 605

Leu Asp Tyr Ser Gln Ile Glu Leu Arg Val Leu Ala His Leu Ser Gly
    610                 615                 620

Asp Glu Asn Leu Ile Arg Val Phe Gln Glu Gly Arg Asp Ile His Thr
625                 630                 635                 640

Glu Thr Ala Ser Trp Met Phe Gly Val Pro Arg Glu Ala Val Asp Pro
                645                 650                 655

Leu Met Arg Arg Ala Ala Lys Thr Ile Asn Phe Gly Val Leu Tyr Gly
            660                 665                 670

Met Ser Ala His Arg Leu Ser Gln Glu Leu Ala Ile Pro Tyr Glu Glu
        675                 680                 685

Ala Gln Ala Phe Ile Glu Arg Tyr Phe Gln Ser Phe Pro Lys Val Arg
    690                 695                 700

Ala Trp Ile Glu Lys Thr Leu Glu Glu Gly Arg Arg Arg Gly Tyr Val
705                 710                 715                 720

Glu Thr Leu Phe Gly Arg Arg Arg Tyr Val Pro Asp Leu Glu Ala Arg
                725                 730                 735

Val Lys Ser Val Arg Glu Ala Ala Glu Arg Met Ala Phe Asn Met Pro
            740                 745                 750

Val Gln Gly Thr Ala Ala Asp Leu Met Lys Leu Ala Met Val Lys Leu
        755                 760                 765

Phe Pro Arg Leu Glu Glu Met Gly Ala Arg Met Leu Leu Gln Val His
    770                 775                 780

Asp Glu Leu Val Leu Glu Ala Pro Lys Glu Arg Ala Glu Ala Val Ala
785                 790                 795                 800

Arg Leu Ala Lys Glu Val Met Glu Gly Val Tyr Pro Leu Ala Val Pro
                805                 810                 815

Leu Glu Val Glu Val Gly Ile Gly Glu Asp Trp Leu Ser Ala Lys Glu
            820                 825                 830

Ser Gly Ser Gly Ser Lys Gln Ala Thr Leu Phe Asp Phe Leu Lys Lys
        835                 840                 845

<210>  15
<211>  2547
<212>  DNA
<213>  Artificial Sequence

<220>
<223>  DNA polymerase variant Taq92

<400>  15
atgcgtggta tgctgccgct gttcgaaccc aagggccggg tcctcctggt ggacggccac      60

cacctggcct accgcacctt ccacgccctg aagggcctca ccaccagccg gggggagccg     120

gtgcaggcgg tctacggctt cgccaagagc ctcctcaagg ccctcaagga ggacggggac     180

gcggtgatcg tggtctttga cgccaaggcc ccctccttcc gccacgaggc ctacgggggg 240

tacaaggcgg gccgggcccc cacgccggag gactttcccc ggcaactcgc cctcatcaag 300

gagctggtgg acctcctggg gctggcgcgc ctcgaggtcc cgggctacga ggcggacgac 360

gtcctggcca gcctggccaa gaaggcggaa aaggagggct acgaggtccg catcctcacc 420

gccgacaaag acctttacca gctcctttcc gaccgcatcc acgccctcca ccccgagggg 480

tacctcatca ccccggcctg gctttgggaa aagtacggcc tgaggcccga ccagtgggcc 540

gactaccggg ccctgaccgg ggacgagtcc gacaaccttc ccggggtcaa gggcatcggg 600

gagaagacgg cgaggaagct tctggaggag tgggggagcc tggaagccct cctcaagaac 660

ctggaccggc tgaagcccgc catccgggag aagatcctgg cccacatgga cgatctgaag 720

ctctcctggg acctggccaa ggtgcgcacc gacctgcccc tggaggtgga cttcgccaaa 780

aggcgggagc ccgaccggga gaggcttagg gcctttctgg agaggcttga gtttggcagc 840

ctcctccacg agttcggcct tctggaaagc cccaaggccc tggaggaggc cccctggccc 900

ccgccggaag gggccttcgt gggctttgtg ctttcccgca aggagcccat gtgggccgat 960

cttctggccc tggccgccgc cagggggggc cgggtccacc gggcccccga gccttataaa 1020

gccctcaggg acctgaagga ggcgcggggg cttctcgcca aagacctgag cgttctggcc 1080

ctgagggaag gccttggcct cccgcccggc gacgacccca tgctcctcgc ctacctcctg 1140

gacccttcca acaccacccc cgagggggtg gcccggcgct acggcgggga gtggacggag 1200

gaggcggggg agcgggccgc cctttccgag aggctcttcg ccaacctgtg ggggaggctt 1260

gagggggagg agaggctcct ttggctttac cgggaggtgg agaggcccct ttccgctgtc 1320

ctggcccaca tggaggccac gggggtgcgc ctggacgtgg cctatctcag ggccttgtcc 1380

ctggaggtgg ccgaggagat cgcccgcctc gaggccgagg tcttccgcct ggccggccac 1440

cccttcaacc tcaactcccg ggaccagctg gaaagggtcc tctttgacga gctagggctt 1500

cccgccatcg gcaagacgga gaagaccggc aagcgctcca ccagcgccgc cgtcctggag 1560

gccctccgcg aggcccaccc catcgtggag aagatcctgc agtaccggga gctcaccaag 1620

ctgaagagca cctacattga ccccttgccg gacctcatcc accccaggac gggccgcctc 1680

cacacccgct tcaaccagac ggccacggcc acgggcaggc taagtagctc cgatcccaac 1740

ctccagaaca tccccgtccg caccccgctt gggcagagga tccgccgggc cttcatcgcc 1800

gaggaggggt ggctattggt ggccctggac tatagccaga tagagctcag ggtgctggcc 1860

cacctctccg gcgacgagaa cctgatccgg gtcttccagg aggggcggga catccacacg 1920

gagaccgcca gctggatgtt cggcgtcccc cgggaggccg tggaccccct gatgcgccgg 1980

gcggccaaga ccatcaactt cggggtcctc tacggcatgt cggcccaccg cctctcccag 2040

gagctagcca tcccttacga ggaggcccag gccttcattg agcgctactt tcagagcttc 2100

cccaaggtgc gggcctggat tgagaagacc ctggaggagg gcaggaggcg ggggtacgtg 2160

gagaccctct tcggccgccg ccgctacgtg ccagacctag aggcccgggt gaagagcgtg 2220

cgggaggcgg ccgagcgcat ggccttcaac atgcccgtcc agggcaccgc cgccgacctc 2280

atgaagctgg ctatggtgaa gctcttcccc aggctggagg aaatgggggc caggatgctc 2340

cttcaggtcc acgacgagct ggtcctcgag gccccaaaag agagggcgga ggccgtggcc 2400

cggctggcca aggaggtcat ggagggggtg tatcccctgg ccgtgcccct ggaggtggag 2460

gtggggatag gggaggactg gctctccgcc aaggagagcg gttctggctc caagcaggcg 2520

acgctgttcg acttcctcaa gaagtga 2547

<210> 16
<211> 853
<212> PRT
<213> Artificial Sequence

<220>
<223> DNA polymerase variant Taq93

<400> 16

Met Arg Gly Met Leu Pro Leu Phe Glu Pro Lys Gly Arg Val Leu Leu
1               5                   10                  15

Val Asp Gly His His Leu Ala Tyr Arg Thr Phe His Ala Leu Lys Gly
            20                  25                  30

Leu Thr Thr Ser Arg Gly Glu Pro Val Gln Ala Val Tyr Gly Phe Ala
        35                  40                  45

Lys Ser Leu Leu Lys Ala Leu Lys Glu Asp Gly Asp Ala Val Ile Val
    50                  55                  60

Val Phe Asp Ala Lys Ala Pro Ser Phe Arg His Glu Ala Tyr Gly Gly
65                  70                  75                  80

Tyr Lys Ala Gly Arg Ala Pro Thr Pro Glu Asp Phe Pro Arg Gln Leu
                85                  90                  95

Ala Leu Ile Lys Glu Leu Val Asp Leu Leu Gly Leu Ala Arg Leu Glu
            100                 105                 110

Val Pro Gly Tyr Glu Ala Asp Asp Val Leu Ala Ser Leu Ala Lys Lys
        115                 120                 125

Ala Glu Lys Glu Gly Tyr Glu Val Arg Ile Leu Thr Ala Asp Lys Asp
    130                 135                 140

Leu Tyr Gln Leu Leu Ser Asp Arg Ile His Ala Leu His Pro Glu Gly
145                 150                 155                 160

Tyr Leu Ile Thr Pro Ala Trp Leu Trp Glu Lys Tyr Gly Leu Arg Pro
                165                 170                 175

Asp Gln Trp Ala Asp Tyr Arg Ala Leu Thr Gly Asp Glu Ser Asp Asn
            180                 185                 190

Leu Pro Gly Val Lys Gly Ile Gly Glu Lys Thr Ala Arg Lys Leu Leu
        195                 200                 205

Glu Glu Trp Gly Ser Leu Glu Ala Leu Leu Lys Asn Leu Asp Arg Leu
    210                 215                 220

Lys Pro Ala Ile Arg Glu Lys Ile Leu Ala His Met Asp Asp Leu Lys
225                 230                 235                 240

Leu Ser Trp Asp Leu Ala Lys Val Arg Thr Asp Leu Pro Leu Glu Val
                245                 250                 255

Asp Phe Ala Lys Arg Arg Glu Pro Asp Arg Glu Arg Leu Arg Ala Phe
            260                 265                 270

Leu Glu Arg Leu Glu Phe Gly Ser Leu Leu His Glu Phe Gly Leu Leu
        275                 280                 285

Glu Ser Pro Lys Ala Leu Glu Glu Ala Pro Trp Pro Pro Pro Glu Gly
    290                 295                 300

Ala Phe Val Gly Phe Val Leu Ser Arg Lys Glu Pro Met Trp Ala Asp
305                 310                 315                 320

Leu Leu Ala Leu Ala Ala Ala Arg Gly Gly Arg Val His Arg Ala Pro
                325                 330                 335

Glu Pro Tyr Lys Ala Leu Arg Asp Leu Lys Glu Ala Arg Gly Leu Leu
            340                 345                 350

Ala Lys Asp Leu Ser Val Leu Ala Leu Arg Glu Gly Leu Gly Leu Pro
        355                 360                 365

Pro Gly Asp Asp Pro Met Leu Leu Ala Tyr Leu Leu Asp Pro Ser Asn
    370                 375                 380

Thr Thr Pro Glu Gly Val Ala Arg Arg Tyr Gly Gly Glu Trp Thr Glu

385                 390                 395                 400

Glu Ala Gly Glu Arg Ala Ala Leu Ser Glu Arg Leu Phe Ala Asn Leu
                405                 410                 415

Trp Gly Arg Leu Glu Gly Glu Glu Arg Leu Leu Trp Leu Tyr Arg Glu
            420                 425                 430

Val Glu Arg Pro Leu Ser Ala Val Leu Ala His Met Glu Ala Thr Gly
        435                 440                 445

Val Arg Leu Asp Val Ala Tyr Leu Arg Ala Leu Ser Leu Glu Val Ala
    450                 455                 460

Glu Glu Ile Ala Arg Leu Glu Ala Glu Val Phe Arg Leu Ala Gly His
465                 470                 475                 480

Pro Phe Asn Leu Asn Ser Arg Asp Gln Leu Glu Arg Val Leu Phe Asp
                485                 490                 495

Glu Leu Gly Leu Pro Ala Ile Gly Lys Thr Glu Lys Thr Gly Lys Arg
            500                 505                 510

Ser Thr Ser Ala Ala Val Leu Glu Ala Leu Arg Glu Ala His Pro Ile
        515                 520                 525

Val Glu Lys Ile Leu Gln Tyr Arg Glu Leu Thr Lys Leu Lys Ser Thr
    530                 535                 540

Tyr Ile Asp Pro Leu Pro Asp Leu Ile His Pro Arg Thr Gly Arg Leu
545                 550                 555                 560

His Thr Arg Phe Asn Gln Thr Ala Thr Ala Thr Gly Arg Leu Ser Ser
                565                 570                 575

Ser Asp Pro Asn Leu Gln Asn Ile Pro Val Arg Thr Pro Leu Gly Gln
            580                 585                 590

Arg Ile Arg Arg Ala Phe Ile Ala Glu Glu Gly Trp Leu Leu Val Ala
        595                 600                 605

Leu Asp Tyr Ser Gln Ile Glu Leu Arg Val Leu Ala His Leu Ser Gly
    610                 615                 620

Asp Glu Asn Leu Ile Arg Val Phe Gln Glu Gly Arg Asp Ile His Thr
625                 630                 635                 640

Glu Thr Ala Ser Trp Met Phe Gly Val Pro Arg Glu Ala Val Asp Pro
                645                 650                 655

Leu Met Arg Arg Ala Ala Lys Thr Ile Asn Phe Gly Val Leu Tyr Gly
            660                 665                 670

Met Ser Ala His Arg Leu Ser Gln Glu Leu Ala Ile Pro Tyr Glu Glu
        675                 680                 685

Ala Gln Ala Phe Ile Glu Arg Tyr Phe Gln Ser Phe Pro Lys Val Arg
    690                 695                 700

Ala Trp Ile Glu Lys Thr Leu Glu Glu Gly Arg Arg Arg Gly Tyr Val
705                 710                 715                 720

Glu Thr Leu Phe Gly Arg Arg Arg Tyr Val Pro Asp Leu Glu Ala Arg
                725                 730                 735

Val Lys Ser Val Arg Glu Ala Ala Glu Arg Met Ala Phe Asn Met Pro
            740                 745                 750

Val Gln Gly Thr Ala Ala Asp Leu Met Lys Leu Ala Met Val Lys Leu
        755                 760                 765

Phe Pro Arg Leu Glu Glu Met Gly Ala Arg Met Leu Leu Gln Val His
    770                 775                 780

Asp Glu Leu Val Leu Glu Ala Pro Lys Glu Arg Ala Glu Ala Val Ala
785                 790                 795                 800

Arg Leu Ala Lys Glu Val Met Glu Gly Val Tyr Pro Leu Ala Val Pro
                805                 810                 815

Leu Glu Val Glu Val Gly Ile Gly Glu Asp Trp Leu Ser Ala Lys Glu
            820                 825                 830

Ser Gly Ser Gly Ser Ser Gly Ser Gly Ser Lys Gln Ala Thr Leu Phe
        835                 840                 845

Asp Phe Leu Lys Lys
    850

<210>  17
<211>  2562
<212>  DNA
<213>  Artificial Sequence

<220>
<223>  DNA polymerase variant Taq93

<400> 17
atgcgtggta tgctgccgct gttcgaaccc aagggccggg tcctcctggt ggacggccac 60

cacctggcct accgcacctt ccacgccctg aagggcctca ccaccagccg gggggagccg 120

gtgcaggcgg tctacggctt cgccaagagc ctcctcaagg ccctcaagga ggacggggac 180

gcggtgatcg tggtctttga cgccaaggcc ccctccttcc gccacgaggc ctacgggggg 240

tacaaggcgg gccgggcccc cacgccggag gactttcccc ggcaactcgc cctcatcaag 300

gagctggtgg acctcctggg gctggcgcgc ctcgaggtcc cgggctacga ggcggacgac 360

gtcctggcca gcctggccaa gaaggcggaa aaggagggct acgaggtccg catcctcacc 420

gccgacaaag acctttacca gctcctttcc gaccgcatcc acgccctcca ccccgagggg 480

tacctcatca ccccggcctg gctttgggaa aagtacggcc tgaggcccga ccagtgggcc 540

gactaccggg ccctgaccgg ggacgagtcc gacaaccttc ccggggtcaa gggcatcggg 600

gagaagacgg cgaggaagct tctggaggag tggggagccc tggaagccct cctcaagaac 660

ctggaccggc tgaagcccgc catccgggag aagatcctgg cccacatgga cgatctgaag 720

ctctcctggg acctggccaa ggtgcgcacc gacctgcccc tggaggtgga cttcgccaaa 780

aggcgggagc ccgaccggga gaggcttagg gcctttctgg agaggcttga gtttggcagc 840

ctcctccacg agttcggcct tctggaaagc cccaaggccc tggaggaggc cccctggccc 900

ccgccggaag gggccttcgt gggctttgtg ctttcccgca aggagcccat gtgggccgat 960

cttctggccc tggccgccgc cagggggggc cgggtccacc gggcccccga gccttataaa 1020

gccctcaggg acctgaagga ggcgcggggg cttctcgcca aagacctgag cgttctggcc 1080

ctgagggaag gccttggcct cccgcccggc gacgacccca tgctcctcgc ctacctcctg 1140

gacccttcca acaccacccc cgagggggtg gcccggcgct acggcgggga gtggacggag 1200

gaggcggggg agcgggccgc cctttccgag aggctcttcg ccaacctgtg ggggaggctt 1260

gagggggagg agaggctcct ttggctttac cgggaggtgg agaggcccct ttccgctgtc 1320

ctggcccaca tggaggccac gggggtgcgc ctggacgtgg cctatctcag ggccttgtcc 1380

ctggaggtgg ccgaggagat cgcccgcctc gaggccgagg tcttccgcct ggccggccac 1440

cccttcaacc tcaactcccg ggaccagctg gaaagggtcc tctttgacga gctagggctt 1500

cccgccatcg gcaagacgga gaagaccggc aagcgctcca ccagcgccgc cgtcctggag 1560

gccctccgcg aggcccaccc catcgtggag aagatcctgc agtaccggga gctcaccaag 1620

ctgaagagca cctacattga ccccttgccg gacctcatcc accccaggac gggccgcctc 1680

cacacccgct tcaaccagac ggccacggcc acgggcaggc taagtagctc cgatcccaac 1740

ctccagaaca tccccgtccg caccccgctt gggcagagga tccgccgggc cttcatcgcc 1800

gaggaggggt ggctattggt ggccctggac tatagccaga tagagctcag ggtgctggcc 1860

cacctctccg gcgacgagaa cctgatccgg gtcttccagg aggggcggga catccacacg 1920

gagaccgcca gctggatgtt cggcgtcccc cgggaggccg tggaccccct gatgcgccgg 1980

gcggccaaga ccatcaactt cggggtcctc tacggcatgt cggcccaccg cctctcccag 2040

gagctagcca tcccttacga ggaggcccag gccttcattg agcgctactt tcagagcttc 2100

cccaaggtgc gggcctggat tgagaagacc ctggaggagg gcaggaggcg ggggtacgtg 2160

gagaccctct tcggccgccg ccgctacgtg ccagacctag aggcccgggt gaagagcgtg 2220

cgggaggcgg ccgagcgcat ggccttcaac atgcccgtcc agggcaccgc cgccgacctc 2280

atgaagctgg ctatggtgaa gctcttcccc aggctggagg aaatgggggc caggatgctc 2340

cttcaggtcc acgacgagct ggtcctcgag gccccaaaag agagggcgga ggccgtggcc 2400

cggctggcca aggaggtcat ggagggggtg tatcccctgg ccgtgcccct ggaggtggag 2460

gtggggatag gggaggactg gctctccgcc aaggagagcg gttctggctc ctctggcagc 2520

ggttccaagc aggcgacgct gttcgacttc ctcaagaagt ga 2562

<210> 18
<211> 858
<212> PRT
<213> Artificial Sequence

<220>
<223> DNA polymerase variant Taq94

<400> 18

Met Arg Gly Met Leu Pro Leu Phe Glu Pro Lys Gly Arg Val Leu Leu
1               5                   10                  15

Val Asp Gly His His Leu Ala Tyr Arg Thr Phe His Ala Leu Lys Gly
            20                  25                  30

Leu Thr Thr Ser Arg Gly Glu Pro Val Gln Ala Val Tyr Gly Phe Ala
        35                  40                  45

Lys Ser Leu Leu Lys Ala Leu Lys Glu Asp Gly Asp Ala Val Ile Val
    50                  55                  60

Val Phe Asp Ala Lys Ala Pro Ser Phe Arg His Glu Ala Tyr Gly Gly
65                  70                  75                  80

Tyr Lys Ala Gly Arg Ala Pro Thr Pro Glu Asp Phe Pro Arg Gln Leu
                85                  90                  95

Ala Leu Ile Lys Glu Leu Val Asp Leu Leu Gly Leu Ala Arg Leu Glu
            100                 105                 110

Val Pro Gly Tyr Glu Ala Asp Asp Val Leu Ala Ser Leu Ala Lys Lys
        115                 120                 125

Ala Glu Lys Glu Gly Tyr Glu Val Arg Ile Leu Thr Ala Asp Lys Asp
    130                 135                 140

Leu Tyr Gln Leu Leu Ser Asp Arg Ile His Ala Leu His Pro Glu Gly
145                 150                 155                 160

Tyr Leu Ile Thr Pro Ala Trp Leu Trp Glu Lys Tyr Gly Leu Arg Pro
                165                 170                 175

Asp Gln Trp Ala Asp Tyr Arg Ala Leu Thr Gly Asp Glu Ser Asp Asn
            180                 185                 190

Leu Pro Gly Val Lys Gly Ile Gly Glu Lys Thr Ala Arg Lys Leu Leu
        195                 200                 205

Glu Glu Trp Gly Ser Leu Glu Ala Leu Leu Lys Asn Leu Asp Arg Leu
    210                 215                 220

Lys Pro Ala Ile Arg Glu Lys Ile Leu Ala His Met Asp Asp Leu Lys
225                 230                 235                 240

Leu Ser Trp Asp Leu Ala Lys Val Arg Thr Asp Leu Pro Leu Glu Val
                245                 250                 255

Asp Phe Ala Lys Arg Arg Glu Pro Asp Arg Glu Arg Leu Arg Ala Phe
            260                 265                 270

Leu Glu Arg Leu Glu Phe Gly Ser Leu Leu His Glu Phe Gly Leu Leu
        275                 280                 285

Glu Ser Pro Lys Ala Leu Glu Glu Ala Pro Trp Pro Pro Pro Glu Gly
    290                 295                 300

Ala Phe Val Gly Phe Val Leu Ser Arg Lys Glu Pro Met Trp Ala Asp
305                 310                 315                 320

Leu Leu Ala Leu Ala Ala Ala Arg Gly Gly Arg Val His Arg Ala Pro
                325                 330                 335

Glu Pro Tyr Lys Ala Leu Arg Asp Leu Lys Glu Ala Arg Gly Leu Leu
            340                 345                 350

Ala Lys Asp Leu Ser Val Leu Ala Leu Arg Glu Gly Leu Gly Leu Pro
        355                 360                 365

Pro Gly Asp Asp Pro Met Leu Leu Ala Tyr Leu Leu Asp Pro Ser Asn
    370                 375                 380

Thr Thr Pro Glu Gly Val Ala Arg Arg Tyr Gly Gly Glu Trp Thr Glu
385                 390                 395                 400

Glu Ala Gly Glu Arg Ala Ala Leu Ser Glu Arg Leu Phe Ala Asn Leu
                405                 410                 415

Trp Gly Arg Leu Glu Gly Glu Glu Arg Leu Leu Trp Leu Tyr Arg Glu
            420                 425                 430

Val Glu Arg Pro Leu Ser Ala Val Leu Ala His Met Glu Ala Thr Gly
        435                 440                 445

Val Arg Leu Asp Val Ala Tyr Leu Arg Ala Leu Ser Leu Glu Val Ala
    450                 455                 460

Glu Glu Ile Ala Arg Leu Glu Ala Glu Val Phe Arg Leu Ala Gly His
465                 470                 475                 480

Pro Phe Asn Leu Asn Ser Arg Asp Gln Leu Glu Arg Val Leu Phe Asp
                485                 490                 495

Glu Leu Gly Leu Pro Ala Ile Gly Lys Thr Glu Lys Thr Gly Lys Arg
            500                 505                 510

Ser Thr Ser Ala Ala Val Leu Glu Ala Leu Arg Glu Ala His Pro Ile
        515                 520                 525

Val Glu Lys Ile Leu Gln Tyr Arg Glu Leu Thr Lys Leu Lys Ser Thr
    530                 535                 540

Tyr Ile Asp Pro Leu Pro Asp Leu Ile His Pro Arg Thr Gly Arg Leu
545                 550                 555                 560

His Thr Arg Phe Asn Gln Thr Ala Thr Ala Thr Gly Arg Leu Ser Ser
                565                 570                 575

Ser Asp Pro Asn Leu Gln Asn Ile Pro Val Arg Thr Pro Leu Gly Gln
            580                 585                 590

Arg Ile Arg Arg Ala Phe Ile Ala Glu Glu Gly Trp Leu Leu Val Ala
        595                 600                 605

Leu Asp Tyr Ser Gln Ile Glu Leu Arg Val Leu Ala His Leu Ser Gly

610                 615                 620

Asp Glu Asn Leu Ile Arg Val Phe Gln Glu Gly Arg Asp Ile His Thr
625                 630                 635                 640

Glu Thr Ala Ser Trp Met Phe Gly Val Pro Arg Glu Ala Val Asp Pro
                645                 650                 655

Leu Met Arg Arg Ala Ala Lys Thr Ile Asn Phe Gly Val Leu Tyr Gly
            660                 665                 670

Met Ser Ala His Arg Leu Ser Gln Glu Leu Ala Ile Pro Tyr Glu Glu
        675                 680                 685

Ala Gln Ala Phe Ile Glu Arg Tyr Phe Gln Ser Phe Pro Lys Val Arg
    690                 695                 700

Ala Trp Ile Glu Lys Thr Leu Glu Glu Gly Arg Arg Arg Gly Tyr Val
705                 710                 715                 720

Glu Thr Leu Phe Gly Arg Arg Arg Tyr Val Pro Asp Leu Glu Ala Arg
                725                 730                 735

Val Lys Ser Val Arg Glu Ala Ala Glu Arg Met Ala Phe Asn Met Pro
            740                 745                 750

Val Gln Gly Thr Ala Ala Asp Leu Met Lys Leu Ala Met Val Lys Leu
        755                 760                 765

Phe Pro Arg Leu Glu Glu Met Gly Ala Arg Met Leu Leu Gln Val His
    770                 775                 780

Asp Glu Leu Val Leu Glu Ala Pro Lys Glu Arg Ala Glu Ala Val Ala
785                 790                 795                 800

Arg Leu Ala Lys Glu Val Met Glu Gly Val Tyr Pro Leu Ala Val Pro
                805                 810                 815

Leu Glu Val Glu Val Gly Ile Gly Glu Asp Trp Leu Ser Ala Lys Glu
            820                 825                 830

Ser Gly Ser Gly Ser Ser Gly Ser Gly Ser Gly Ser Gly Ser Gly Lys
        835                 840                 845

Gln Ala Thr Leu Phe Asp Phe Leu Lys Lys
    850                 855

<210>  19
<211>  2577
<212>  DNA
<213>  Artificial Sequence

<220>
<223>  DNA polymerase variant Taq94

<400>  19
atgcgtggta tgctgccgct gttcgaaccc aagggccggg tcctcctggt ggacggccac      60

cacctggcct accgcacctt ccacgccctg aagggcctca ccaccagccg gggggagccg     120

gtgcaggcgg tctacggctt cgccaagagc ctcctcaagg ccctcaagga ggacggggac     180

gcggtgatcg tggtctttga cgccaaggcc ccctccttcc gccacgaggc ctacgggggg     240

tacaaggcgg gccgggcccc cacgccggag gactttcccc ggcaactcgc cctcatcaag     300

gagctggtgg acctcctggg gctggcgcgc ctcgaggtcc cgggctacga ggcggacgac     360

gtcctggcca gcctggccaa gaaggcggaa aaggagggct acgaggtccg catcctcacc     420

gccgacaaag acctttacca gctcctttcc gaccgcatcc acgccctcca ccccgagggg     480

tacctcatca ccccggcctg gctttgggaa aagtacggcc tgaggcccga ccagtgggcc     540

gactaccggg ccctgaccgg ggacgagtcc gacaaccttc ccggggtcaa gggcatcggg     600

gagaagacgg cgaggaagct tctggaggag tgggggagcc tggaagccct cctcaagaac     660

ctggaccggc tgaagcccgc catccgggag aagatcctgg cccacatgga cgatctgaag     720

ctctcctggg acctggccaa ggtgcgcacc gacctgcccc tggaggtgga cttcgccaaa     780

aggcgggagc ccgaccggga gaggcttagg gcctttctgg agaggcttga gtttggcagc     840

ctcctccacg agttcggcct tctggaaagc cccaaggccc tggaggaggc cccctggccc     900

ccgccggaag gggccttcgt gggctttgtg ctttcccgca aggagcccat gtgggccgat     960

cttctggccc tggccgccgc cagggggggc cgggtccacc gggcccccga gccttataaa    1020

gccctcaggg acctgaagga ggcgcggggg cttctcgcca aagacctgag cgttctggcc    1080

ctgagggaag gccttggcct cccgcccggc gacgacccca tgctcctcgc ctacctcctg    1140

gacccttcca acaccacccc cgagggggtg gcccggcgct acggcgggga gtggacggag    1200

gaggcggggg agcgggccgc cctttccgag aggctcttcg ccaacctgtg ggggaggctt    1260

gagggggagg agaggctcct ttggctttac cgggaggtgg agaggcccct ttccgctgtc    1320

ctggcccaca tggaggccac gggggtgcgc ctggacgtgg cctatctcag ggccttgtcc    1380

ctggaggtgg ccgaggagat cgcccgcctc gaggccgagg tcttccgcct ggccggccac    1440

cccttcaacc tcaactcccg ggaccagctg gaaagggtcc tctttgacga gctagggctt    1500

cccgccatcg gcaagacgga gaagaccggc aagcgctcca ccagcgccgc cgtcctggag    1560

gccctccgcg aggcccaccc catcgtggag aagatcctgc agtaccggga gctcaccaag    1620

ctgaagagca cctacattga ccccttgccg gacctcatcc accccaggac gggccgcctc 1680

cacacccgct tcaaccagac ggccacggcc acgggcaggc taagtagctc cgatcccaac 1740

ctccagaaca tccccgtccg caccccgctt gggcagagga tccgccgggc cttcatcgcc 1800

gaggaggggt ggctattggt ggccctggac tatagccaga tagagctcag ggtgctggcc 1860

cacctctccg gcgacgagaa cctgatccgg gtcttccagg aggggcggga catccacacg 1920

gagaccgcca gctggatgtt cggcgtcccc cgggaggccg tggaccccct gatgcgccgg 1980

gcggccaaga ccatcaactt cggggtcctc tacggcatgt cggcccaccg cctctcccag 2040

gagctagcca tcccttacga ggaggcccag gccttcattg agcgctactt tcagagcttc 2100

cccaaggtgc gggcctggat tgagaagacc ctggaggagg gcaggaggcg ggggtacgtg 2160

gagaccctct tcggccgccg ccgctacgtg ccagacctag aggcccgggt gaagagcgtg 2220

cgggaggcgg ccgagcgcat ggccttcaac atgcccgtcc agggcaccgc cgccgacctc 2280

atgaagctgg ctatggtgaa gctcttcccc aggctggagg aaatgggggc caggatgctc 2340

cttcaggtcc acgacgagct ggtcctcgag gccccaaaag agagggcgga ggccgtggcc 2400

cggctggcca aggaggtcat ggagggggtg tatcccctgg ccgtgcccct ggaggtggag 2460

gtggggatag gggaggactg gctctccgcc aaggagagcg gttctggctc ctctggcagc 2520

ggttccggta gcggctccgg caagcaggcg acgctgttcg acttcctcaa gaagtga 2577

<210> 20
<211> 885
<212> PRT
<213> Artificial Sequence

<220>
<223> DNA polymerase variant Taq95

<400> 20

Met Ser Gly Lys Gln Ala Thr Leu Phe Asp Phe Leu Lys Ser Gly Ser
1               5                   10                  15

Gly Ser Gly Ser Gly Ser Gly Ser Gly Ser Gly Lys Gln Ala Thr Leu
            20                  25                  30

Phe Asp Phe Leu Lys Lys Gly Ser Gly Ser Gly Ser Gly Ser Gly Ser
        35                  40                  45

Gly Ser Gly Ser Gly Met Arg Gly Met Leu Pro Leu Phe Glu Pro Lys
    50                  55                  60

Gly Arg Val Leu Leu Val Asp Gly His His Leu Ala Tyr Arg Thr Phe
65                  70                  75                  80

His Ala Leu Lys Gly Leu Thr Thr Ser Arg Gly Glu Pro Val Gln Ala
                85                  90                  95

Val Tyr Gly Phe Ala Lys Ser Leu Leu Lys Ala Leu Lys Glu Asp Gly
            100                 105                 110

Asp Ala Val Ile Val Val Phe Asp Ala Lys Ala Pro Ser Phe Arg His
        115                 120                 125

Glu Ala Tyr Gly Gly Tyr Lys Ala Gly Arg Ala Pro Thr Pro Glu Asp
    130                 135                 140

Phe Pro Arg Gln Leu Ala Leu Ile Lys Glu Leu Val Asp Leu Leu Gly
145                 150                 155                 160

Leu Ala Arg Leu Glu Val Pro Gly Tyr Glu Ala Asp Asp Val Leu Ala
                165                 170                 175

Ser Leu Ala Lys Lys Ala Glu Lys Glu Gly Tyr Glu Val Arg Ile Leu
            180                 185                 190

Thr Ala Asp Lys Asp Leu Tyr Gln Leu Leu Ser Asp Arg Ile His Ala
        195                 200                 205

Leu His Pro Glu Gly Tyr Leu Ile Thr Pro Ala Trp Leu Trp Glu Lys
    210                 215                 220

Tyr Gly Leu Arg Pro Asp Gln Trp Ala Asp Tyr Arg Ala Leu Thr Gly
225                 230                 235                 240

Asp Glu Ser Asp Asn Leu Pro Gly Val Lys Gly Ile Gly Glu Lys Thr
                245                 250                 255

Ala Arg Lys Leu Leu Glu Glu Trp Gly Ser Leu Glu Ala Leu Leu Lys
            260                 265                 270

Asn Leu Asp Arg Leu Lys Pro Ala Ile Arg Glu Lys Ile Leu Ala His
        275                 280                 285

Met Asp Asp Leu Lys Leu Ser Trp Asp Leu Ala Lys Val Arg Thr Asp
    290                 295                 300

Leu Pro Leu Glu Val Asp Phe Ala Lys Arg Arg Glu Pro Asp Arg Glu
305                 310                 315                 320

Arg Leu Arg Ala Phe Leu Glu Arg Leu Glu Phe Gly Ser Leu Leu His
                325                 330                 335

Glu Phe Gly Leu Leu Glu Ser Pro Lys Ala Leu Glu Glu Ala Pro Trp
            340                 345                 350

Pro Pro Pro Glu Gly Ala Phe Val Gly Phe Val Leu Ser Arg Lys Glu
        355                 360                 365

Pro Met Trp Ala Asp Leu Leu Ala Leu Ala Ala Ala Arg Gly Gly Arg
    370                 375                 380

Val His Arg Ala Pro Glu Pro Tyr Lys Ala Leu Arg Asp Leu Lys Glu
385                 390                 395                 400

Ala Arg Gly Leu Leu Ala Lys Asp Leu Ser Val Leu Ala Leu Arg Glu
                405                 410                 415

Gly Leu Gly Leu Pro Pro Gly Asp Asp Pro Met Leu Leu Ala Tyr Leu
            420                 425                 430

Leu Asp Pro Ser Asn Thr Thr Pro Glu Gly Val Ala Arg Arg Tyr Gly
        435                 440                 445

Gly Glu Trp Thr Glu Glu Ala Gly Glu Arg Ala Ala Leu Ser Glu Arg
    450                 455                 460

Leu Phe Ala Asn Leu Trp Gly Arg Leu Glu Gly Glu Glu Arg Leu Leu
465                 470                 475                 480

Trp Leu Tyr Arg Glu Val Glu Arg Pro Leu Ser Ala Val Leu Ala His
                485                 490                 495

Met Glu Ala Thr Gly Val Arg Leu Asp Val Ala Tyr Leu Arg Ala Leu
            500                 505                 510

Ser Leu Glu Val Ala Glu Glu Ile Ala Arg Leu Glu Ala Glu Val Phe
        515                 520                 525

Arg Leu Ala Gly His Pro Phe Asn Leu Asn Ser Arg Asp Gln Leu Glu
    530                 535                 540

Arg Val Leu Phe Asp Glu Leu Gly Leu Pro Ala Ile Gly Lys Thr Glu
545                 550                 555                 560

Lys Thr Gly Lys Arg Ser Thr Ser Ala Ala Val Leu Glu Ala Leu Arg
                565                 570                 575

Glu Ala His Pro Ile Val Glu Lys Ile Leu Gln Tyr Arg Glu Leu Thr
            580                 585                 590

Lys Leu Lys Ser Thr Tyr Ile Asp Pro Leu Pro Asp Leu Ile His Pro
        595                 600                 605

Arg Thr Gly Arg Leu His Thr Arg Phe Asn Gln Thr Ala Thr Ala Thr
    610                 615                 620

Gly Arg Leu Ser Ser Ser Asp Pro Asn Leu Gln Asn Ile Pro Val Arg
625                 630                 635                 640

Thr Pro Leu Gly Gln Arg Ile Arg Arg Ala Phe Ile Ala Glu Glu Gly
                645                 650                 655

Trp Leu Leu Val Ala Leu Asp Tyr Ser Gln Ile Glu Leu Arg Val Leu
            660                 665                 670

Ala His Leu Ser Gly Asp Glu Asn Leu Ile Arg Val Phe Gln Glu Gly
        675                 680                 685

Arg Asp Ile His Thr Glu Thr Ala Ser Trp Met Phe Gly Val Pro Arg
    690                 695                 700

Glu Ala Val Asp Pro Leu Met Arg Arg Ala Ala Lys Thr Ile Asn Phe
705                 710                 715                 720

Gly Val Leu Tyr Gly Met Ser Ala His Arg Leu Ser Gln Glu Leu Ala
                725                 730                 735

Ile Pro Tyr Glu Glu Ala Gln Ala Phe Ile Glu Arg Tyr Phe Gln Ser
            740                 745                 750

Phe Pro Lys Val Arg Ala Trp Ile Glu Lys Thr Leu Glu Glu Gly Arg
        755                 760                 765

Arg Arg Gly Tyr Val Glu Thr Leu Phe Gly Arg Arg Arg Tyr Val Pro
    770                 775                 780

Asp Leu Glu Ala Arg Val Lys Ser Val Arg Glu Ala Ala Glu Arg Met
785                 790                 795                 800

Ala Phe Asn Met Pro Val Gln Gly Thr Ala Ala Asp Leu Met Lys Leu
                805                 810                 815

Ala Met Val Lys Leu Phe Pro Arg Leu Glu Glu Met Gly Ala Arg Met
            820                 825                 830

Leu Leu Gln Val His Asp Glu Leu Val Leu Glu Ala Pro Lys Glu Arg

835                 840                 845

Ala Glu Ala Val Ala Arg Leu Ala Lys Glu Val Met Glu Gly Val Tyr
    850                 855                 860

Pro Leu Ala Val Pro Leu Glu Val Glu Val Gly Ile Gly Glu Asp Trp
865                 870                 875                 880

Leu Ser Ala Lys Glu
                885

<210>  21
<211>  2658
<212>  DNA
<213>  Artificial Sequence

<220>
<223>  DNA polymerase variant Taq95

<400>  21
atgtccggta aacaagccac ccttttcgat tttctgaaaa gcggttctgg ttctggcagc      60

ggtagcggct ccggcagcgg caagcaggcg acgctgttcg acttcctcaa gaagggctct     120

ggtagcggta gcggttctgg ctccggtagc ggctccggca tgcgtggtat gctgccgctg     180

ttcgaaccca agggccgggt cctcctggtg gacggccacc acctggccta ccgcaccttc     240

cacgccctga agggcctcac caccagccgg ggggagccgg tgcaggcggt ctacggcttc     300

gccaagagcc tcctcaaggc cctcaaggag gacggggacg cggtgatcgt ggtctttgac     360

gccaaggccc cctccttccg ccacgaggcc tacggggggt acaaggcggg ccgggccccc     420

acgccggagg actttccccg gcaactcgcc ctcatcaagg agctggtgga cctcctgggg     480

ctggcgcgcc tcgaggtccc gggctacgag gcggacgacg tcctggccag cctggccaag     540

aaggcggaaa aggagggcta cgaggtccgc atcctcaccg ccgacaaaga cctttaccag     600

ctcctttccg accgcatcca cgccctccac cccgaggggt acctcatcac cccggcctgg     660

ctttgggaaa agtacggcct gaggcccgac cagtgggccg actaccgggc cctgaccggg     720

gacgagtccg acaaccttcc cggggtcaag ggcatcgggg agaagacggc gaggaagctt     780

ctggaggagt gggggagcct ggaagccctc ctcaagaacc tggaccggct gaagcccgcc     840

atccgggaga agatcctggc ccacatggac gatctgaagc tctcctggga cctggccaag     900

gtgcgcaccg acctgcccct ggaggtggac ttcgccaaaa ggcgggagcc cgaccgggag     960

aggcttaggg cctttctgga gaggcttgag tttggcagcc tcctccacga gttcggcctt    1020

ctggaaagcc ccaaggccct ggaggaggcc ccctggcccc cgccggaagg ggccttcgtg    1080

ggctttgtgc tttcccgcaa ggagcccatg tgggccgatc ttctggccct ggccgccgcc    1140

agggggggcc gggtccaccg ggcccccgag ccttataaag ccctcaggga cctgaaggag    1200

gcgcgggggc ttctcgccaa agacctgagc gttctggccc tgagggaagg ccttggcctc 1260

ccgcccggcg acgaccccat gctcctcgcc tacctcctgg acccttccaa caccaccccc 1320

gagggggtgg cccggcgcta cggcggggag tggacggagg aggcggggga gcgggccgcc 1380

ctttccgaga ggctcttcgc caacctgtgg gggaggcttg agggggagga gaggctcctt 1440

tggctttacc gggaggtgga gaggcccctt tccgctgtcc tggcccacat ggaggccacg 1500

ggggtgcgcc tggacgtggc ctatctcagg gccttgtccc tggaggtggc cgaggagatc 1560

gcccgcctcg aggccgaggt cttccgcctg gccggccacc ccttcaacct caactcccgg 1620

gaccagctgg aaagggtcct ctttgacgag ctagggcttc ccgccatcgg caagacggag 1680

aagaccggca agcgctccac cagcgccgcc gtcctggagg ccctccgcga ggcccacccc 1740

atcgtggaga agatcctgca gtaccgggag ctcaccaagc tgaagagcac ctacattgac 1800

cccttgccgg acctcatcca ccccaggacg ggccgcctcc acacccgctt caaccagacg 1860

gccacggcca cgggcaggct aagtagctcc gatcccaacc tccagaacat ccccgtccgc 1920

accccgcttg ggcagaggat ccgccgggcc ttcatcgccg aggaggggtg gctattggtg 1980

gccctggact atagccagat agagctcagg gtgctggccc acctctccgg cgacgagaac 2040

ctgatccggg tcttccagga ggggcgggac atccacacgg agaccgccag ctggatgttc 2100

ggcgtccccc gggaggccgt ggaccccctg atgcgccggg cggccaagac catcaacttc 2160

ggggtcctct acggcatgtc ggcccaccgc ctctcccagg agctagccat cccttacgag 2220

gaggcccagg ccttcattga gcgctacttt cagagcttcc ccaaggtgcg ggcctggatt 2280

gagaagaccc tggaggaggg caggaggcgg gggtacgtgg agaccctctt cggccgccgc 2340

cgctacgtgc cagacctaga ggcccgggtg aagagcgtgc gggaggcggc cgagcgcatg 2400

gccttcaaca tgcccgtcca gggcaccgcc gccgacctca tgaagctggc tatggtgaag 2460

ctcttcccca ggctggagga aatgggggcc aggatgctcc ttcaggtcca cgacgagctg 2520

gtcctcgagg ccccaaaaga gagggcggag gccgtggccc ggctggccaa ggaggtcatg 2580

gagggggtgt atcccctggc cgtgcccctg gaggtggagg tggggatagg ggaggactgg 2640

ctctccgcca aggagtga 2658

<210> 22
<211> 909
<212> PRT
<213> Artificial Sequence

<220>
<223> DNA polymerase variant Taq96

<400> 22

Met Ser Gly Lys Gln Ala Thr Leu Phe Asp Phe Leu Lys Ser Gly Ser

1               5                   10                  15

Gly Ser Gly Ser Gly Ser Gly Ser Gly Ser Gly Lys Gln Ala Thr Leu
            20                  25                  30

Phe Asp Phe Leu Lys Ser Gly Ser Gly Ser Gly Ser Gly Ser Gly Ser
        35                  40                  45

Gly Ser Gly Lys Gln Ala Thr Leu Phe Asp Phe Leu Lys Lys Gly Ser
    50                  55                  60

Gly Ser Gly Ser Gly Ser Gly Ser Gly Ser Gly Ser Gly Met Arg Gly
65                  70                  75                  80

Met Leu Pro Leu Phe Glu Pro Lys Gly Arg Val Leu Leu Val Asp Gly
                85                  90                  95

His His Leu Ala Tyr Arg Thr Phe His Ala Leu Lys Gly Leu Thr Thr
            100                 105                 110

Ser Arg Gly Glu Pro Val Gln Ala Val Tyr Gly Phe Ala Lys Ser Leu
        115                 120                 125

Leu Lys Ala Leu Lys Glu Asp Gly Asp Ala Val Ile Val Val Phe Asp
    130                 135                 140

Ala Lys Ala Pro Ser Phe Arg His Glu Ala Tyr Gly Gly Tyr Lys Ala
145                 150                 155                 160

Gly Arg Ala Pro Thr Pro Glu Asp Phe Pro Arg Gln Leu Ala Leu Ile
                165                 170                 175

Lys Glu Leu Val Asp Leu Leu Gly Leu Ala Arg Leu Glu Val Pro Gly
            180                 185                 190

Tyr Glu Ala Asp Asp Val Leu Ala Ser Leu Ala Lys Lys Ala Glu Lys
        195                 200                 205

Glu Gly Tyr Glu Val Arg Ile Leu Thr Ala Asp Lys Asp Leu Tyr Gln
    210                 215                 220

Leu Leu Ser Asp Arg Ile His Ala Leu His Pro Glu Gly Tyr Leu Ile
225                 230                 235                 240

Thr Pro Ala Trp Leu Trp Glu Lys Tyr Gly Leu Arg Pro Asp Gln Trp
                245                 250                 255

Ala Asp Tyr Arg Ala Leu Thr Gly Asp Glu Ser Asp Asn Leu Pro Gly
            260                 265                 270

Val Lys Gly Ile Gly Glu Lys Thr Ala Arg Lys Leu Leu Glu Glu Trp
        275                 280                 285

Gly Ser Leu Glu Ala Leu Leu Lys Asn Leu Asp Arg Leu Lys Pro Ala
    290                 295                 300

Ile Arg Glu Lys Ile Leu Ala His Met Asp Asp Leu Lys Leu Ser Trp
305                 310                 315                 320

Asp Leu Ala Lys Val Arg Thr Asp Leu Pro Leu Glu Val Asp Phe Ala
                325                 330                 335

Lys Arg Arg Glu Pro Asp Arg Glu Arg Leu Arg Ala Phe Leu Glu Arg
            340                 345                 350

Leu Glu Phe Gly Ser Leu Leu His Glu Phe Gly Leu Leu Glu Ser Pro
        355                 360                 365

Lys Ala Leu Glu Glu Ala Pro Trp Pro Pro Pro Glu Gly Ala Phe Val
    370                 375                 380

Gly Phe Val Leu Ser Arg Lys Glu Pro Met Trp Ala Asp Leu Leu Ala
385                 390                 395                 400

Leu Ala Ala Ala Arg Gly Gly Arg Val His Arg Ala Pro Glu Pro Tyr
                405                 410                 415

Lys Ala Leu Arg Asp Leu Lys Glu Ala Arg Gly Leu Leu Ala Lys Asp
            420                 425                 430

Leu Ser Val Leu Ala Leu Arg Glu Gly Leu Gly Leu Pro Pro Gly Asp
        435                 440                 445

Asp Pro Met Leu Leu Ala Tyr Leu Leu Asp Pro Ser Asn Thr Thr Pro
    450                 455                 460

Glu Gly Val Ala Arg Arg Tyr Gly Gly Glu Trp Thr Glu Glu Ala Gly
465                 470                 475                 480

Glu Arg Ala Ala Leu Ser Glu Arg Leu Phe Ala Asn Leu Trp Gly Arg
                485                 490                 495

Leu Glu Gly Glu Glu Arg Leu Leu Trp Leu Tyr Arg Glu Val Glu Arg
            500                 505                 510

Pro Leu Ser Ala Val Leu Ala His Met Glu Ala Thr Gly Val Arg Leu
        515                 520                 525

Asp Val Ala Tyr Leu Arg Ala Leu Ser Leu Glu Val Ala Glu Glu Ile
    530                 535                 540

Ala Arg Leu Glu Ala Glu Val Phe Arg Leu Ala Gly His Pro Phe Asn
545                 550                 555                 560

Leu Asn Ser Arg Asp Gln Leu Glu Arg Val Leu Phe Asp Glu Leu Gly
                565                 570                 575

Leu Pro Ala Ile Gly Lys Thr Glu Lys Thr Gly Lys Arg Ser Thr Ser
            580                 585                 590

Ala Ala Val Leu Glu Ala Leu Arg Glu Ala His Pro Ile Val Glu Lys
        595                 600                 605

Ile Leu Gln Tyr Arg Glu Leu Thr Lys Leu Lys Ser Thr Tyr Ile Asp
    610                 615                 620

Pro Leu Pro Asp Leu Ile His Pro Arg Thr Gly Arg Leu His Thr Arg
625                 630                 635                 640

Phe Asn Gln Thr Ala Thr Ala Thr Gly Arg Leu Ser Ser Ser Asp Pro
                645                 650                 655

Asn Leu Gln Asn Ile Pro Val Arg Thr Pro Leu Gly Gln Arg Ile Arg
            660                 665                 670

Arg Ala Phe Ile Ala Glu Glu Gly Trp Leu Leu Val Ala Leu Asp Tyr
        675                 680                 685

Ser Gln Ile Glu Leu Arg Val Leu Ala His Leu Ser Gly Asp Glu Asn
    690                 695                 700

Leu Ile Arg Val Phe Gln Glu Gly Arg Asp Ile His Thr Glu Thr Ala
705                 710                 715                 720

Ser Trp Met Phe Gly Val Pro Arg Glu Ala Val Asp Pro Leu Met Arg
                725                 730                 735

Arg Ala Ala Lys Thr Ile Asn Phe Gly Val Leu Tyr Gly Met Ser Ala
            740                 745                 750

His Arg Leu Ser Gln Glu Leu Ala Ile Pro Tyr Glu Glu Ala Gln Ala
        755                 760                 765

Phe Ile Glu Arg Tyr Phe Gln Ser Phe Pro Lys Val Arg Ala Trp Ile
    770                 775                 780

Glu Lys Thr Leu Glu Glu Gly Arg Arg Arg Gly Tyr Val Glu Thr Leu
785                 790                 795                 800

Phe Gly Arg Arg Arg Tyr Val Pro Asp Leu Glu Ala Arg Val Lys Ser
                805                 810                 815

Val Arg Glu Ala Ala Glu Arg Met Ala Phe Asn Met Pro Val Gln Gly
            820                 825                 830

Thr Ala Ala Asp Leu Met Lys Leu Ala Met Val Lys Leu Phe Pro Arg
        835                 840                 845

Leu Glu Glu Met Gly Ala Arg Met Leu Leu Gln Val His Asp Glu Leu
    850                 855                 860

Val Leu Glu Ala Pro Lys Glu Arg Ala Glu Ala Val Ala Arg Leu Ala
865                 870                 875                 880

Lys Glu Val Met Glu Gly Val Tyr Pro Leu Ala Val Pro Leu Glu Val
                885                 890                 895

Glu Val Gly Ile Gly Glu Asp Trp Leu Ser Ala Lys Glu
            900                 905

<210>  23
<211>  2730
<212>  DNA
<213>  Artificial Sequence

<220>
<223>  DNA polymerase variant Taq96

<400>  23
atgagcggca aacaggctac actctttgac tttctgaaaa gcggcagcgg ttccggctct      60

ggtagcggct ctggttccgg taaacaagcc acccttttcg attttctgaa aagcggttct      120

ggttctggca gcggtagcgg ctccggcagc ggcaagcagg cgacgctgtt cgacttcctc      180

aagaagggct ctggtagcgg tagcggttct ggctccggta gcggctccgg catgcgtggt      240

atgctgccgc tgttcgaacc caagggccgg gtcctcctgg tggacggcca ccacctggcc      300

taccgcacct tccacgccct gaagggcctc accaccagcc ggggggagcc ggtgcaggcg      360

gtctacggct tcgccaagag cctcctcaag gccctcaagg aggacgggga cgcggtgatc      420

gtggtctttg acgccaaggc cccctccttc cgccacgagg cctacggggg gtacaaggcg      480

ggccgggccc ccacgccgga ggactttccc cggcaactcg ccctcatcaa ggagctggtg 540
gacctcctgg ggctggcgcg cctcgaggtc ccgggctacg aggcggacga cgtcctggcc 600
agcctggcca agaaggcgga aaaggagggc tacgaggtcc gcatcctcac cgccgacaaa 660
gacctttacc agctcctttc cgaccgcatc cacgccctcc accccgaggg gtacctcatc 720
accccggcct ggctttggga aaagtacggc ctgaggcccg accagtgggc cgactaccgg 780
gccctgaccg gggacgagtc cgacaacctt cccggggtca agggcatcgg ggagaagacg 840
gcgaggaagc ttctggagga gtgggggagc ctggaagccc tcctcaagaa cctggaccgg 900
ctgaagcccg ccatccggga gaagatcctg gcccacatgg acgatctgaa gctctcctgg 960
gacctggcca aggtgcgcac cgacctgccc ctggaggtgg acttcgccaa aaggcgggag 1020
cccgaccggg agaggcttag ggcctttctg gagaggcttg agtttggcag cctcctccac 1080
gagttcggcc ttctggaaag ccccaaggcc ctggaggagg ccccctggcc cccgccggaa 1140
ggggccttcg tgggctttgt gctttcccgc aaggagccca tgtgggccga tcttctggcc 1200
ctggccgccg ccaggggggg ccgggtccac cgggcccccg agccttataa agccctcagg 1260
gacctgaagg aggcgcgggg gcttctcgcc aaagacctga gcgttctggc cctgagggaa 1320
ggccttggcc tcccgcccgg cgacgacccc atgctcctcg cctacctcct ggacccttcc 1380
aacaccaccc ccgagggggt ggcccggcgc tacggcgggg agtggacgga ggaggcgggg 1440
gagcgggccg ccctttccga gaggctcttc gccaacctgt gggggaggct tgagggggag 1500
gagaggctcc tttggcttta ccgggaggtg gagaggcccc tttccgctgt cctggcccac 1560
atggaggcca cgggggtgcg cctggacgtg gcctatctca gggccttgtc cctggaggtg 1620
gccgaggaga tcgcccgcct cgaggccgag gtcttccgcc tggccggcca ccccttcaac 1680
ctcaactccc gggaccagct ggaaagggtc ctctttgacg agctagggct tcccgccatc 1740
ggcaagacgg agaagaccgg caagcgctcc accagcgccg ccgtcctgga ggccctccgc 1800
gaggcccacc ccatcgtgga gaagatcctg cagtaccggg agctcaccaa gctgaagagc 1860
acctacattg accccttgcc ggacctcatc caccccagga cgggccgcct ccacacccgc 1920
ttcaaccaga cggccacggc cacgggcagg ctaagtagct ccgatcccaa cctccagaac 1980
atccccgtcc gcaccccgct tgggcagagg atccgccggg ccttcatcgc ccgaggaggg 2040
tggctattgg tggccctgga ctatagccag atagagctca gggtgctggc ccacctctcc 2100
ggcgacgaga acctgatccg ggtcttccag gaggggcggg acatccacac ggagaccgcc 2160
agctggatgt tcggcgtccc ccgggaggcc gtggaccccc tgatgcgccg ggcggccaag 2220
accatcaact tcggggtcct ctacggcatg tcggcccacc gcctctccca ggagctagcc 2280
atcccttacg aggaggccca ggccttcatt gagcgctact ttcagagctt ccccaaggtg 2340
cgggcctgga ttgagaagac cctggaggag ggcaggaggc gggggtacgt ggagaccctc 2400

ttcggccgcc gccgctacgt gccagaccta gaggcccggg tgaagagcgt gcgggaggcg 2460

gccgagcgca tggccttcaa catgcccgtc cagggcaccg ccgccgacct catgaagctg 2520

gctatggtga agctcttccc caggctggag gaaatggggg ccaggatgct ccttcaggtc 2580

cacgacgagc tggtcctcga ggccccaaaa gagagggcgg aggccgtggc ccggctggcc 2640

aaggaggtca tggagggggt gtatcccctg gccgtgcccc tggaggtgga ggtggggata 2700

ggggaggact ggctctccgc caaggagtga 2730

<210> 24
<211> 933
<212> PRT
<213> Artificial sequence

<220>
<223> DNA polymerase variant Taq97

<400> 24

Met Ser Gly Lys Gln Ala Thr Leu Phe Asp Phe Leu Lys Ser Gly Ser
1               5                   10                  15

Gly Ser Gly Ser Gly Ser Gly Ser Gly Ser Gly Lys Gln Ala Thr Leu
            20                  25                  30

Phe Asp Phe Leu Lys Ser Gly Ser Gly Ser Gly Ser Gly Ser Gly Ser
        35                  40                  45

Gly Ser Gly Lys Gln Ala Thr Leu Phe Asp Phe Leu Lys Ser Gly Ser
    50                  55                  60

Gly Ser Gly Ser Gly Ser Gly Ser Gly Ser Gly Lys Gln Ala Thr Leu
65                  70                  75                  80

Phe Asp Phe Leu Lys Lys Gly Ser Gly Ser Gly Ser Gly Ser Gly Ser
                85                  90                  95

Gly Ser Gly Ser Gly Met Arg Gly Met Leu Pro Leu Phe Glu Pro Lys
            100                 105                 110

Gly Arg Val Leu Leu Val Asp Gly His His Leu Ala Tyr Arg Thr Phe
        115                 120                 125

His Ala Leu Lys Gly Leu Thr Thr Ser Arg Gly Glu Pro Val Gln Ala
    130                 135                 140

Val Tyr Gly Phe Ala Lys Ser Leu Leu Lys Ala Leu Lys Glu Asp Gly
145                 150                 155                 160

Asp Ala Val Ile Val Val Phe Asp Ala Lys Ala Pro Ser Phe Arg His
                165                 170                 175

Glu Ala Tyr Gly Gly Tyr Lys Ala Gly Arg Ala Pro Thr Pro Glu Asp
            180                 185                 190

Phe Pro Arg Gln Leu Ala Leu Ile Lys Glu Leu Val Asp Leu Leu Gly
        195                 200                 205

Leu Ala Arg Leu Glu Val Pro Gly Tyr Glu Ala Asp Asp Val Leu Ala
    210                 215                 220

Ser Leu Ala Lys Lys Ala Glu Lys Glu Gly Tyr Glu Val Arg Ile Leu
225                 230                 235                 240

Thr Ala Asp Lys Asp Leu Tyr Gln Leu Leu Ser Asp Arg Ile His Ala
                245                 250                 255

Leu His Pro Glu Gly Tyr Leu Ile Thr Pro Ala Trp Leu Trp Glu Lys
            260                 265                 270

Tyr Gly Leu Arg Pro Asp Gln Trp Ala Asp Tyr Arg Ala Leu Thr Gly
        275                 280                 285

Asp Glu Ser Asp Asn Leu Pro Gly Val Lys Gly Ile Gly Glu Lys Thr
    290                 295                 300

Ala Arg Lys Leu Leu Glu Glu Trp Gly Ser Leu Glu Ala Leu Leu Lys
305                 310                 315                 320

Asn Leu Asp Arg Leu Lys Pro Ala Ile Arg Glu Lys Ile Leu Ala His
                325                 330                 335

Met Asp Asp Leu Lys Leu Ser Trp Asp Leu Ala Lys Val Arg Thr Asp
            340                 345                 350

Leu Pro Leu Glu Val Asp Phe Ala Lys Arg Arg Glu Pro Asp Arg Glu
        355                 360                 365

Arg Leu Arg Ala Phe Leu Glu Arg Leu Glu Phe Gly Ser Leu Leu His
    370                 375                 380

Glu Phe Gly Leu Leu Glu Ser Pro Lys Ala Leu Glu Glu Ala Pro Trp
385                 390                 395                 400

Pro Pro Pro Glu Gly Ala Phe Val Gly Phe Val Leu Ser Arg Lys Glu
                405                 410                 415

Pro Met Trp Ala Asp Leu Leu Ala Leu Ala Ala Ala Arg Gly Gly Arg
        420                 425                 430

Val His Arg Ala Pro Glu Pro Tyr Lys Ala Leu Arg Asp Leu Lys Glu
    435                 440                 445

Ala Arg Gly Leu Leu Ala Lys Asp Leu Ser Val Leu Ala Leu Arg Glu
    450                 455                 460

Gly Leu Gly Leu Pro Pro Gly Asp Asp Pro Met Leu Leu Ala Tyr Leu
465                 470                 475                 480

Leu Asp Pro Ser Asn Thr Thr Pro Glu Gly Val Ala Arg Arg Tyr Gly
                485                 490                 495

Gly Glu Trp Thr Glu Glu Ala Gly Glu Arg Ala Ala Leu Ser Glu Arg
        500                 505                 510

Leu Phe Ala Asn Leu Trp Gly Arg Leu Glu Gly Glu Glu Arg Leu Leu
    515                 520                 525

Trp Leu Tyr Arg Glu Val Glu Arg Pro Leu Ser Ala Val Leu Ala His
    530                 535                 540

Met Glu Ala Thr Gly Val Arg Leu Asp Val Ala Tyr Leu Arg Ala Leu
545                 550                 555                 560

Ser Leu Glu Val Ala Glu Glu Ile Ala Arg Leu Glu Ala Glu Val Phe
                565                 570                 575

Arg Leu Ala Gly His Pro Phe Asn Leu Asn Ser Arg Asp Gln Leu Glu
        580                 585                 590

Arg Val Leu Phe Asp Glu Leu Gly Leu Pro Ala Ile Gly Lys Thr Glu
    595                 600                 605

Lys Thr Gly Lys Arg Ser Thr Ser Ala Ala Val Leu Glu Ala Leu Arg
    610                 615                 620

Glu Ala His Pro Ile Val Glu Lys Ile Leu Gln Tyr Arg Glu Leu Thr
625                 630                 635                 640

Lys Leu Lys Ser Thr Tyr Ile Asp Pro Leu Pro Asp Leu Ile His Pro
                645                 650                 655

Arg Thr Gly Arg Leu His Thr Arg Phe Asn Gln Thr Ala Thr Ala Thr

            660                 665                 670

Gly Arg Leu Ser Ser Ser Asp Pro Asn Leu Gln Asn Ile Pro Val Arg
        675                 680                 685

Thr Pro Leu Gly Gln Arg Ile Arg Arg Ala Phe Ile Ala Glu Glu Gly
    690                 695                 700

Trp Leu Leu Val Ala Leu Asp Tyr Ser Gln Ile Glu Leu Arg Val Leu
705                 710                 715                 720

Ala His Leu Ser Gly Asp Glu Asn Leu Ile Arg Val Phe Gln Glu Gly
                725                 730                 735

Arg Asp Ile His Thr Glu Thr Ala Ser Trp Met Phe Gly Val Pro Arg
            740                 745                 750

Glu Ala Val Asp Pro Leu Met Arg Arg Ala Ala Lys Thr Ile Asn Phe
        755                 760                 765

Gly Val Leu Tyr Gly Met Ser Ala His Arg Leu Ser Gln Glu Leu Ala
    770                 775                 780

Ile Pro Tyr Glu Glu Ala Gln Ala Phe Ile Glu Arg Tyr Phe Gln Ser
785                 790                 795                 800

Phe Pro Lys Val Arg Ala Trp Ile Glu Lys Thr Leu Glu Glu Gly Arg
                805                 810                 815

Arg Arg Gly Tyr Val Glu Thr Leu Phe Gly Arg Arg Arg Tyr Val Pro
            820                 825                 830

Asp Leu Glu Ala Arg Val Lys Ser Val Arg Glu Ala Ala Glu Arg Met
        835                 840                 845

Ala Phe Asn Met Pro Val Gln Gly Thr Ala Ala Asp Leu Met Lys Leu
    850                 855                 860

Ala Met Val Lys Leu Phe Pro Arg Leu Glu Glu Met Gly Ala Arg Met
865                 870                 875                 880

Leu Leu Gln Val His Asp Glu Leu Val Leu Glu Ala Pro Lys Glu Arg
                885                 890                 895

Ala Glu Ala Val Ala Arg Leu Ala Lys Glu Val Met Glu Gly Val Tyr
            900                 905                 910

Pro Leu Ala Val Pro Leu Glu Val Glu Val Gly Ile Gly Glu Asp Trp
        915                 920                 925

Leu Ser Ala Lys Glu
    930

<210>  25
<211>  2802
<212>  DNA
<213>  Artificial sequence

<220>
<223>  DNA polymerase variant Taq97

<400>  25
   atgagcggta aacaagcaac tcttttcgat ttccttaagt ctgggagtgg gagcggttcc      60
   ggcagcggtt cgggcagcgg caaacaggct acactctttg actttctgaa aagcggcagc     120
   ggttccggct ctggtagcgg ctctggttcc ggtaaacaag ccaccctttt cgattttctg     180
   aaaagcggtt ctggttctgg cagcggtagc ggctccggca gcggcaagca ggcgacgctg     240
   ttcgacttcc tcaagaaggg ctctggtagc ggtagcggtt ctggctccgg tagcggctcc     300
   ggcatgcgtg gtatgctgcc gctgttcgaa cccaagggcc gggtcctcct ggtggacggc     360
   caccacctgg cctaccgcac cttccacgcc ctgaagggcc tcaccaccag ccggggggag     420
   ccggtgcagg cggtctacgg cttcgccaag agcctcctca aggccctcaa ggaggacggg     480
   gacgcggtga tcgtggtctt tgacgccaag gccccctcct tccgccacga ggcctacggg     540
   gggtacaagg cgggccgggc ccccacgccg gaggactttc cccggcaact cgccctcatc     600
   aaggagctgg tggacctcct ggggctggcg cgcctcgagg tcccgggcta cgaggcggac     660
   gacgtcctgg ccagcctggc caagaaggcg gaaaaggagg gctacgaggt ccgcatcctc     720
   accgccgaca aagaccttta ccagctcctt tccgaccgca tccacgccct ccaccccgag      780
   gggtacctca tcaccccggc ctggctttgg gaaaagtacg gcctgaggcc cgaccagtgg     840
   gccgactacc gggccctgac cggggacgag tccgacaacc ttcccggggt caagggcatc     900
   ggggagaaga cggcgaggaa gcttctggag gagtggggga gcctggaagc cctcctcaag     960
   aacctggacc ggctgaagcc cgccatccgg gagaagatcc tggcccacat ggacgatctg    1020
   aagctctcct gggacctggc caaggtgcgc accgacctgc ccctggaggt ggacttcgcc    1080
   aaaaggcggg agcccgaccg ggagaggctt agggcctttc tggagaggct tgagtttggc    1140
   agcctcctcc acgagttcgg ccttctggaa agccccaagg ccctggagga ggccccctgg    1200
   cccccgccgg aaggggcctt cgtgggcttt gtgctttccc gcaaggagcc catgtgggcc    1260
   gatcttctgg ccctggccgc cgccaggggg ggccgggtcc accgggcccc cgagccttat    1320
   aaagccctca gggacctgaa ggaggcgcgg gggcttctcg ccaaagacct gagcgttctg    1380
   gccctgaggg aaggccttgg cctcccgccc ggcgacgacc ccatgctcct cgcctacctc 1440
   ctggaccctt ccaacaccac ccccgagggg gtggcccggc gctacggcgg ggagtggacg 1500
   gaggaggcgg gggagcgggc cgccctttcc gagaggctct tcgccaacct gtgggggagg 1560
   cttgaggggg aggagaggct cctttggctt taccgggagg tggagaggcc cctttccgct 1620
   gtcctggccc acatggaggc cacgggggtg cgcctggacg tggcctatct cagggccttg 1680
   tccctggagg tggccgagga gatcgcccgc ctcgaggccg aggtcttccg cctggccggc 1740
   caccccttca acctcaactc ccgggaccag ctggaaaggg tcctctttga cgagctaggg 1800
   cttcccgcca tcggcaagac ggagaagacc ggcaagcgct ccaccagcgc cgccgtcctg 1860
   gaggccctcc gcgaggccca ccccatcgtg gagaagatcc tgcagtaccg ggagctcacc 1920
   aagctgaaga gcacctacat tgaccccttg ccggacctca tccaccccag gacgggccgc 1980
   ctccacaccc gcttcaacca gacggccacg gccacgggca ggctaagtag ctccgatccc 2040
   aacctccaga acatccccgt ccgcaccccg cttgggcaga ggatccgccg ggccttcatc 2100
   gccgaggagg ggtggctatt ggtggccctg gactatagcc agatagagct cagggtgctg 2160
   gcccacctct ccggcgacga gaacctgatc cgggtcttcc aggaggggcg ggacatccac 2220
   acggagaccg ccagctggat gttcggcgtc ccccgggagg ccgtggaccc cctgatgcgc 2280
   cgggcggcca agaccatcaa cttcggggtc ctctacggca tgtcggccca ccgcctctcc 2340
   caggagctag ccatccctta cgaggaggcc caggccttca ttgagcgcta ctttcagagc 2400
   ttccccaagg tgcgggcctg gattgagaag accctggagg agggcaggag gcgggggtac 2460
   gtggagaccc tcttcggccg ccgccgctac gtgccagacc tagaggcccg ggtgaagagc 2520
   gtgcgggagg cggccgagcg catggccttc aacatgcccg tccagggcac cgccgccgac 2580
   ctcatgaagc tggctatggt gaagctcttc cccaggctgg aggaaatggg ggccaggatg 2640
   ctccttcagg tccacgacga gctggtcctc gaggccccaa aagagagggc ggaggccgtg 2700
   gcccggctgg ccaaggaggt catggagggg gtgtatcccc tggccgtgcc cctggaggtg 2760
   gaggtgggga taggggagga ctggctctcc gccaaggagt ga 2802

<210> 26
<211> 957
<212> PRT
<213> Artificial sequence

<220>
<223> DNA polymerase variant Taq98

<400> 26

Met Ser Ala Lys Gln Ala Thr Leu Phe Asp Phe Leu Lys Ser Gly Ser
1               5                   10                  15

Gly Ser Gly Ser Gly Ser Gly Ser Gly Ser Gly Lys Gln Ala Thr Leu
                20                  25                  30

Phe Asp Phe Leu Lys Ser Gly Ser Gly Ser Gly Ser Gly Ser Gly Ser
            35                  40                  45

Gly Ser Gly Lys Gln Ala Thr Leu Phe Asp Phe Leu Lys Ser Gly Ser
    50                  55                  60

Gly Ser Gly Ser Gly Ser Gly Ser Gly Ser Gly Lys Gln Ala Thr Leu
65                  70                  75                  80

Phe Asp Phe Leu Lys Ser Gly Ser Gly Ser Gly Ser Gly Ser Gly Ser
                85                  90                  95

Gly Ser Gly Lys Gln Ala Thr Leu Phe Asp Phe Leu Lys Lys Gly Ser
            100                 105                 110

Gly Ser Gly Ser Gly Ser Gly Ser Gly Ser Gly Ser Gly Met Arg Gly
        115                 120                 125

Met Leu Pro Leu Phe Glu Pro Lys Gly Arg Val Leu Leu Val Asp Gly
    130                 135                 140

His His Leu Ala Tyr Arg Thr Phe His Ala Leu Lys Gly Leu Thr Thr
145                 150                 155                 160

Ser Arg Gly Glu Pro Val Gln Ala Val Tyr Gly Phe Ala Lys Ser Leu
                165                 170                 175

Leu Lys Ala Leu Lys Glu Asp Gly Asp Ala Val Ile Val Val Phe Asp
            180                 185                 190

Ala Lys Ala Pro Ser Phe Arg His Glu Ala Tyr Gly Gly Tyr Lys Ala
        195                 200                 205

Gly Arg Ala Pro Thr Pro Glu Asp Phe Pro Arg Gln Leu Ala Leu Ile
    210                 215                 220

Lys Glu Leu Val Asp Leu Leu Gly Leu Ala Arg Leu Glu Val Pro Gly
225                 230                 235                 240

Tyr Glu Ala Asp Asp Val Leu Ala Ser Leu Ala Lys Lys Ala Glu Lys
                245                 250                 255

Glu Gly Tyr Glu Val Arg Ile Leu Thr Ala Asp Lys Asp Leu Tyr Gln
            260                 265                 270

Leu Leu Ser Asp Arg Ile His Ala Leu His Pro Glu Gly Tyr Leu Ile
        275                 280                 285

Thr Pro Ala Trp Leu Trp Glu Lys Tyr Gly Leu Arg Pro Asp Gln Trp
    290                 295                 300

Ala Asp Tyr Arg Ala Leu Thr Gly Asp Glu Ser Asp Asn Leu Pro Gly
305                 310                 315                 320

Val Lys Gly Ile Gly Glu Lys Thr Ala Arg Lys Leu Leu Glu Glu Trp
                325                 330                 335

Gly Ser Leu Glu Ala Leu Leu Lys Asn Leu Asp Arg Leu Lys Pro Ala
            340                 345                 350

Ile Arg Glu Lys Ile Leu Ala His Met Asp Asp Leu Lys Leu Ser Trp
        355                 360                 365

Asp Leu Ala Lys Val Arg Thr Asp Leu Pro Leu Glu Val Asp Phe Ala
    370                 375                 380

Lys Arg Arg Glu Pro Asp Arg Glu Arg Leu Arg Ala Phe Leu Glu Arg
385                 390                 395                 400

Leu Glu Phe Gly Ser Leu Leu His Glu Phe Gly Leu Leu Glu Ser Pro
                405                 410                 415

Lys Ala Leu Glu Glu Ala Pro Trp Pro Pro Pro Glu Gly Ala Phe Val
            420                 425                 430

Gly Phe Val Leu Ser Arg Lys Glu Pro Met Trp Ala Asp Leu Leu Ala
        435                 440                 445

Leu Ala Ala Ala Arg Gly Gly Arg Val His Arg Ala Pro Glu Pro Tyr
    450                 455                 460

Lys Ala Leu Arg Asp Leu Lys Glu Ala Arg Gly Leu Leu Ala Lys Asp
465                 470                 475                 480

Leu Ser Val Leu Ala Leu Arg Glu Gly Leu Gly Leu Pro Pro Gly Asp
                485                 490                 495

Asp Pro Met Leu Leu Ala Tyr Leu Leu Asp Pro Ser Asn Thr Thr Pro
            500                 505                 510

Glu Gly Val Ala Arg Arg Tyr Gly Gly Glu Trp Thr Glu Glu Ala Gly
        515                 520                 525

Glu Arg Ala Ala Leu Ser Glu Arg Leu Phe Ala Asn Leu Trp Gly Arg
    530                 535                 540

Leu Glu Gly Glu Glu Arg Leu Leu Trp Leu Tyr Arg Glu Val Glu Arg
545                 550                 555                 560

Pro Leu Ser Ala Val Leu Ala His Met Glu Ala Thr Gly Val Arg Leu
                565                 570                 575

Asp Val Ala Tyr Leu Arg Ala Leu Ser Leu Glu Val Ala Glu Glu Ile
            580                 585                 590

Ala Arg Leu Glu Ala Glu Val Phe Arg Leu Ala Gly His Pro Phe Asn
        595                 600                 605

Leu Asn Ser Arg Asp Gln Leu Glu Arg Val Leu Phe Asp Glu Leu Gly
    610                 615                 620

Leu Pro Ala Ile Gly Lys Thr Glu Lys Thr Gly Lys Arg Ser Thr Ser
625                 630                 635                 640

Ala Ala Val Leu Glu Ala Leu Arg Glu Ala His Pro Ile Val Glu Lys
                645                 650                 655

Ile Leu Gln Tyr Arg Glu Leu Thr Lys Leu Lys Ser Thr Tyr Ile Asp
            660                 665                 670

Pro Leu Pro Asp Leu Ile His Pro Arg Thr Gly Arg Leu His Thr Arg
        675                 680                 685

Phe Asn Gln Thr Ala Thr Ala Thr Gly Arg Leu Ser Ser Ser Asp Pro
    690                 695                 700

Asn Leu Gln Asn Ile Pro Val Arg Thr Pro Leu Gly Gln Arg Ile Arg
705                 710                 715                 720

Arg Ala Phe Ile Ala Glu Glu Gly Trp Leu Leu Val Ala Leu Asp Tyr
                725                 730                 735

Ser Gln Ile Glu Leu Arg Val Leu Ala His Leu Ser Gly Asp Glu Asn
            740                 745                 750

Leu Ile Arg Val Phe Gln Glu Gly Arg Asp Ile His Thr Glu Thr Ala
        755                 760                 765

Ser Trp Met Phe Gly Val Pro Arg Glu Ala Val Asp Pro Leu Met Arg

770                 775                 780

Arg Ala Ala Lys Thr Ile Asn Phe Gly Val Leu Tyr Gly Met Ser Ala
785                 790                 795                 800

His Arg Leu Ser Gln Glu Leu Ala Ile Pro Tyr Glu Glu Ala Gln Ala
                805                 810                 815

Phe Ile Glu Arg Tyr Phe Gln Ser Phe Pro Lys Val Arg Ala Trp Ile
            820                 825                 830

Glu Lys Thr Leu Glu Glu Gly Arg Arg Arg Gly Tyr Val Glu Thr Leu
        835                 840                 845

Phe Gly Arg Arg Arg Tyr Val Pro Asp Leu Glu Ala Arg Val Lys Ser
    850                 855                 860

Val Arg Glu Ala Ala Glu Arg Met Ala Phe Asn Met Pro Val Gln Gly
865                 870                 875                 880

Thr Ala Ala Asp Leu Met Lys Leu Ala Met Val Lys Leu Phe Pro Arg
                885                 890                 895

Leu Glu Glu Met Gly Ala Arg Met Leu Leu Gln Val His Asp Glu Leu
            900                 905                 910

Val Leu Glu Ala Pro Lys Glu Arg Ala Glu Ala Val Ala Arg Leu Ala
        915                 920                 925

Lys Glu Val Met Glu Gly Val Tyr Pro Leu Ala Val Pro Leu Glu Val
    930                 935                 940

Glu Val Gly Ile Gly Glu Asp Trp Leu Ser Ala Lys Glu
945                 950                 955

<210>  27
<211>  2874
<212>  DNA
<213>  Artificial sequence

<220>
<223>  DNA polymerase variant Taq98

<400>  27
atgtccgcta agcaagccac gctgttcgac tttctcaaat ccggctcggg cagtgggagc      60

ggctctggca gcgggagcgg taaacaagca actcttttcg atttccttaa gtctgggagt     120

gggagcggtt ccggcagcgg ttcgggcagc ggcaaacagg ctacactctt tgactttctg     180

aaaagcggca gcggttccgg ctctggtagc ggctctggtt ccggtaaaca agccaccctt     240

ttcgattttc tgaaaagcgg ttctggttct ggcagcggta gcggctccgg cagcggcaag 300

caggcgacgc tgttcgactt cctcaagaag ggctctggta gcggtagcgg ttctggctcc 360

ggtagcggct ccggcatgcg tggtatgctg ccgctgttcg aacccaaggg ccgggtcctc 420

ctggtggacg gccaccacct ggcctaccgc accttccacg ccctgaaggg cctcaccacc 480

agccgggggg agccggtgca ggcggtctac ggcttcgcca agagcctcct caaggccctc 540

aaggaggacg gggacgcggt gatcgtggtc tttgacgcca aggccccctc cttccgccac 600

gaggcctacg gggggtacaa ggcgggccgg gcccccacgc cggaggactt tccccggcaa 660

ctcgccctca tcaaggagct ggtggacctc ctggggctgg cgcgcctcga ggtcccgggc 720

tacgaggcgg acgacgtcct ggccagcctg gccaagaagg cggaaaagga gggctacgag 780

gtccgcatcc tcaccgccga caaagacctt taccagctcc tttccgaccg catccacgcc 840

ctccaccccg aggggtacct catcaccccg gcctggcttt gggaaaagta cggcctgagg 900

cccgaccagt gggccgacta ccgggccctg accggggacg agtccgacaa ccttcccggg 960

gtcaagggca tcggggagaa gacggcgagg aagcttctgg aggagtgggg gagcctggaa 1020

gccctcctca agaacctgga ccggctgaag cccgccatcc gggagaagat cctggcccac 1080

atggacgatc tgaagctctc ctgggacctg gccaaggtgc gcaccgacct gcccctggag 1140

gtggacttcg ccaaaaggcg ggagcccgac cgggagaggc ttagggcctt tctggagagg 1200

cttgagtttg gcagcctcct ccacgagttc ggccttctgg aaagccccaa ggccctggag 1260

gaggccccct ggcccccgcc ggaaggggcc ttcgtgggct ttgtgctttc ccgcaaggag 1320

cccatgtggg ccgatcttct ggccctggcc gccgccaggg ggggccgggt ccaccgggcc 1380

cccgagcctt ataaagccct cagggacctg aaggaggcgc gggggcttct cgccaaagac 1440

ctgagcgttc tggccctgag ggaaggcctt ggcctcccgc ccggcgacga ccccatgctc 1500

ctcgcctacc tcctggaccc ttccaacacc acccccgagg gggtggcccg gcgctacggc 1560

ggggagtgga cggaggaggc gggggagcgg gccgcccttt ccgagaggct cttcgccaac 1620

ctgtggggga ggcttgaggg ggaggagagg ctcctttggc tttaccggga ggtggagagg 1680

cccctttccg ctgtcctggc ccacatggag gccacggggg tgcgcctgga cgtggcctat 1740

ctcagggcct tgtccctgga ggtggccgag gagatcgccc gcctcgaggc cgaggtcttc 1800

cgcctggccg gccacccctt caacctcaac tcccgggacc agctggaaag ggtcctcttt 1860

gacgagctag ggcttcccgc catcggcaag acggagaaga ccggcaagcg ctccaccagc 1920

gccgccgtcc tggaggccct ccgcgaggcc caccccatcg tggagaagat cctgcagtac 1980

cgggagctca ccaagctgaa gagcacctac attgacccct tgccggacct catccacccc 2040

aggacgggcc gcctccacac ccgcttcaac cagacggcca cggccacggg caggctaagt 2100

agctccgatc ccaacctcca gaacatcccc gtccgcaccc cgcttgggca gaggatccgc 2160

cgggccttca tcgccgagga ggggtggcta ttggtggccc tggactatag ccagatagag 2220

ctcagggtgc tggcccacct ctccggcgac gagaacctga tccgggtctt ccaggagggg 2280

cgggacatcc acacggagac cgccagctgg atgttcggcg tcccccggga ggccgtggac 2340

cccctgatgc gccgggcggc caagaccatc aacttcgggg tcctctacgg catgtcggcc 2400

caccgcctct cccaggagct agccatccct tacgaggagg cccaggcctt cattgagcgc 2460

tactttcaga gcttccccaa ggtgcgggcc tggattgaga agaccctgga ggagggcagg 2520

aggcgggggt acgtggagac cctcttcggc cgccgccgct acgtgccaga cctagaggcc 2580

cgggtgaaga gcgtgcggga ggcggccgag cgcatggcct tcaacatgcc cgtccagggc 2640

accgccgccg acctcatgaa gctggctatg gtgaagctct tccccaggct ggaggaaatg 2700

ggggccagga tgctccttca ggtccacgac gagctggtcc tcgaggcccc aaaagagagg 2760

gcggaggccg tggcccggct ggccaaggag gtcatggagg gggtgtatcc cctggccgtg 2820

cccctggagg tggaggtggg gataggggag gactggctct ccgccaagga gtga 2874

<210> 28
<211> 85
<212> DNA
<213> Artificial Sequence

<220>
<223> TaqNPIP-5 primer

<400> 28
cgcgcatatg agcggcaagc aggcgacgct gttcgacttc ctcaagaagg gtagcggctc 60

cggcatgcgt ggtatgctgc cgctg 85

<210> 29
<211> 37
<212> DNA
<213> Artificial Sequence

<220>
<223> Taq-3 primer

<400> 29
gcgcgcggcc gctcactcct tggcggagag ccagtcc 37

<210> 30
<211> 52
<212> DNA
<213> Artificial Sequence

<220>
<223> taqN10-5 primer

<400> 30
gctgttcgac ttcctcaaga agagcggttc tggctccggt agcggctccg gc 52

<210>  31
<211>  52
<212>  DNA
<213>  Artificial Sequence

<220>
<223>  taqN10-3 primer

<400>  31
gccggagccg ctaccggagc cagaaccgct cttcttgagg aagtcgaaca gc          52


<210>  32
<211>  67
<212>  DNA
<213>  Artificial Sequence

<220>
<223>  taqN15-5 primer

<400>  32
gctgttcgac ttcctcaaga agggctctgg tagcggtagc ggttctggct ccggtagcgg      60

ctccggc                                                                67


<210>  33
<211>  67
<212>  DNA
<213>  Artificial Sequence

<220>
<223>  taqN15-3 primer

<400>  33
gccggagccg ctaccggagc cagaaccgct accgctacca gagcccttct tgaggaagtc      60

gaacagc                                                                67


<210>  34
<211>  100
<212>  DNA
<213>  Artificial Sequence

<220>
<223>  taq-plus20-5 primer

<400>  34
gctgttcgac ttcctcaaga agggttctgg ttccggctcc ggcagcggct ctggctccgg      60

ctccggtagc ggttccggta gcggctctgg tagcggtagc                           100


<210>  35
<211>  100
<212>  DNA
<213>  Artificial Sequence

<220>
<223>  taq-plus20-3 primer

<400>  35
gctaccgcta ccagagccgc taccggaacc gctaccggag ccggagccag agccgctgcc      60

ggagccggaa ccagaaccct tcttgaggaa gtcgaacagc                            100


<210>  36
<211>  72
<212>  DNA
<213>  Artificial Sequence

<220>
<223>  Taq-plus12-5 primer

<400>  36
ggctctggct ccggctccgg tagcggttcc ggtagcggct ctggtagcgg ctctggtagc      60

ggttccggta gc                                                          72


<210>  37
<211>  72
<212>  DNA
<213>  Artificial Sequence

<220>
<223>  Taq-plus12-3 primer

<400>  37
gctaccggaa ccgctaccag agccgctacc agagccgcta ccggaaccgc taccggagcc      60

ggagccagag cc                                                          72


<210>  38
<211>  28
<212>  DNA
<213>  Artificial Sequence

<220>
<223>  Taq-5 primer

<400>  38
gcgccccata tgcgtggtat gctgccgc                                         28


<210>  39
<211>  77
<212>  DNA
<213>  Artificial Sequence

<220>
<223>  Tq-L5-PIP-3 primer

<400>  39
ggggcggccg ctcacttctt gaggaagtcg aacagcgtcg cctgcttgga gccagaaccg      60

ctctccttgg cggagag                                                     77


<210>  40
<211>  92

<212>  DNA
<213>  Artificial Sequence

<220>
<223>  Tq-L10-PIP-3 primer

<400>  40
ggggcggccg ctcacttctt gaggaagtcg aacagcgtcg cctgcttgga accgctgcca      60

gaggagccag aaccgctctc cttggcggag ag                                    92


<210>  41
<211>  107
<212>  DNA
<213>  Artificial Sequence

<220>
<223>  Tq-L15-PIP-3 primer

<400>  41
ggggcggccg ctcacttctt gaggaagtcg aacagcgtcg cctgcttgcc ggagccgcta      60

ccggaaccgc tgccagagga gccagaaccg ctctccttgg cggagag                   107


<210>  42
<211>  35
<212>  DNA
<213>  Artificial Sequence

<220>
<223>  F1 primer

<400>  42
gagttcgtgt ccgtacaact ggcgtaatca tggcc                                 35


<210>  43
<211>  27
<212>  DNA
<213>  Artificial Sequence

<220>
<223>  R2-2 primer

<400>  43
cttttcagcc tggccctttc ctttacc                                          27


<210>  44
<211>  35
<212>  DNA
<213>  Artificial Sequence

<220>
<223>  LF-35 primer

<400>  44
tgctgaaatg aattctaagc ggagatcgcc tagtg                                 35


<210>  45

<211>  35
<212>  DNA
<213>  Artificial Sequence

<220>
<223>  LR8-35 primer

<400>  45
atgggcaata cgaacgacgg caatgattgc cagag                                35


<210>  46
<211>  35
<212>  DNA
<213>  Artificial Sequence

<220>
<223>  LR12-35 primer

<400>  46
atatacgccg agatctttag ctgtcttggt ttgcc                                35


<210>  47
<211>  35
<212>  DNA
<213>  Artificial Sequence

<220>
<223>  LR15-35 primer

<400>  47
ctcgctttcc actccagagc cagtctcgct tcgtc                                35


<210>  48
<211>  100
<212>  DNA
<213>  Artificial Sequence

<220>
<223>  Taq95-5 primer

<400>  48
gcgccatatg tccggtaaac aagccaccct tttcgatttt ctgaaaagcg gttctggttc      60

tggcagcggt agcggctccg gcagcggcaa gcaggcgacg                          100


<210>  49
<211>  100
<212>  DNA
<213>  Artificial Sequence

<220>
<223>  Taq96-5 primer

<400>  49
gcgccatatg agcggcaaac aggctacact ctttgacttt ctgaaaagcg gcagcggttc      60

cggctctggt agcggctctg gttccggtaa acaagccacc                          100

<210>  50
<211>  100
<212>  DNA
<213>  Artificial Sequence

<220>
<223>  Taq97-5 primer

<400>  50
gcgccatatg agcggtaaac aagcaactct tttcgatttc cttaagtctg ggagtgggag      60

cggttccggc agcggttcgg gcagcggcaa acaggctaca                          100


<210>  51
<211>  100
<212>  DNA
<213>  Artificial Sequence

<220>
<223>  Taq98-5 primer

<400>  51
gcgccatatg tccgctaagc aagccacgct gttcgacttt ctcaaatccg gctcgggcag      60

tgggagcggc tctggcagcg ggagcggtaa acaagcaact                          100


<210>  52
<211>  8
<212>  PRT
<213>  Pyrococcus furiosus

<400>  52

Gln Ala Thr Leu Phe Asp Phe Leu
1               5


<210>  53
<211>  8
<212>  PRT
<213>  Methanococcus jannaschii

<400>  53

Gln Leu Thr Leu Asp Ala Phe Phe
1               5


<210>  54
<211>  8
<212>  PRT
<213>  Pyrococcus furiosus

<400>  54

Gln Val Gly Leu Thr Ser Trp Leu
1               5


<210>  55
<211>  8

<212>  PRT
<213>  Thermococcus litoralis

<400>  55

Gln Thr Gly Leu Asp Ala Trp Leu
1               5


<210>  56
<211>  8
<212>  PRT
<213>  Archaeoglobus fulgidus

<400>  56

Gln Met Ser Leu Asp Ser Phe Phe
1               5


<210>  57
<211>  8
<212>  PRT
<213>  Pyrodictium occultum

<400>  57

Gln Arg Ser Leu Phe Asp Phe Phe
1               5


<210>  58
<211>  8
<212>  PRT
<213>  Methanococcus jannaschii

<400>  58

Gln Val Lys Leu Ser Asp Phe Phe
1               5


<210>  59
<211>  8
<212>  PRT
<213>  Methanobacterium thermoautotrophicum

<400>  59

Gln Ser Ser Leu Asp Val Phe Leu
1               5


<210>  60
<211>  8
<212>  PRT
<213>  Homo sapiens

<400>  60

Gln Val Ser Ile Thr Gly Phe Phe
1               5

<210>  61
<211>  8
<212>  PRT
<213>  Saccharomyces cerevisiae

<400>  61

Gln Gly Thr Leu Glu Ser Phe Phe
1               5


<210>  62
<211>  8
<212>  PRT
<213>  Schizosaccharomyces pombe

<400>  62

Gln Lys Ser Ile Met Ser Phe Phe
1               5


<210>  63
<211>  8
<212>  PRT
<213>  Saccharomyces cerevisiae

<400>  63

Gln Thr Ser Leu Thr Lys Phe Phe
1               5


<210>  64
<211>  8
<212>  PRT
<213>  Pyrococcus furiosus

<400>  64

Gln Ser Thr Leu Glu Ser Trp Phe
1               5


<210>  65
<211>  8
<212>  PRT
<213>  Methanococcus jannaschii

<400>  65

Gln Lys Thr Leu Asp Ala Trp Phe
1               5


<210>  66
<211>  8
<212>  PRT
<213>  Archaeoglobus fulgidus

<400>  66

Gln Ala Thr Leu Glu Arg Trp Phe
1               5

<210>  67
<211>  8
<212>  PRT
<213>  Homo sapiens

<400>  67

Gln Gly Arg Leu Asp Asp Phe Phe
1               5

<210>  68
<211>  8
<212>  PRT
<213>  Drosophila melanogaster

<400>  68

Gln Val Arg Leu Asp Ser Phe Phe
1               5

<210>  69
<211>  8
<212>  PRT
<213>  Saccharomyces cerevisiae

<400>  69

Gln Gly Arg Leu Asp Gly Phe Phe
1               5

<210>  70
<211>  8
<212>  PRT
<213>  Schizosaccharomyces pombe

<400>  70

Gln Gly Arg Leu Asp Ser Phe Phe
1               5

<210>  71
<211>  8
<212>  PRT
<213>  Homo sapiens

<400>  71

Gln Arg Ser Ile Met Ser Phe Phe
1               5

<210>  72
<211>  8
<212>  PRT
<213>  Xenopus laevis

<400>  72

Gln Arg Thr Ile Lys Ser Phe Phe
1               5


<210>  73
<211>  8
<212>  PRT
<213>  Saccharomyces cerevisiae

<400>  73

Gln Ala Thr Leu Ala Arg Phe Phe
1               5


<210>  74
<211>  8
<212>  PRT
<213>  Schizosaccharomyces pombe

<400>  74

Gln Ser Asp Ile Ser Asn Phe Phe
1               5


<210>  75
<211>  8
<212>  PRT
<213>  Homo sapiens

<400>  75

Gln Ala Val Leu Ser Arg Phe Phe
1               5


<210>  76
<211>  8
<212>  PRT
<213>  Saccharomyces cerevisiae

<400>  76

Gln Pro Thr Ile Ser Arg Phe Phe
1               5


<210>  77
<211>  8
<212>  PRT
<213>  Homo sapiens

<400>  77

Gln Ser Thr Leu Tyr Ser Phe Phe
1               5


<210>  78

<211>  8
<212>  PRT
<213>  Saccharomyces cerevisiae

<400>  78

Gln Ser Ser Leu Leu Ser Phe Phe
1               5


<210>  79
<211>  8
<212>  PRT
<213>  Homo sapiens

<400>  79

Gln Lys Thr Leu Tyr Ser Phe Phe
1               5


<210>  80
<211>  8
<212>  PRT
<213>  Saccharomyces cerevisiae

<400>  80

Gln Thr Thr Ile Glu Asp Phe Phe
1               5


<210>  81
<211>  8
<212>  PRT
<213>  Homo sapiens

<400>  81

Gln Gln Val Leu Asp Asn Phe Phe
1               5


<210>  82
<211>  8
<212>  PRT
<213>  Homo sapiens

<400>  82

Gln Leu Arg Ile Asp Ser Phe Phe
1               5


<210>  83
<211>  8
<212>  PRT
<213>  Caenorhabditis elegans

<400>  83

Gln Met Arg Leu Asp Arg Phe Phe
1               5

<210>  84
<211>  8
<212>  PRT
<213>  Saccharomyces cerevisiae

<400>  84

Gln Lys Arg Ile Asn Glu Phe Phe
1               5


<210>  85
<211>  8
<212>  PRT
<213>  Schizosaccharomyces pombe

<400>  85

Gln Ser Asn Leu Thr Gln Phe Phe
1               5


<210>  86
<211>  8
<212>  PRT
<213>  Saccharomyces cerevisiae

<400>  86

Gln Ser Arg Ile Gly Asn Phe Phe
1               5


<210>  87
<211>  8
<212>  PRT
<213>  Homo sapiens

<400>  87

Gln Asn Leu Ile Arg His Phe Phe
1               5


<210>  88
<211>  8
<212>  PRT
<213>  Saccharomyces cerevisiae

<400>  88

Gln Gln Thr Leu Ser Ser Phe Phe
1               5


<210>  89
<211>  8
<212>  PRT
<213>  Homo sapiens

<400>  89

Gln Glu Glu Leu Phe Asn Phe Phe
1               5

<210>  90
<211>  8
<212>  PRT
<213>  Homo sapiens

<400>  90

Gln Lys Gly Ile Gly Glu Phe Phe
1               5

<210>  91
<211>  8
<212>  PRT
<213>  Homo sapiens

<400>  91

Gln Leu Ile Ile Arg Asn Phe Trp
1               5